(19) Europäisches Patentamt
European Patent Office
Office européen des brevets

(11) EP 4 611 901 B1

(12) EUROPEAN PATENT SPECIFICATION

(45) Date of publication and mention of the grant of the patent:
**22.07.2026 Bulletin 2026/30**

(21) Application number: **23818609.2**

(22) Date of filing: **03.11.2023**

(51) International Patent Classification (IPC):
***A61P 35/00*** *(2006.01)* ***C07D 487/04*** *(2006.01)*
***C07D 519/00*** *(2006.01)* ***A61K 31/53*** *(2006.01)*

(52) Cooperative Patent Classification (CPC):
**A61P 35/00; C07D 487/04; C07D 519/00**

(86) International application number:
**PCT/US2023/078680**

(87) International publication number:
**WO 2024/097989 (10.05.2024 Gazette 2024/19)**

(54) **RET-LDD PROTEIN DEGRADERS**

RET-LDD-PROTEIN-ABBAUER

AGENTS DE DÉGRADATION DE PROTÉINE RET-LDD

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **04.11.2022 US 202263422741 P**

(43) Date of publication of application:
**10.09.2025 Bulletin 2025/37**

(73) Proprietor: **Bristol-Myers Squibb Company
Princeton, NJ 08543 (US)**

(72) Inventors:
- **DHAR, T.G. Murali
Princeton, New Jersey 08543 (US)**
- **PAUL, Anirudra
Princeton, New Jersey 08543 (US)**

(74) Representative: **Carpmaels & Ransford LLP
One Southampton Row
London WC1B 5HA (GB)**

(56) References cited:
**EP-A1- 3 974 422**
**WO-A1-2020/063751**
**US-A1- 2009 131 425**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 611 901 B1

## Description

### FIELD OF INVENTION

This disclosure relates to rearranged during transfection (RET) proto-oncogene tyrosine- protein kinase receptor degrading compounds. The degraders described herein are useful in the treatment of diseases and disorders associated with the modulation of RET proteins. In particular, the invention is concerned with compounds and pharmaceutical compositions that degrade the proto-oncogene tyrosine-protein kinase receptor (RET) via the ubiquitin proteasome pathway (UPP), and use of said compounds and pharmaceutical compositions in methods of treating diseases and disorders associated with said RET protein and pathway.

### BACKGROUND OF THE INVENTION

Protein degradation is a highly regulated and essential process that maintains cellular homeostasis. The selective identification and removal of damaged, misfolded, or excess proteins is achieved via the ubiquitin-proteasome pathway (UPP). The UPP is central to the regulation of almost all cellular processes, including antigen processing, apoptosis, biogenesis of organelles, cell cycling, DNA transcription and repair, differentiation and development, immune response and inflammation, neural and muscular degeneration, morphogenesis of neural networks, modulation of cell surface receptors, ion channels and the secretory pathway, the response to stress and extracellular modulators, ribosome biogenesis and viral infection. Covalent attachment of multiple ubiquitin molecules by an E3 ubiquitin ligase to a terminal lysine residue marks the protein for proteasome degradation, where the protein is digested into small peptides and eventually into its constituent amino acids that serve as building blocks for new proteins.

Defective proteasomal degradation has been linked to a variety of disorders including cancer and others.

Cereblon forms part of an E3 ubiquitin ligase complex which interacts with damaged DNA binding protein 1, forming an E3 ubiquitin ligase complex with Cullin 4 and the E2-binding protein ROC1 (known as RBX1) where it functions as a substrate receptor to select proteins for ubiquitination. The binding of lenalidomide to cereblon facilitates subsequent binding of cereblon to Ikaros and Aiolos, leading to their ubiquitination and degradation by the proteasome (see Lu, G. et al. "The myeloma drug lenalidomide promotes the cereblon-dependent destruction of Ikaros proteins" Science, 2014, 343:305-309; Krönke, J. et al. "Lenalidomide causes selective degradation of IKZF1 and IKZF3 in multiple myeloma cells" Science, 2014, 343:301-305).

The rearranged during transfection (RET) proto-oncogene tyrosine-protein kinase receptor, a cell surface tyrosine kinase receptor, is widely known for its essential role in cell survival, differentiation, proliferation, migration and chemotaxis. RET germline missense and somatic mutations cause medullary thyroid cancer (MTC) and neuroendocrine tumors, whereas RET fusion proteins, overexpression, and copy number gains are present in a broad spectrum of additional cancers such as papillary thyroid cancer, pancreatic cancer, melanoma, leukemia, lung adenocarcinomas, and breast cancer. (Liu Xuan et al., "RET kinase alterations in targeted cancer therapy", Cancer Drug Resist, 2020; and Mulligan LM., "RET revisited: expanding the oncogenic portfolio", Nat Rev Cancer., 2014, 14(3), 173-186).

The academic and clinical interest in RET has led to the identification of several RET mutations that are clinically relevant including RET G810R, RET G810S, and RET G810C. (Solomon, et al., "RET Solvent Front Mutations Mediated Acquired Resistance to Selective RET Inhibition in RET-driven malignancies", J Thoracic Oncolog., 2020). Treatment of patients with non small cell lung cancer selpercatinib has been shown to cause RET mutations that infer resistances including the RET G810R, RET G810S, and RET G810C mutations.

Other approved tyrosine kinase inhibitors such as sunitinib, sorafenib, ponatinib and lenvatinib have also shown some RET activity in pre-clinical trials and are currently under investigation in numerous phase II clinical trials for treatment of RET fusion positive lung adenocarcinoma (LAD). (Song M., "Progress in Discovery of KIF5B-RET Kinase Inhibitors for the Treatment of Non-Small-Cell Lung Cancer", J Med Chem., 2015, 58(9), 3672-3681; Watson AJ., et al., "Identification of selective inhibitors of RET and comparison with current clinical candidates through development and validation of a robust screening cascade", F1000Research 2016, 5:1005).

WO2020063751A1 discusses imidazo[1, 2-a]pyridine and [1, 2, 4]triazolo[1, 5-a]pyridine compounds as RET kinase inhibitors. EP3974422A1 discusses compounds as RET kinase inhibitors. US2009/131425A1 discusses compounds as inhibitors of Aurora-kinases.

Despite these efforts, there remains an unmet need in the field new RET modulators to treat disorders mediated by RET in hosts in need thereof, including humans.

### SUMMARY OF THE INVENTION

A first aspect of the instant disclosure is directed to compounds of Formula I:

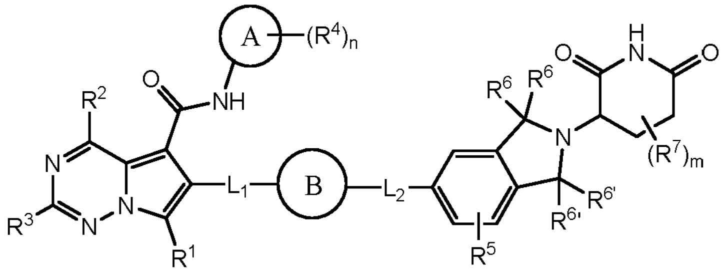

(I),

or a pharmaceutically acceptable salt thereof,
wherein

$R^1$ is $C_1$-$C_6$ alkyl, $C_3$-$C_8$ cycloalkyl, heterocycloalkyl, aryl, or heteroaryl, wherein the alkyl, cycloalkyl, heterocycloalkyl, aryl or heteroaryl is optionally substituted with one or more $R^8$;
A is aryl or heteroaryl, wherein the aryl or heteroaryl is optionally substituted with one or more $R^9$;
B is heterocycloalkyl or $C_3$-$C_8$ cycloalkyl;
$L_1$ is $C_1$-$C_6$ alkyl, $C_2$-$C_6$ alkenyl, or $C_2$-$C_6$ alkynyl;
$L_2$ is -C(O)-$(CH_2)_p$-(4- to 12-heterocycloalkyl)-; -C(O)-$(OCH_2CH_2)_p$-(4- to 12-heterocycloalkyl)-; -C(O)NH-$(CH_2)_p$-(4- to 12-heterocycloalkyl)-; -C(O)NH-$(CH_2CH_2O)_p$-(4- to 12-heterocycloalkyl)-; -C(O)-$(CH_2CH_2O)_p$-(4- to 12-heterocycloalkyl)-; -$(OCH_2CH_2)_p$-(4- to 12-heterocycloalkyl)-; -C(O)-$(CH_2)_p$-(4- to 12-heterocycloalkyl)-C(O)-; or -$(CH_2)_p$-(4- to 12-heterocycloalkyl)-C(O)-;
$R^2$ is $NR^{10}R^{11}$;
$R^3$ is H, halogen, $NH_2$, or $C_1$-$C_4$ alkyl;
each $R^4$ is independently $C_1$-$C_6$ alkyl, $C_2$-$C_6$ alkenyl, or $C_2$-$C_6$ alkynyl, wherein the alkyl, alkenyl, or alkynyl is optionally substituted with one or more $C_1$-$C_6$ alkoxy, $C_1$-$C_6$ thioalkyl, $NH_2$, -NH($C_1$-$C_6$ alkyl), or -N($C_1$-$C_6$ alkyl)($C_1$-$C_6$ alkyl); or two $R^4$, on adjacent carbons taken together, can combine to form $C_3$-$C_8$ cycloalkyl, heterocycloalkyl, aryl, or heteroaryl;
$R^5$ is H, OH, CN, $NO_2$. or $C_1$-$C_4$ alkyl;
each $R^6$ or $R^{6'}$ is H; or
two $R^6$ can combine to form an oxo group; or
two $R^{6'}$ can combine to form an oxo group;
$R^7$, $R^8$, and $R^9$ are each independently H, halogen, CN, $NO_2$, OH, $NH_2$, $C_1$-$C_6$ alkyl, $C_2$-$C_6$ alkenyl, $C_2$-$C_6$ alkynyl, $C_3$-$C_8$ cycloalkyl, heterocycloalkyl, aryl, or heteroaryl; or
two $R^7$on adjacent carbons taken together, can combine to form $C_3$-$C_8$ cycloalkyl, heterocycloalkyl, aryl, or heteroaryl;
$R^{10}$ and $R^{11}$ are independently H or $C_1$-$C_4$ alkyl;
m is an integer from 0 to 2;
n is an integer from 0 to 4; and

p is an integer from 1 to 8.

Another aspect of the disclosure is directed to pharmaceutical compositions comprising a compound of Formula I, or a pharmaceutically acceptable salt thereof, and a pharmaceutically acceptable carrier. The pharmaceutically acceptable carrier can further include an excipient, diluent, or surfactant.

The pharmaceutical compositions can comprise the compounds of the present invention for use in treating diseases described herein. The compositions can contain at least one compound of the invention and a pharmaceutically acceptable carrier.

Another aspect of the present disclosure relates to a compound of Formula I, or a pharmaceutically acceptable salt thereof, for use in treating or preventing a disease associated with RET modulation. The invention also provides the use of the compounds described herein in the manufacture of a medicament for the treatment of a disease associated with RET.

The present invention also provides compounds of Formula I that bind and inhibit to E3 ligase and further degrade the RET protein. These E3 ligase-binding compounds (degraders) can also be useful in the treatment of RET mediated diseases and cancer including Hirschsprung disease, medullary thyroid cancer (MTC), thyroid carcinoma, familial medullary thyroid carcinoma, multiple endocrine neoplasia, multiple endocrine neoplasia type 2 (MEN-2, MEN-2A, MEN-2B), neuroendocrine tumors, central nervous system tumors, central hypoventilation syndrome, renal agenesis, pheochromocytoma and parathyroid hyperplasia.

The present invention further provides compounds that can degrade RET while binding E3 ligase. In some embodiments, the efficacy-safety profile of the compounds of the current disclosure can be improved relative to other

known RET inhibitors. Additionally, the present technology also has the advantage of being able to be used for a number of different types of diseases, including disorder mediated by RET fusion proteins, overexpression, or copy number gains, such as papillary thyroid cancer, pancreatic cancer, melanoma, leukemia, acute myeloid leukemia (AML), chronic myelomonocytic leukemia, lung adenocarcinomas, lung cancer, non-small cell lung cancer (NSCLC), nonsyndromic paraganglioma, breast cancer, nonhereditary (sporadic) cancers, colorectal, or a hematologic malignancy. Additional features and advantages of the present technology will be apparent to one of skill in the art upon reading the Detailed Description of the Invention, below.

## DETAILED DESCRIPTION OF THE INVENTION

The present disclosure relates to RET-degrading compounds of Formula I, or pharmaceutically acceptable salts thereof, which may inhibit E3 ligase activity and are accordingly useful in methods of treatment of the human or animal body. The present disclosure also relates pharmaceutical compositions comprising these compounds and to their use in the treatment of disorders and diseases in which RET is implicated, such as inflammation, an autoimmune disease, a cancer, an infection, a disease or disorder of the central nervous system, a metabolic disease, a cardiovascular disease, a respiratory disease, a kidney disease, a liver disease, an ocular disease, a skin disease, a lymphatic disease, a rheumatic disease, a psychological disease, graft versus host disease, allodynia, or an RET-related disease in a subject that has been determined to carry a germline or somatic mutation in RET.

## Definitions

Unless otherwise stated, the following terms used in the specification and claims have the following meanings set out below.

As used herein, "alkyl", "C1, C2, C3, C4, C5 or C6 alkyl" or "C1-C6 alkyl" is intended to include C1, C2, C3, C4, C5 or C6 straight chain (linear) saturated aliphatic hydrocarbon groups and C3, C4, C5 or C6 branched saturated aliphatic hydrocarbon groups. For example, C1-C6 alkyl is intends to include C1, C2, C3, C4, C5 and C6 alkyl groups. Examples of alkyl include, moieties having from one to six carbon atoms, such as, but not limited to, methyl, ethyl, n-propyl, i-propyl, n-butyl, s-butyl, t-butyl, n-pentyl, i-pentyl, or n-hexyl. In some embodiments, a straight chain or branched alkyl has six or fewer carbon atoms (e.g., C1-C6 for straight chain, C3-C6 for branched chain), and in another embodiment, a straight chain or branched alkyl has four or fewer carbon atoms.

As used herein, the term "optionally substituted alkyl" refers to unsubstituted alkyl or alkyl having designated substituents replacing one or more hydrogen atoms, e.g., 1, 2, or 3, on one or more, e.g., 1, 2, 3, 4, or more, carbons of the hydrocarbon backbone. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

As used herein, the term "alkenyl" includes unsaturated aliphatic groups analogous in length and possible substitution to the alkyls described above, but that contain at least one double bond. For example, the term "alkenyl" includes straight chain alkenyl groups (e.g., ethenyl, propenyl, butenyl, pentenyl, hexenyl, heptenyl, octenyl, nonenyl, decenyl), and branched alkenyl groups. In certain embodiments, a straight chain or branched alkenyl group has six or fewer carbon atoms in its backbone (e.g., C2-C6 for straight chain, C3-C6 for branched chain). The term "C2-C6" includes alkenyl groups containing two to six carbon atoms. The term "C3-C6" includes alkenyl groups containing three to six carbon atoms.

As used herein, the term "optionally substituted alkenyl" refers to unsubstituted alkenyl or alkenyl having designated substituents replacing one or more hydrogen atoms, e.g., 1, 2, or 3, on one or more, e.g., 1, 2, 3, 4, or more, hydrocarbon backbone carbon atoms. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, heterocyclyl, alkylaryl, or an aromatic or heteroaromatic moiety.

As used herein, the term "alkynyl" includes unsaturated aliphatic groups analogous in length and possible substitution to the alkyls described above, but which contain at least one triple bond. For example, "alkynyl" includes straight chain alkynyl groups (e.g., ethynyl, propynyl, butynyl, pentynyl, hexynyl, heptynyl, octynyl, nonynyl, decynyl), and

branched alkynyl groups. In certain embodiments, a straight chain or branched alkynyl group has six or fewer carbon atoms in its backbone (e.g., C2-C6 for straight chain, C3-C6 for branched chain). The term "C2-C6" includes alkynyl groups containing two to six carbon atoms. The term "C3-C6" includes alkynyl groups containing three to six carbon atoms. As used herein, "C2-C6 alkenylene linker" or "C2-C6 alkynylene linker" is intended to include C2, C3, C4, C5 or C6 chain (linear or branched) divalent unsaturated aliphatic hydrocarbon groups. For example, C2-C6 alkenylene linker is intended to include C2, C3, C4, C5 and C6 alkenylene linker groups.

As used herein, the term "optionally substituted alkynyl" refers to unsubstituted alkynyl or alkynyl having designated substituents replacing one or more hydrogen atoms, e.g., 1, 2, or 3, on one or more, e.g., 1, 2, 3, 4, or more, hydrocarbon backbone carbon atoms. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

Other optionally substituted moieties (such as optionally substituted cycloalkyl, heterocycloalkyl, aryl, or heteroaryl) include both the unsubstituted moieties and the moieties having one or more of the designated substituents. For example, substituted heterocycloalkyl includes those substituted with one or more, e.g., 1, 2, 3, 4, or more, alkyl groups, such as 2,2,6,6-tetramethyl-piperidinyl and 2,2,6,6-tetramethyl-1,2,3,6-tetrahydropyridinyl.

As used herein, the term "cyano" refers to a nitrile radical (e.g., -CN).

As used herein, the term "cycloalkyl" refers to a saturated or partially unsaturated hydrocarbon monocyclic or polycyclic (e.g., fused, bridged, or spiro rings) system having 3 to 30 carbon atoms (e.g., C3-C12, C3-C10, or C3-C8). Examples of cycloalkyl include, but are not limited to, cyclopropyl, cyclobutyl, cyclopentyl, cyclohexyi, cycloheptyl, cyclooctyl, cyclopentenyl, cyclohexenyl, cycloheptenyl, 1,2,3,4-tetrahydronaphthalenyl, and adamantyl. In the case of polycyclic cycloalkyl, only one of the rings in the cycloalkyl needs to be non-aromatic.

As used herein, the term "heterocycloalkyl" refers to a saturated or partially unsaturated 3-8 membered monocyclic or bicyclic, 7-12 membered bicyclic (fused, bridged, or spiro rings), or 11-14 membered tricyclic ring system (fused, bridged, or spiro rings) having one or more heteroatoms (such as O, N, S, P, or Se), e.g., 1 or 1-2 or 1-3 or 1-4 or 1-5 or 1-6 heteroatoms, or e.g., 1, 2, 3, 4, 5, or 6 heteroatoms, independently selected from the group consisting of nitrogen, oxygen and sulfur, unless specified otherwise. Examples of heterocycloalkyl groups include, but are not limited to, piperidinyl, piperazinyl, pyrrolidinyl, dioxanyl, tetrahydrofuranyl, isoindolinyl, indolinyl, imidazolidinyl, pyrazolidinyl, oxazolidinyl, isoxazolidinyl, triazolidinyl, oxiranyl, azetidinyl, oxetanyl, thietanyl, 1,2,3,6-tetrahydropyridinyl, tetrahydropyranyl, dihydropyranyl, pyranyl, morpholinyl, tetrahydrothiopyranyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, 1,4-dioxa-8-azaspiro[4.5]decanyl, 1,4-dioxaspiro[4.5]decanyl, 1-oxaspiro[4.5]decanyl, 1-azaspiro[4.5]decanyl, 3'H-spiro[cyclohexane-1,1'-isobenzofurran]-yl, 7'H-spiro[cyclohexane-1,5'-furo[3,4-b]pyridin]-yl, 3'H-spiro[cyclohexane-1,1'-furo[3,4-c]pyridin]-yl, 3-azabicyclo[3.1.0]hexanyl, 3-azabicyclo[3.1.0]hexan-3-yl, 1,4,5,6-tetrahydropyrrolo[3,4-c]pyrazolyl, 3,4,5,6,7,8-hexahydropyrido[4,3-d]pyrimidinyl, 4,5,6,7-tetrahydro-1H-pyrazolo[3,4-c]pyridinyl, 5,6,7,8-tetrahydropyrido[4,3-d]pyrimidinyl, 2-azaspiro[3.3]heptanyl, 2-methyl-2-azaspiro[3.3]heptanyl, 2-azaspiro[3.5]nonanyl, 2-methyl-2-azaspiro[3.5]nonanyl, 2-azaspiro[4.5]decanyl, 2-methyl-2-azaspiro[4.5]decanyl, 2-oxa-azaspiro[3.4]octanyl, 2-oxa-azaspiro[3.4]octan-6-yl, 5,6-dihydro-4H-cyclopenta[b]thiophenyl, and the like. In the case of multicyclic heterocycloalkyl, only one of the rings in the heterocycloalkyl needs to be non-aromatic (e.g., 4,5,6,7-tetrahydrobenzo[c]isoxazolyl).

As used herein, the term "optionally substituted heterocycloalkyl" refers to unsubstituted heterocycloalkyl having designated substituents replacing one or more hydrogen atoms, e.g., 1, 2, or 3, on one or more, e.g., 1, 2, 3, 4, or more, carbon or heteroatom. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

Unless otherwise specifically defined, the term "aryl" refers to cyclic, aromatic hydrocarbon groups that have 1 to 3 aromatic rings, including monocyclic or bicyclic groups such as phenyl, biphenyl, or naphthyl. Where containing two aromatic rings (bicyclic, etc.), the aromatic rings of the aryl group may be joined at a single point (e.g., biphenyl), or fused (e.g., naphthyl). The aryl group may be optionally substituted by one or more substituents, e.g., 1 to 5 substituents, at any point of attachment. Exemplary substituents include, but are not limited to, -H, -halogen. -O-(C1-C6) alkyl, (C1-C6) alkyl, -O-(C2-C6) alkenyl, -O-(C2-C6) alkynyl, (C2-C6) alkenyl, (C2-C6) alkynyl, -OH, -OP(O)(OH)2, -OC(O)(C1-C6) alkyl, -C(O)(C1-C6) alkyl, -OC(O)O(C1-C6) alkyl, -NH2, NH((C1-C6) alkyl), N((C1-C6) alkyl)2, -S(O)2-(C1-C6) alkyl, -S(O)NH(C1-C6) alkyl, and -S(O)N((C1-C6) alkyl)2. The substituents can themselves be optionally substituted. Furthermore, when containing two or more fused rings, the aryl groups herein defined may have a saturated or partially unsaturated ring fused with a fully unsaturated aromatic ring Exemplary ring systems of these aryl groups include, but are not limited to, phenyl, biphenyl, naphthyl, anthracenyl, phenalenyl, phenanthrenyl, indanyl, indenyl, tetrahydronaphthalenyl, tetrahydrobenzoannulenyl, 10,11-dihydro-5H-dibenzo[a,d][7]annulenyl, and the like.

Unless otherwise specifically defined, "heteroaryl" means a monovalent monocyclic or polycyclic aromatic radical of 5 to 24 ring atoms, containing one or more ring heteroatoms selected from N, O, S, P, Se, or B, the remaining ring atoms being C. Heteroaryl as herein defined also means a bicyclic heteroaromatic group wherein the heteroatom is selected from N, O, S, P, Se, or B. Heteroaryl as herein defined also means a tricyclic heteroaromatic group containing one or more ring heteroatoms selected from N, O, S, P, Se, or B. The aromatic radical is optionally substituted independently with one or more substituents described herein. Examples include, but are not limited to, furyl, thienyl, pyrrolyl, pyridyl, pyrazolyl, pyrimidinyl, imidazolyl, isoxazolyl, oxazolyl, oxadiazolyl, pyrazinyl, indolyl, thiophen-2-yl, quinolinyl, benzopyranyl, isothiazolyl, thiazolyl, thiadiazole, indazole, benzimidazolyl, thieno[3,2-b]thiophene, triazolyl, triazinyl, imidazo[1,2-b]pyrazolyl, furo[2,3-c]pyridinyl, imidazo[1,2-a]pyridinyl, indazolyl, pyrrolo[2,3-c]pyridinyl, pyrrolo[3,2-c]pyridinyl, pyrazolo[3,4-c]pyridinyl, thieno[3,2-c]pyridinyl, thieno[2,3-c]pyridinyl, thieno[2,3-b]pyridinyl, benzothiazolyl, indolyl, indolinyl, indolinonyl, dihydrobenzothiophenyl, dihydrobenzofuranyl, benzofuran, chromanyl, thiochromanyl, tetrahydroquinolinyl, dihydrobenzothiazine, quinolinyl, isoquinolinyl, 1,6-naphthyridinyl, benzo[de]isoquinolinyl, pyrido[4,3-b][1,6]naphthyridinyl, thieno[2,3-b]pyrazinyl, quinazolinyl, tetrazolo[1,5-a]pyridinyl, [1,2,4]triazolo[4,3-a]pyridinyl, isoindolyl, pyrrolo[2,3-b]pyridinyl, pyrrolo[3,4-b]pyridinyl, pyrrolo[3,2-b]pyridinyl, imidazo[5,4-b]pyridinyl, pyrrolo[1,2-a]pyrimidinyl, tetrahydro pyrrolo[1,2-a]pyrimidinyl, 3,4-dihydro-2H-1λ2-pyrrolo[2,1-b]pyrimidine, dibenzo[b,d] thiophene, pyridin-2-one, furo[3,2-c]pyridinyl, furo[2,3-c]pyridinyl, 1H-pyrido[3,4-b][1,4] thiazinyl, benzoxazolyl, benzisoxazolyl, furo[2,3-b]pyridinyl, benzothiophenyl, 1,5-naphthyridinyl, furo[3,2-b]pyridine, [1,2,4]triazolo[1,5-a]pyridinyl, benzo [1,2,3]triazolyl, imidazo[1,2-a]pyrimidinyl, [1,2,4]triazolo[4,3-b]pyridazinyl, benzo[c][1,2,5]thiadiazolyl, benzo[c][1,2,5]oxadiazole, 1,3-dihydro-2H-benzo[d]imidazol-2-one, 3,4-dihydro-2H-pyrazolo [1,5-b][1,2]oxazinyl, 4,5,6,7-tetrahydropyrazolo[1,5-a]pyridinyl, thiazolo [5,4-d]thiazolyl, imidazo[2,1-b][1,3,4]thiadiazolyl, thieno[2,3-b]pyrrolyl, 3H-indolyl, and derivatives thereof. Furthermore, when containing two or more fused rings, the heteroaryl groups defined herein may have one or more saturated or partially unsaturated ring fused with a fully unsaturated aromatic ring, e.g., a 5-membered heteroaromatic ring containing 1 to 3 heteroatoms selected from N, O, S, P, Se, or B, or a 6-membered heteroaromatic ring containing 1 to 3 nitrogens, wherein the saturated or partially unsaturated ring includes 0 to 4 heteroatoms selected from N, O, S, P, Se, or B, and is optionally substituted with one or more oxo. In heteroaryl ring systems containing more than two fused rings, a saturated or partially unsaturated ring may further be fused with a saturated or partially unsaturated ring described herein. Exemplary ring systems of these heteroaryl groups include, for example, indolinyl, indolinonyl, dihydrobenzothiophenyl, dihydrobenzofuran, chromanyl, thiochromanyl, tetrahydroquinolinyl, dihydrobenzothiazine, 3,4-dihydro-11H-isoquinolinyl, 2,3-dihydrobenzofuranyl, benzofuranonyl, indolinyl, oxindolyl, indolyl, 1,6-dihydro-7H-pyrazolo[3,4-c]pyridin-7-onyl, 7,8-dihydro-6H-pyrido[3,2-b]pyrrolizinyl, 8H-pyrido[3,2-b]pyrrolizinyl, 1,5,6,7-tetrahydrocyclopenta[b]pyrazolo[4,3-e]pyridinyl, 7,8-dihydro-6H-pyrido[3,2-b]pyrrolizine, pyrazolo[1,5-a]pyrimidin-7(4H)-only, 3,4-dihydropyrazino[1,2-a]indol-1(2H)-onyl, or benzo[c][1,2]oxaborol-1(3H)-olyl.

The cycloalkyl, heterocycloalkyl, aryl, or heteroaryl ring can be substituted at one or more, e.g., 1, 2, 3, 4, or more, ring positions (e.g., the ring-forming carbon or heteroatom such as N) with such substituents as described above, for example, alkyl, alkenyl, alkynyl, halogen, hydroxyl, alkoxy, alkylcarbonyloxy, arylcarbonyloxy, alkoxycarbonyloxy, aryloxycarbonyloxy, carboxylate, alkylcarbonyl, alkylaminocarbonyl, aralkylaminocarbonyl, alkenylaminocarbonyl, alkylcarbonyl, arylcarbonyl, aralkylcarbonyl, alkenylcarbonyl, alkoxycarbonyl, aminocarbonyl, alkylthiocarbonyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Aryl and heteroaryl groups can also be fused or bridged with alicyclic or heterocyclic rings, which are not aromatic so as to form a multicyclic system (e.g., tetralin, methylenedioxyphenyl such as benzo[d][1,3]dioxole-5-yl).

As used herein, the term "substituted," means that any one or more hydrogen atoms on the designated atom is replaced with a selection from the indicated groups, provided that the designated atom's normal valency is not exceeded, and that the substitution results in a stable compound. When a substituent is oxo or keto (i.e., =O), then 2 hydrogen atoms on the atom are replaced Keto substituents are not present on aromatic moieties. Ring double bonds, as used herein, are double bonds that are formed between two adjacent ring atoms (e.g., C=C, C=N or N=N). "Stable compound" and "stable structure" are meant to indicate a compound that is sufficiently robust to survive isolation to a useful degree of purity from a RM, and formulation into an efficacious therapeutic agent.

When a bond to a substituent is shown to cross a bond connecting two atoms in a ring, then such substituent may be bonded to any atom in the ring. When a substituent is listed without indicating the atom via which such substituent is bonded to the rest of the compound of a given formula, then such substituent may be bonded via any atom in such formula.

Combinations of substituents and/or variables are permissible, but only if such combinations result in stable compounds.

When any variable (e.g., R) occurs more than one time in any constituent or formula for a compound, its definition at each occurrence is independent of its definition at every other occurrence. Thus, for example, if a group is shown to be substituted with 0-2 R moieties, then the group may optionally be substituted with up to two R moieties and R at each occurrence is selected independently from the definition of R. Also, combinations of substituents and/or variables are permissible, but only if such combinations result in stable compounds.

As used herein, the term "hydroxy" or "hydroxyl" includes groups with an -OH or - O-.

As used herein, the term "halo" or "halogen" refers to fluoro, chloro, bromo and iodo.

The term "haloalkyl" or "haloalkoxyl" refers to an alkyl or alkoxyl substituted with one or more halogen atoms.

As used herein, the term "optionally substituted haloalkyl" refers to unsubstituted haloalkyl having designated substituents replacing one or more hydrogen atoms, e.g., 1, 2, or 3, on one or more, e.g., 1, 2, 3, 4, or more, hydrocarbon backbone carbon atoms. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

As used herein, the term "alkoxy" or "alkoxyl" includes substituted and unsubstituted alkyl, alkenyl and alkynyl groups covalently linked to an oxygen atom. Examples of alkoxy groups or alkoxyl radicals include, but are not limited to, methoxy, ethoxy, isopropyloxy, propoxy, butoxy and pentoxy groups. Examples of substituted alkoxy groups include halogenated alkoxy groups. The alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Examples of halogen substituted alkoxy groups include, but are not limited to, fluoromethoxy, difluoromethoxy, trifluoromethoxy, chloromethoxy, dichloromethoxy and trichloromethoxy.

It is to be understood that the present disclosure provides methods for the synthesis of the compounds of any of the Formulae described herein. The present disclosure also provides detailed methods for the synthesis of various disclosed compounds of the present disclosure according to the following schemes as well as those shown in the Examples.

It is to be understood that, throughout the description, where compositions are described as having, including, or comprising specific components, it is contemplated that compositions also consist essentially of, or consist of, the recited components. Similarly, where methods or processes are described as having, including, or comprising specific process steps, the processes also consist essentially of, or consist of, the recited processing steps. Further, it should be understood that the order of steps or order for performing certain actions is immaterial so long as the invention remains operable. Moreover, two or more steps or actions can be conducted simultaneously.

It is to be understood that the synthetic processes of the disclosure can tolerate a wide variety of functional groups, therefore various substituted starting materials can be used. The processes generally provide the desired final compound at or near the end of the overall process, although it may be desirable in certain instances to further convert the compound to a pharmaceutically acceptable salt thereof.

It is to be understood that compounds of the present disclosure can be prepared in a variety of ways using commercially available starting materials, compounds known in the literature, or from readily prepared intermediates, by employing standard synthetic methods and procedures either known to those skilled in the art, or which will be apparent to the skilled artisan in light of the teachings herein. Standard synthetic methods and procedures for the preparation of organic molecules and functional group transformations and manipulations can be obtained from the relevant scientific literature or from standard textbooks in the field. Although not limited to any one or several sources, classic texts such as Smith, M. B., March, J., March's Advanced Organic Chemistry: Reactions, Mechanisms, and Structure, 5th edition, John Wiley & Sons: New York, 2001; Greene, T. W., Wuts, P. G. M., Protective Groups in Organic Synthesis, 3rd edition, John Wiley & Sons: New York, 1999; R. Larock, Comprehensive Organic Transformations, VCH Publishers (1989); L. Fieser and M. Fieser, Fieser and Fieser's Reagents for Organic Synthesis, John Wiley and Sons (1994); and L. Paquette, ed, Encyclopedia of Reagents for Organic Synthesis, John Wiley and Sons (1995), are useful and recognised reference textbooks of organic synthesis known to those in the art.

One of ordinary skill in the art will note that, during the reaction sequences and synthetic schemes described herein, the order of certain steps may be changed, such as the introduction and removal of protecting groups. One of ordinary skill in the art will recognise that certain groups may require protection from the reaction conditions via the use of

protecting groups. Protecting groups may also be used to differentiate similar functional groups in molecules. A list of protecting groups and how to introduce and remove these groups can be found in Greene, T. W., Wuts, P. G. M., Protective Groups in Organic Synthesis, 3rd edition. John Wiley & Sons. New York, 1999.

It is to be understood that any description of a method of treatment or prevention is to be interpreted as relating to the compound for use in such a treatment or prevention as is described herein. The treatment or prevention includes treatment or prevention of human or non-human animals including rodents and other disease models.

As used herein, the term "subject" is interchangeable with the term "subject in need thereof", both of which refer to a subject having a disease or having an increased risk of developing the disease. A "subject" includes a mammal. The mammal can be e.g., a human or appropriate non-human mammal, such as primate, mouse, rat, dog, cat, cow, horse, goat, camel, sheep or a pig. In one embodiment, the mammal is a human. A subject in need thereof can be one who has been previously diagnosed or identified as having a disease or disorder disclosed herein. A subject in need thereof can also be one who is suffering from a disease or disorder disclosed herein. Alternatively, a subject in need thereof can be one who has an increased risk of developing such disease or disorder relative to the population at large (i.e., a subject who is predisposed to developing such disorder relative to the population at large). A subject in need thereof can have a refractory or resistant a disease or disorder disclosed herein (i.e., a disease or disorder disclosed herein that does not respond or has not yet responded to treatment). The subject may be resistant at start of treatment or may become resistant during treatment. In some embodiments, the subject in need thereof received and failed all known effective therapies for a disease or disorder disclosed herein. In some embodiments, the subject in need thereof received at least one prior therapy.

As used herein, the term "treating" or "treat" describes the management and care of a patient for the purpose of combating a disease, condition, or disorder and includes the administration of a compound of the present disclosure, or a pharmaceutically acceptable salt, thereof, to alleviate the symptoms or complications of a disease, condition or disorder, or to eliminate the disease, condition or disorder. The term "treat" can also include treatment of a cell in vitro or an animal model. It is to be appreciated that references to "treating" or "treatment" include the alleviation of established symptoms of a condition. "Treating" or "treatment" of a state, disorder or condition therefore includes: (1) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, i.e., arresting, reducing or delaying the development of the disease or a relapse thereof (in case of maintenance treatment) or at least one clinical or subclinical symptom thereof, or (3) relieving or attenuating the disease, i.e., causing regression of the state, disorder or condition or at least one of its clinical or subclinical symptoms.

It is to be understood that a compound of the present disclosure, or a pharmaceutically acceptable salt thereof, can or may also be used to prevent a relevant disease, condition or disorder, or used to identify suitable candidates for such purposes.

As used herein, the term "preventing," "prevent," or "protecting against" describes reducing or eliminating the onset of the symptoms or complications of such disease, condition or disorder.

It is to be understood that one skilled in the art may refer to general reference texts for detailed descriptions of known techniques discussed herein or equivalent techniques. These texts include Ausubel et al., Current Protocols in Molecular Biology, John Wiley and Sons, Inc. (2005), Sambrook et al., Molecular Cloning, A Laboratory Manual 3rd edition), Cold Spring Harbor Press, Cold Spring Harbor, N.Y. (2000); Coligan et al., Current Protocols in Immunology, John Wiley & Sons, N Y.: Enna et al., Current Protocols in Pharmacology, John Wiley & Sons, N.Y.; Fingl et al., The Pharmacological Basis of Therapeutics (1975), Remington's Pharmaceutical Sciences, Mack Publishing Co., Easton, Pa., 18th edition (1990). These texts can, of course, also be referred to in making or using an aspect of the disclosure.

It is to be understood that the present disclosure also provides pharmaceutical compositions comprising any compound described herein in combination with at least one pharmaceutically acceptable excipient or carrier.

As used herein, the term "pharmaceutical composition" is a formulation containing the compounds of the present disclosure in a form suitable for administration to a subject. In one embodiment, the pharmaceutical composition is in bulk or in unit dosage form. The unit dosage form is any of a variety of forms, including, for example, a capsule, an IV bag, a tablet, a single pump on an aerosol inhaler or a vial. The quantity of active ingredient (e.g., a formulation of the disclosed compound or salt thereof) in a unit dose of composition is an effective amount and is varied according to the particular treatment involved. One skilled in the art will appreciate that it is sometimes necessary to make routine variations to the dosage depending on the age and condition of the patient. The dosage will also depend on the route of administration. A variety of routes are contemplated, including oral, pulmonary, rectal, parenteral, transdermal, subcutaneous, intravenous, intramuscular, intraperitoneal, inhalational, buccal, sublingual, intrapleural, intrathecal, intranasal, and the like. Dosage forms for the topical or transdermal administration of a compound of this disclosure include powders, sprays, ointments, pastes, creams, lotions, gels, solutions, patches and inhalants. In one embodiment, the active compound is mixed under sterile conditions with a pharmaceutically acceptable carrier, and with any preservatives, buffers, or propellants that are required.

As used herein, the term "pharmaceutically acceptable" refers to those compounds, anions, cations, materials,

compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the term "pharmaceutically acceptable excipient" means an excipient that is useful in preparing a pharmaceutical composition that is generally safe, non-toxic and neither biologically nor otherwise undesirable, and includes excipient that is acceptable for veterinary use as well as human pharmaceutical use. A "pharmaceutically acceptable excipient" as used in the specification and claims includes both one and more than one such excipient.

It is to be understood that a pharmaceutical composition of the disclosure is formulated to be compatible with its intended route of administration. Examples of routes of administration include parenteral, e.g., intravenous, intradermal, subcutaneous, oral (e.g., ingestion), inhalation, transdermal (topical), and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens, antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

It is to be understood that a compound or pharmaceutical composition of the disclosure can be administered to a subject in many of the well-known methods currently used for chemotherapeutic treatment. For example, a compound of the disclosure may be injected into the blood stream or body cavities or taken orally or applied through the skin with patches. The dose chosen should be sufficient to constitute effective treatment but not so high as to cause unacceptable side effects. The state of the disease condition (e.g., a disease or disorder disclosed herein) and the health of the patient should preferably be closely monitored during and for a reasonable period after treatment.

As used herein, the term "therapeutically effective amount", refers to an amount of a pharmaceutical agent to treat, ameliorate, or prevent an identified disease or condition, or to exhibit a detectable therapeutic or inhibitory effect. The effect can be detected by any assay method known in the art. The precise effective amount for a subject will depend upon the subject's body weight, size, and health; the nature and extent of the condition; and the therapeutic or combination of therapeutics selected for administration. Therapeutically effective amounts for a given situation can be determined by routine experimentation that is within the skill and judgment of the clinician.

It is to be understood that, for any compound, the therapeutically effective amount can be estimated initially either in cell culture assays, e.g., of neoplastic cells, or in animal models, usually rats, mice, rabbits, dogs, or pigs. The animal model may also be used to determine the appropriate concentration range and route of administration. Such information can then be used to determine useful doses and routes for administration in humans. Therapeutic/prophylactic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between toxic and therapeutic effects is the therapeutic index, and it can be expressed as the ratio, LD50/ED50. Pharmaceutical compositions that exhibit large therapeutic indices are preferred. The dosage may vary within this range depending upon the dosage form employed, sensitivity of the patient, and the route of administration.

Dosage and administration are adjusted to provide sufficient levels of the active agent(s) or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy.

The pharmaceutical compositions containing active compounds of the present disclosure may be manufactured in a manner that is generally known, e.g., by means of conventional mixing, dissolving, granulating, dragee-making, levigating, emulsifying, encapsulating, entrapping, or lyophilising processes Pharmaceutical compositions may be formulated in a conventional manner using one or more pharmaceutically acceptable carriers comprising excipients and/or auxiliaries that facilitate processing of the active compounds into preparations that can be used pharmaceutically. Of course, the appropriate formulation is dependent upon the route of administration chosen.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for

example, parabens, chlorobutanol, phenol, ascorbic acid, thimerosal, and the like. In many cases, it will be preferable to include isotonic agents, for example, sugars, polyalcohols such as mannitol and sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilisation. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible pharmaceutically acceptable carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser, which contains a suitable propellant, e.g., a gas such as carbon dioxide, or a nebuliser.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The active compounds can be prepared with pharmaceutically acceptable carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated, each unit containing a predetermined quantity of active compound calculated to produce the desired therapeutic effect in association with the required pharmaceutical carrier. The specification for the dosage unit forms of the disclosure are dictated by and directly dependent on the unique characteristics of the active compound and the particular therapeutic effect to be achieved.

In therapeutic applications, the dosages of the pharmaceutical compositions used in accordance with the disclosure vary depending on the agent, the age, weight, and clinical condition of the recipient patient, and the experience and judgment of the clinician or practitioner administering the therapy, among other factors affecting the selected dosage. Generally, the dose should be sufficient to result in slowing, and preferably regressing, the symptoms of the disease or disorder disclosed herein and also preferably causing complete regression of the disease or disorder. Dosages can range from about 0.01 mg/kg per day to about 5000 mg/kg per day. An effective amount of a pharmaceutical agent is that which provides an objectively identifiable improvement as noted by the clinician or other qualified observer. Improvement in survival and growth indicates regression. As used herein, the term "dosage effective manner" refers to amount of an active compound to produce the desired biological effect in a subject or cell.

It is to be understood that the pharmaceutical compositions can be included in a container, pack, or dispenser together with instructions for administration.

It is to be understood that, for the compounds of the present disclosure being capable of further forming salts, all of these forms are also contemplated within the scope of the claimed disclosure.

As used herein, the term "pharmaceutically acceptable salts" refer to derivatives of the compounds of the present disclosure wherein the parent compound is modified by making acid or base salts thereof. Examples of pharmaceutically acceptable salts include, but are not limited to, mineral or organic acid salts of basic residues such as amines, alkali or

organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2-acetoxybenzoic, 2-hydroxyethane sulfonic, acetic, ascorbic, benzene sulfonic, benzoic, bicarbonic, carbonic, citric, edetic, ethane disulfonic, 1,2-ethane sulfonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulfonic, maleic, malic, mandelic, methane sulfonic, napsylic, nitric, oxalic, pamoic, pantothenic, phenylacetic, phosphoric, polygalacturonic, propionic, salicylic, stearic, subacetic, succinic, sulfamic, sulfanilic, sulfuric, tannic, tartaric, toluene sulfonic, and the commonly occurring amine acids, e.g., glycine, alanine, phenylalanine, arginine, etc.

In some embodiments, the pharmaceutically acceptable salt is a sodium salt, a potassium salt, a calcium salt, a magnesium salt, a diethylamine salt, a choline salt, a meglumine salt, a benzathine salt, a tromethamine salt, an ammonia salt, an arginine salt, or a lysine salt.

Other examples of pharmaceutically acceptable salts include hexanoic acid, cyclopentane propionic acid, pyruvic acid, malonic acid, 3-(4-hydroxybenzoyl)benzoic acid, cinnamic acid, 4-chlorobenzenesulfonic acid, 2-naphthalenesulfonic acid, 4-toluenesulfonic acid, camphorsulfonic acid, 4-methylbicyclo-[2.2.2]-oct-2-ene-1-carboxylic acid, 3-phenylpropionic acid, trimethylacetic acid, tertiary butylacetic acid, muconic acid, and the like. The present disclosure also encompasses salts formed when an acidic proton present in the parent compound either is replaced by a metal ion, e.g., an alkali metal ion, an alkaline earth ion, or an aluminum ion, or coordinates with an organic base such as ethanolamine, diethanolamine, triethanolamine, tromethamine, N-methylglucamine, and the like. In the salt form, it is understood that the ratio of the compound to the cation or anion of the salt can be 1:1, or any ratio other than 1:1, e.g., 3:1, 2:1, 1:2, or 1:3.

The compounds, or pharmaceutically acceptable salts thereof, are administered orally, nasally, transdermally, pulmonary, inhalationally, buccally, sublingually, intraperitoneally, subcutaneously, intramuscularly, intravenously, rectally, intrapleurally, intrathecally and parenterally. In one embodiment, the compound is administered orally. One skilled in the art will recognise the advantages of certain routes of administration.

The dosage regimen utilising the compounds is selected in accordance with a variety of factors including type, species, age, weight, sex and medical condition of the patient; the severity of the condition to be treated, the route of administration; the renal and hepatic function of the patient; and the particular compound or salt thereof employed. An ordinarily skilled physician or veterinarian can readily determine and prescribe the effective amount of the drug required to prevent, counter, or arrest the progress of the condition. An ordinarily skilled physician or veterinarian can readily determine and prescribe the effective amount of the drug required to counter or arrest the progress of the condition.

Techniques for formulation and administration of the disclosed compounds of the disclosure can be found in Remington: the Science and Practice of Pharmacy, 19th edition, Mack Publishing Co, Easton, Pa. (1995) In an embodiment, the compounds described herein, and the pharmaceutically acceptable salts thereof, are used in pharmaceutical preparations in combination with a pharmaceutically acceptable carrier or diluent. Suitable pharmaceutically acceptable carriers include inert solid fillers or diluents and sterile aqueous or organic solutions. The compounds will be present in such pharmaceutical compositions in amounts sufficient to provide the desired dosage amount in the range described herein.

All percentages and ratios used herein, unless otherwise indicated, are by weight. Other features and advantages of the present disclosure are apparent from the different examples. The provided examples illustrate different components and methodology useful in practicing the present disclosure. The examples do not limit the claimed disclosure. Based on the present disclosure the skilled artisan can identify and employ other components and methodology useful for practicing the present disclosure.

**In** the synthetic schemes described herein, compounds may be drawn with one particular configuration for simplicity. Such particular configurations are not to be construed as limiting the disclosure to one or another isomer, tautomer, regioisomer or stereoisomer, nor does it exclude mixtures of isomers, tautomers, regioisomers or stereoisomers, however, it will be understood that a given isomer, tautomer, regioisomer or stereoisomer may have a higher level of activity than another isomer, tautomer, regioisomer or stereoisomer.

Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow.

As use herein, the phrase "compound of the disclosure", "degraders of the disclosure", "degraders", refers to those compounds which are disclosed herein, both generically and specifically.

**Compounds of the disclosure**

In one aspect, the present disclosure provides, inter alia, compounds of Formula (I):

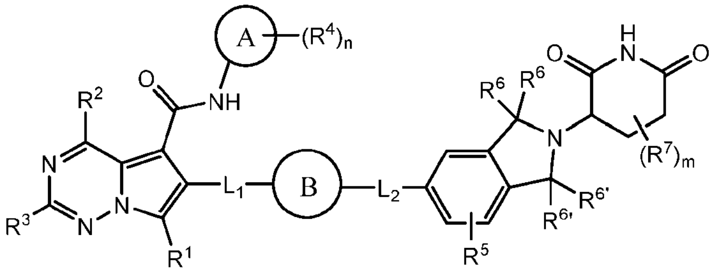

(I),

or a pharmaceutically acceptable salt thereof,
wherein

$R^1$ is $C_1$-$C_6$ alkyl, $C_3$-$C_8$ cycloalkyl, heterocycloalkyl, aryl, or heteroaryl, wherein the alkyl, cycloalkyl, heterocycloalkyl, aryl or heteroaryl is optionally substituted with one or more, e.g., 1, 2, 3, 4, or more, $R^8$;
A is aryl or heteroaryl, wherein the aryl or heteroaryl is optionally substituted with one or more, e.g., 1, 2, 3, 4, or more, $R^9$;
B is heterocycloalkyl or $C_3$-$C_8$ cycloalkyl;
$L_1$ is $C_1$-$C_6$ alkyl, $C_2$-$C_6$ alkenyl, or $C_2$-$C_6$ alkynyl;
$L_2$ is -C(O)-$(CH_2)_p$-(4- to 12-heterocycloalkyl)-; -C(O)-$(OCH_2CH_2)_p$-(4- to 12-heterocycloalkyl)-; -C(O)NH-$(CH_2)_p$-(4- to 12-heterocycloalkyl)-; -C(O)NH-$(CH_2CH_2O)_p$-(4- to 12-heterocycloalkyl)-; -C(O)-$(CH_2CH_2O)_p$-(4- to 12-heterocycloalkyl)-; -$(OCH_2CH_2)_p$-(4- to 12-heterocycloalkyl)-; -C(O)-$(CH_2)_p$-(4- to 12-heterocycloalkyl)-C(O)-; or -$(CH_2)_p$-(4- to 12-heterocycloalkyl)-C(O)-;
$R^2$ is $NR^{10}R^{11}$;
$R^3$ is H, halogen, $NH_2$, or $C_1$-$C_4$ alkyl;
each $R^4$ is independently $C_1$-$C_6$ alkyl, $C_2$-$C_6$ alkenyl, or $C_2$-$C_6$ alkynyl, wherein the alkyl, alkenyl, or alkynyl is optionally substituted with one or more, e.g., 1, 2, 3, 4, or more, $C_1$-$C_6$ alkoxy, $C_1$-$C_6$ thioalkyl, $NH_2$, -NH($C_1$-$C_6$ alkyl), or-N($C_1$-$C_6$ alkyl)($C_1$-$C_6$ alkyl); or
two $R^4$, on adjacent carbons taken together, can combine to form $C_3$-$C_8$ cycloalkyl, heterocycloalkyl, aryl, or heteroaryl;
$R^5$ is H, OH, CN, $NO_2$. or $C_1$-$C_4$ alkyl;
each $R^6$ or $R^{6'}$ is H; or
two $R^6$ can combine to form an oxo group; or
two $R^{6'}$ can combine to form an oxo group;
$R^7$, $R^8$, and $R^9$ are each independently H, halogen, CN, $NO_2$, OH, $NH_2$, $C_1$-$C_6$ alkyl, $C_2$-$C_6$ alkenyl, $C_2$-$C_6$ alkynyl, $C_3$-$C_8$ cycloalkyl, heterocycloalkyl, aryl, or heteroaryl; or
two $R^7$on adjacent carbons taken together, can combine to form $C_3$-$C_8$ cycloalkyl, heterocycloalkyl, aryl, or heteroaryl;
$R^{10}$ and $R^{11}$ are independently H or $C_1$-$C_4$ alkyl;
m is an integer from 0 to 2;
n is an integer from 0 to 4; and

p is an integer from 1 to 8.

In some embodiments, A, B, $L_1$, $L_2$, $R_1$, $R_2$, $R_3$, $R_4$, $R_5$, $R_6$, $R_{6'}$, $R_7$, m, and n are described herein as set forth in the disclosure.

In some embodiments, the disclosure relates to compounds of Formula I(a):

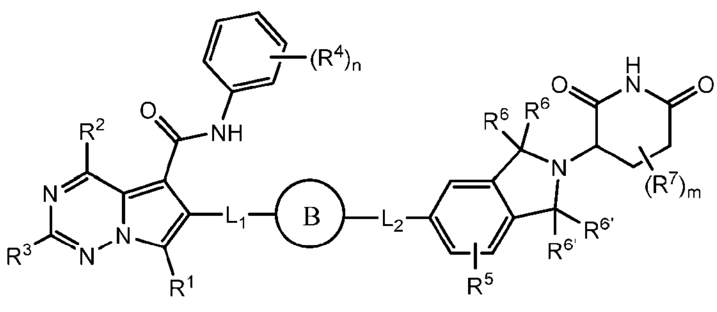

I(a),

or a pharmaceutically acceptable salt thereof.

In other embodiments, the compounds of the disclosure are of Formula I(b):

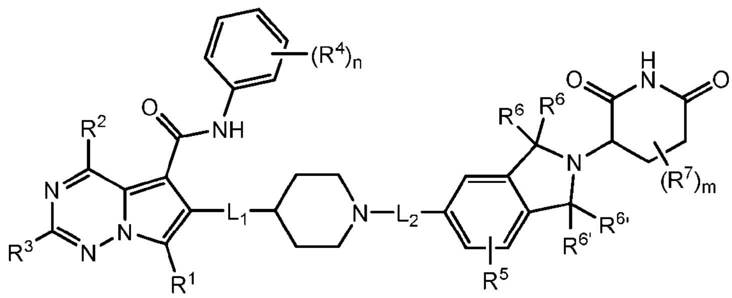

I(b),

or a pharmaceutically acceptable salt thereof.

In other embodiments, the compounds of the disclosure are of Formula I(c):

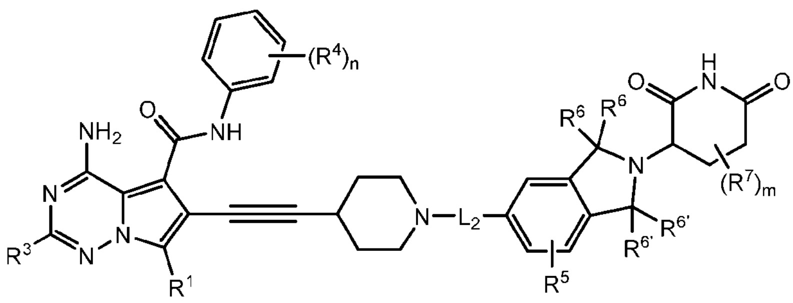

I(c),

or a pharmaceutically acceptable salt thereof.

In other embodiments, the compounds of the disclosure are of Formula I(d):

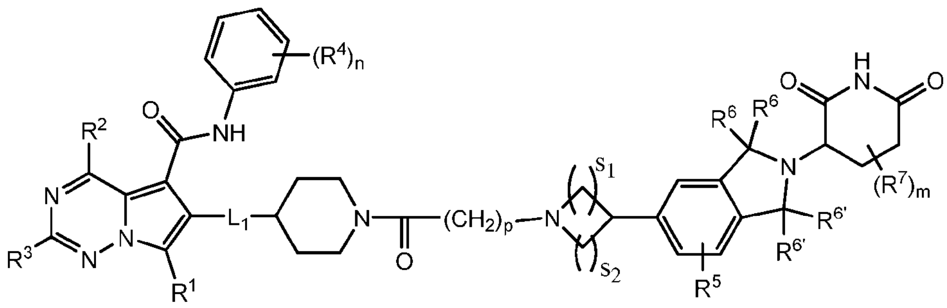

I(d),

or a pharmaceutically acceptable salt thereof,
wherein $s_1$ and $s_2$ are independently integers from 1 to 2.

In other embodiments, the compounds of the disclosure are of Formula I(e):

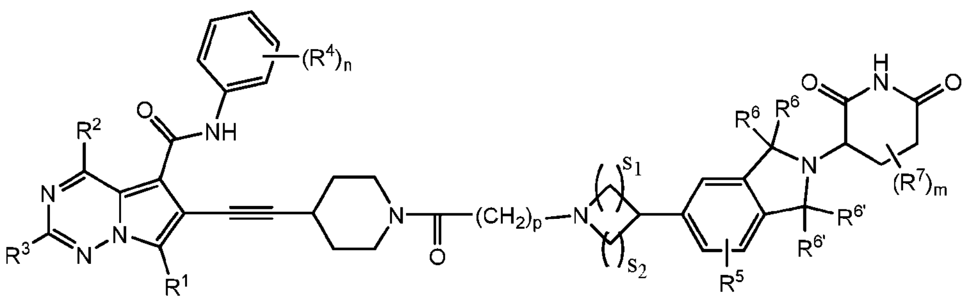

I(e),

or a pharmaceutically acceptable salt thereof, wherein $s_1$ and $s_2$ are independently integers from 1 to 2.

In yet other embodiments, the compounds of the disclosure are of Formula I(f):

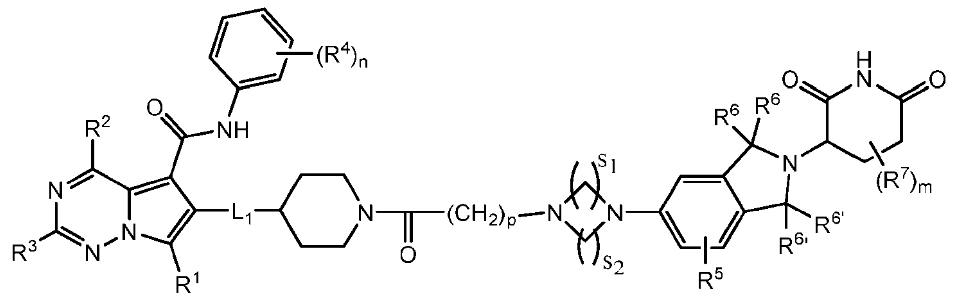

I(f),

or a pharmaceutically acceptable salt thereof,
wherein $s_1$ and $s_2$ are independently integers from 1 to 2.

In other embodiments, the compounds of the disclosure are of Formula I(g):

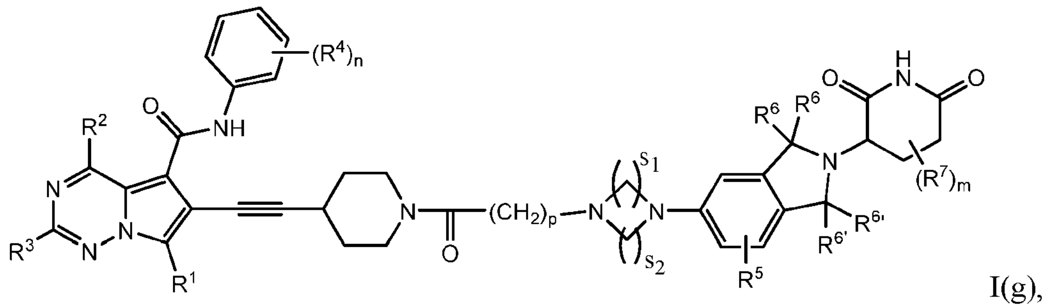

I(g),

or a pharmaceutically acceptable salt thereof, wherein $s_1$ and $s_2$ are independently integers from 1 to 2.

In other embodiments, the compounds of the disclosure are of Formula I(h):

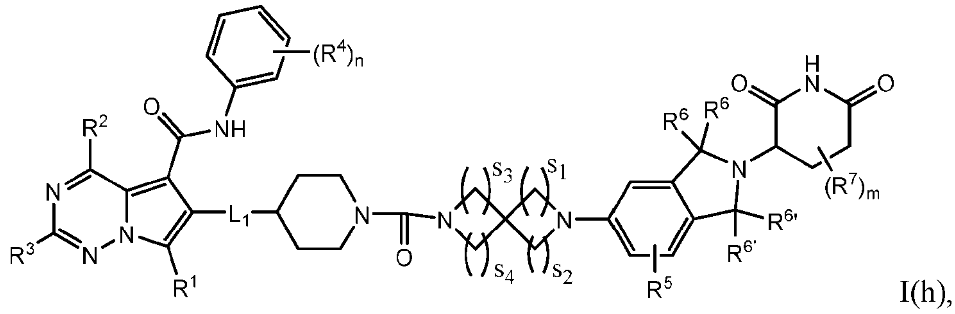

I(h),

or a pharmaceutically acceptable salt thereof, wherein $s_1$, $s_2$, $s_3$, and $s_4$ are independently integers from 1 to 2.

In certain embodiments of the compounds of the disclosure, or a pharmaceutically acceptable salt thereof, $R^1$ is $C_1$-$C_6$ alkyl, $C_3$-$C_8$ cycloalkyl, heterocycloalkyl, aryl, or heteroaryl. In other embodiments, $R^1$ is $C_1$-$C_6$ alkyl, $C_3$-$C_8$ cycloalkyl, heterocycloalkyl or aryl. In other embodiments, $R^1$ is $C_1$-$C_6$ alkyl, $C_3$-$C_8$ cycloalkyl, or heterocycloalkyl. In other embodiments, $R^1$ is $C_1$-$C_6$ alkyl or $C_3$-$C_8$ cycloalkyl. In other embodiments, $R^1$ is $C_1$-$C_6$ alkyl. In other embodiments, $R^1$ is $C_3$-$C_8$ cycloalkyl. In other embodiments, $R^1$ heterocycloalkyl. In other embodiments, $R^1$ is aryl. In other embodiments, $R^1$ is heteroaryl. In futher embodiments, $R^1$ is $C_1$-$C_6$ alkyl optionally substituted with one or more $R^8$. In other embodiments, $R^1$ is $C_3$-$C_8$ cycloalkyl optionally substituted with one or more $R^8$. In further embodiments, $R^1$ is heterocycloalkyl optionally substituted with one or more $R^8$. In further embodiments, $R^1$ is aryl optionally substituted with one or more $R^8$. In further embodiments, $R^1$ is heteroaryl optionally substituted with one or more $R^8$.

In some embodiments, $R^8$ is H, halogen, CN, $NO_2$, OH, $NH_2$, $C_1$-$C_6$ alkyl, $C_2$-$C_6$ alkenyl, $C_2$-$C_6$ alkynyl, $C_3$-$C_8$ cycloalkyl, heterocycloalkyl, aryl, or heteroaryl. In other embodiments, $R^8$ is H, halogen, CN, $NO_2$, OH, $NH_2$, $C_1$-$C_6$ alkyl, $C_2$-$C_6$ alkenyl, $C_2$-$C_6$ alkynyl, $C_3$-$C_8$ cycloalkyl, heterocycloalkyl or aryl. In other embodiments, $R^8$ is H, halogen, CN, $NO_2$, OH, $NH_2$, $C_1$-$C_6$ alkyl, $C_2$-$C_6$ alkenyl, $C_2$-$C_6$ alkynyl, $C_3$-$C_8$ cycloalkyl or heterocycloalkyl. In other embodiments, $R^8$ is H, halogen, CN, $NO_2$, OH, $NH_2$, $C_1$-$C_6$ alkyl, $C_2$-$C_6$ alkenyl, $C_2$-$C_6$ alkynyl or $C_3$-$C_8$ cycloalkyl. In other embodiments, $R^8$ is H, halogen, CN, $NO_2$, OH, $NH_2$, $C_1$-$C_6$ alkyl, $C_2$-$C_6$ alkenyl or $C_2$-$C_6$ alkynyl. In other embodiments, $R^8$ is H, halogen, CN, $NO_2$, OH, $NH_2$, $C_1$-$C_6$ alkyl or $C_2$-$C_6$ alkenyl. In other embodiments, $R^8$ is H, halogen, CN, $NO_2$, OH, $NH_2$ or $C_1$-$C_6$ alkyl. In other embodiments, $R^8$ is H, halogen, CN, $NO_2$, OH or $NH_2$. In other embodiments, $R^8$ is H, halogen, CN, $NO_2$ or OH. In

other embodiments, $R^8$ is H, halogen, CN or $NO_2$. In other embodiments, $R^8$ is H, halogen or CN. In other embodiments, $R^8$ is H or halogen. In further embodiments, $R^8$ is H. In further embodiments, $R^8$ is halogen. In further embodiments, $R^8$ is CN. In further embodiments, $R^8$ is $NO_2$. In further embodiments, $R^8$ is OH. In further embodiments, $R^8$ is $NH_2$. In further embodiments, $R^8$ is $C_1$-$C_6$ alkyl. In further embodiments, $R^8$ is $C_2$-$C_6$ alkenyl. In further embodiments, $R^8$ is $C_2$-$C_6$ alkynyl. In further embodiments, $R^8$ is $C_3$-$C_8$ cycloalkyl. In further embodiments, $R^8$ is heterocycloalkyl. In further embodiments, $R^8$ is aryl. In further embodiments, $R^8$ is heteroaryl.

In some embodiments of the compounds of the disclosure, or a pharmaceutically acceptable salt thereof, A is aryl or heteroaryl. In other embodiments, A is aryl. In other embodiments, A is heteroaryl. In other embodiments, A is aryl optionally substituted with one or more $R^9$. In other embodiments, A is heteroaryl optionally substituted with one or more $R^9$.

In some embodiments, $R^9$ is H, halogen, CN, $NO_2$, OH, $NH_2$, $C_1$-$C_6$ alkyl, $C_2$-$C_6$ alkenyl, $C_2$-$C_6$ alkynyl, $C_3$-$C_8$ cycloalkyl, heterocycloalkyl, aryl, or heteroaryl. In other embodiments, $R^9$ is H, halogen, CN, $NO_2$, OH, $NH_2$, $C_1$-$C_6$ alkyl, $C_2$-$C_6$ alkenyl, $C_2$-$C_6$ alkynyl, $C_3$-$C_8$ cycloalkyl, heterocycloalkyl or aryl. In other embodiments, $R^9$ is H, halogen, CN, $NO_2$, OH, $NH_2$, $C_1$-$C_6$ alkyl, $C_2$-$C_6$ alkenyl, $C_2$-$C_6$ alkynyl, $C_3$-$C_8$ cycloalkyl or heterocycloalkyl. In other embodiments, $R^9$ is H, halogen, CN, $NO_2$, OH, $NH_2$, $C_1$-$C_6$ alkyl, $C_2$-$C_6$ alkenyl, $C_2$-$C_6$ alkynyl or $C_3$-$C_8$ cycloalkyl. In other embodiments, $R^9$ is H, halogen, CN, $NO_2$, OH, $NH_2$, $C_1$-$C_6$ alkyl, $C_2$-$C_6$ alkenyl, or $C_2$-$C_6$ alkynyl. In other embodiments, $R^9$ is H, halogen, CN, $NO_2$, OH, $NH_2$, $C_1$-$C_6$ alkyl, or $C_2$-$C_6$ alkenyl. In other embodiments, $R^9$ is H, halogen, CN, $NO_2$, OH, $NH_2$, or $C_1$-$C_6$ alkyl. In other embodiments, $R^9$ is H, halogen, CN, $NO_2$, OH, or $NH_2$. In other embodiments, $R^9$ is H, halogen, CN, $NO_2$, or OH. In other embodiments, $R^9$ is H, halogen, CN or $NO_2$. In other embodiments, $R^9$ is H, halogen or CN. In other embodiments, $R^9$ is H or halogen. In further embodiments, $R^9$ is H. In further embodiments, $R^9$ is halogen. In further embodiments, $R^9$ is CN. In further embodiments, $R^9$ is $NO_2$. In further embodiments, $R^9$ is OH. In further embodiments, $R^9$ is $NH_2$. In further embodiments, $R^9$ is $C_1$-$C_6$ alkyl. In further embodiments, $R^9$ is $C_2$-$C_6$ alkenyl. In further embodiments, $R^9$ is $C_2$-$C_6$ alkynyl. In further embodiments, $R^9$ is $C_3$-$C_8$ cycloalkyl. In further embodiments, $R^9$ is heterocycloalkyl. In further embodiments, $R^9$ is aryl. In further embodiments, $R^9$ is heteroaryl.

In certain embodiments of the compounds of the disclosure, or a pharmaceutically acceptable salt thereof, B is heterocycloalkyl or $C_3$-$C_8$ cycloalkyl. In further embodiments, B is heterocycloalkyl. In further embodiments, B is $C_3$-$C_8$ cycloalkyl.

In some embodiments of the compounds of the disclosure, or a pharmaceutically acceptable salt thereof, $L_1$ is $C_1$-$C_6$ alkyl, $C_2$-$C_6$ alkenyl, or $C_2$-$C_6$ alkynyl. In other embodiments, $L_1$ is $C_1$-$C_6$ alkyl or $C_2$-$C_6$ alkenyl. In other embodiments, $L_1$ is $C_1$-$C_6$ alkyl. In other embodiments, $L_1$ is $C_2$-$C_6$ alkenyl. In other embodiments, $L_1$ is $C_2$-$C_6$ alkynyl.

In some embodiments of the compounds of the disclosure, or a pharmaceutically acceptable salt thereof, $L_2$ is is -C(O)-$(CH_2)_p$-(4- to 12-heterocycloalkyl)-, -C(O)-$(OCH_2CH_2)_p$-(4- to 12-heterocycloalkyl)-, -C(O)NH-$(CH_2)_p$-(4- to 12-heterocycloalkyl)-, - C(O)NH-$(CH_2CH_2O)_p$-(4- to 12-heterocycloalkyl)-, -C(O)-$(CH_2CH_2O)_p$-(4- to 12-heterocycloalkyl)-, -$(OCH_2CH_2)_p$-(4- to 12-heterocycloalkyl)-, -C(O)-$(CH_2)_p$-(4- to 12-heterocycloalkyl)-C(O)-, or -$(CH_2)_p$-(4- to 12-heterocycloalkyl)-C(O)-. In further embodiments, $L_2$ may be -C(O)-$(CH_2)_p$-(4- to 12-heterocycloalkyl)-, -C(O)-$(OCH_2CH_2)_p$-(4- to 12-heterocycloalkyl)-, -C(O)NH-$(CH_2)_p$-(4- to 12-heterocycloalkyl)-, -C(O)NH-$(CH_2CH_2O)_p$-(4- to 12-heterocycloalkyl)-, -C(O)-$(CH_2CH_2O)_p$-(4- to 12-heterocycloalkyl)-, -$(OCH_2CH_2)_p$-(4- to 12-heterocycloalkyl)-, or -C(O)-$(CH_2)_p$-(4- to 12-heterocycloalkyl)-C(O)-. In further embodiments, $L_2$ may be -C(O)-$(CH_2)_p$-(4- to 12-heterocycloalkyl)-, -C(O)-$(OCH_2CH_2)_p$-(4- to 12-heterocycloalkyl)-, -C(O)NH-$(CH_2)_p$-(4- to 12-heterocycloalkyl)-, -C(O)NH-$(CH_2CH_2O)_p$-(4- to 12-heterocycloalkyl)-, -C(O)-$(CH_2CH_2O)_p$-(4- to 12-heterocycloalkyl)-, or - $(OCH_2CH_2)_p$-(4- to 12-heterocycloalkyl)-. In further embodiments, $L_2$ may be -C(O)-$(CH_2)_p$-(4-to 12-heterocycloalkyl)-, -C(O)-$(OCH_2CH_2)_p$-(4- to 12-heterocycloalkyl)-, -C(O)NH-$(CH_2)_p$-(4- to 12-heterocycloalkyl)-, -C(O)NH-$(CH_2CH_2O)_p$-(4- to 12-heterocycloalkyl)-, or -C(O)-$(CH_2CH_2O)_p$-(4- to 12-heterocycloalkyl)-. In further embodiments, $L_2$ may be -C(O)-$(CH_2)_p$-(4-to 12-heterocycloalkyl)-, -C(O)-$(OCH_2CH_2)_p$-(4- to 12-heterocycloalkyl)-, -C(O)NH-$(CH_2)_p$-(4- to 12-heterocycloalkyl)-, or -C(O)NH-$(CH_2CH_2O)_p$-(4- to 12-heterocycloalkyl)-. In further embodiments, $L_2$ may be, -C(O)-$(CH_2)_p$-(4- to 12-heterocycloalkyl)-, -C(O)-$(OCH_2CH_2)_p$-(4-to 12-heterocycloalkyl)-, or -C(O)NH-$(CH_2)_p$-(4- to 12-heterocycloalkyl)-. In further embodiments, $L_2$ may be -C(O)-$(CH_2)_p$-(4- to 12-heterocycloalkyl)- or -C(O)-$(OCH_2CH_2)_p$-(4-to 12-heterocycloalkyl)-. Yet, in further embodiments, $L_2$ may be -C(O)-$(CH_2)_p$-(4- to 12-heterocycloalkyl)-. Yet, in further embodiments, $L_2$ may be -C(O)-$(OCH_2CH_2)_p$-(4- to 12-heterocycloalkyl)-. Yet, in further embodiments, $L_2$ may be -C(O)NH-$(CH_2)_p$-(4- to 12-heterocycloalkyl)-. Yet, in further embodiments, $L_2$ may be -C(O)NH-$(CH_2CH_2O)_p$-(4- to 12-heterocycloalkyl)-. Yet, in further embodiments, $L_2$ may be -C(O)-$(CH_2CH_2O)_p$-(4- to 12-heterocycloalkyl)-. Yet, in further embodiments, $L_2$ may be -$(OCH_2CH_2)_p$-(4- to 12-heterocycloalkyl)-. Yet, in further embodiments, $L_2$ may be -C(O)-$(CH_2)_p$-(4- to 12-heterocycloalkyl)-C(O)-. Yet, in further embodiments, $L_2$ may be -$(CH_2)_p$-(4- to 12-heterocycloalkyl)-C(O)-.

In certain embodiments of the compounds of the disclosure, or a pharmaceutically acceptable salt thereof, $R^2$ is independently $NR^{10}R^{11}$.

In some embodiments of the compounds of the disclosure, or a pharmaceutically acceptable salt thereof, $R^{10}$ is independently H or $C_1$-$C_4$ alkyl. In other embodiments, $R^{10}$ is H. In other embodiments, $R^{10}$ is $C_1$-$C_4$ alkyl.

In some embodiments of the compounds of the disclosure, or a pharmaceutically acceptable salt thereof, $R^{11}$ is

independently H or $C_1$-$C_4$ alkyl. In other embodiments, $R^{11}$ is H. In other embodiments, $R^{11}$ is $C_1$-$C_4$ alkyl.

In certain embodiments of the compounds of the disclosure, or a pharmaceutically acceptable salt thereof, $R^3$ is H, halogen, $NH_2$, or $C_1$-$C_4$ alkyl. In further embodiments, $R^3$ is H, halogen or $NH_2$. In further embodiments, $R^3$ is H or halogen. Yet, in further embodiments, $R^3$ is H. Yet, in further embodiments, $R^3$ is halogen. Yet, in further embodiments, $R^3$ is $NH_2$. Yet, in further embodiments, $R^3$ is $C_1$-$C_4$ alkyl.

In some embodiments of the compounds of the disclosure, or a pharmaceutically acceptable salt thereof, $R^4$ is $C_1$-$C_6$ alkyl, $C_2$-$C_6$ alkenyl, or $C_2$-$C_6$ alkynyl. In other embodiments, $R^4$ is $C_1$-$C_6$ alkyl or $C_2$-$C_6$ alkenyl. In other embodiments, $R^4$ is $C_1$-$C_6$ alkyl. In other embodiments, $R^4$ is $C_2$-$C_6$ alkenyl. In other embodiments, $R^4$ is $C_2$-$C_6$ alkynyl.

In further embodiments, $R^4$ is $C_1$-$C_6$ alkyl optionally substituted with one or more $C_1$-$C_6$ alkoxy, $C_1$-$C_6$ thioalkyl, $NH_2$, -NH($C_1$-$C_6$ alkyl), or -N($C_1$-$C_6$ alkyl)($C_1$-$C_6$ alkyl). In further embodiments, $R^4$ is $C_1$-$C_6$ alkyl optionally substituted with one or more $C_1$-$C_6$ alkoxy, $C_1$-$C_6$ thioalkyl, $NH_2$, or -NH($C_1$-$C_6$ alkyl). In further embodiments, $R^4$ is $C_1$-$C_6$ alkyl optionally substituted with one or more $C_1$-$C_6$ alkoxy, $C_1$-$C_6$ thioalkyl or $NH_2$. In further embodiments, $R^4$ is $C_1$-$C_6$ alkyl optionally substituted with one or more $C_1$-$C_6$ alkoxy or $C_1$-$C_6$ thioalkyl. Yet, in further embodiments, $R^4$ is $C_1$-$C_6$ alkyl optionally substituted with one or more $C_1$-$C_6$ alkoxy. Yet, in further embodiments, $R^4$ is $C_1$-$C_6$ alkyl optionally substituted with one or more $C_1$-$C_6$ thioalkyl. Yet, in further embodiments, $R^4$ is $C_1$-$C_6$ alkyl optionally substituted with one or more $NH_2$. Yet, in further embodiments, $R^4$ is $C_1$-$C_6$ alkyl optionally substituted with one or more - NH($C_1$-$C_6$ alkyl). Yet, in further embodiments, $R^4$ is $C_1$-$C_6$ alkyl optionally substituted with one or more -N($C_1$-$C_6$ alkyl)($C_1$-$C_6$ alkyl).

In further embodiments, $R^4$ is $C_2$-$C_6$ alkenyl optionally substituted with one or more $C_1$-$C_6$ alkoxy, $C_1$-$C_6$ thioalkyl, $NH_2$, -NH($C_1$-$C_6$ alkyl), or -N($C_1$-$C_6$ alkyl)($C_1$-$C_6$ alkyl). In further embodiments, $R^4$ is $C_2$-$C_6$ alkenyl optionally substituted with one or more $C_1$-$C_6$ alkoxy, $C_1$-$C_6$ thioalkyl, $NH_2$, or -NH($C_1$-$C_6$ alkyl). In further embodiments, $R^4$ is $C_2$-$C_6$ alkenyl optionally substituted with one or more $C_1$-$C_6$ alkoxy, $C_1$-$C_6$ thioalkyl or $NH_2$. In further embodiments, $R^4$ is $C_2$-$C_6$ alkenyl optionally substituted with one or more $C_1$-$C_6$ alkoxy or $C_1$-$C_6$ thioalkyl. Yet, in further embodiments, $R^4$ is $C_2$-$C_6$ alkenyl optionally substituted with one or more $C_1$-$C_6$ alkoxy. Yet, in further embodiments, $R^4$ is $C_2$-$C_6$ alkenyl optionally substituted with one or more $C_1$-$C_6$ thioalkyl. Yet, in further embodiments, $R^4$ is $C_2$-$C_6$ alkenyl optionally substituted with one or more $NH_2$. Yet, in further embodiments, $R^4$ is $C_2$-$C_6$ alkenyl optionally substituted with one or more -NH($C_1$-$C_6$ alkyl). Yet, in further embodiments, $R^4$ is $C_2$-$C_6$ alkenyl optionally substituted with one or more -N($C_1$-$C_6$ alkyl)($C_1$-$C_6$ alkyl).

In further embodiments, $R^4$ is $C_2$-$C_6$ alkynyl optionally substituted with one or more $C_1$-$C_6$ alkoxy, $C_1$-$C_6$ thioalkyl, $NH_2$, -NH($C_1$-$C_6$ alkyl), or -N($C_1$-$C_6$ alkyl)($C_1$-$C_6$ alkyl). In further embodiments, $R^4$ is $C_2$-$C_6$ alkynyl optionally substituted with one or more $C_1$-$C_6$ alkoxy, $C_1$-$C_6$ thioalkyl, $NH_2$, or -NH($C_1$-$C_6$ alkyl). In further embodiments, $R^4$ is $C_2$-$C_6$ alkynyl optionally substituted with one or more $C_1$-$C_6$ alkoxy, $C_1$-$C_6$ thioalkyl or $NH_2$. In further embodiments, $R^4$ is $C_2$-$C_6$ alkynyl optionally substituted with one or more $C_1$-$C_6$ alkoxy or $C_1$-$C_6$ thioalkyl. Yet, in further embodiments, $R^4$ is $C_2$-$C_6$ alkynyl optionally substituted with one or more $C_1$-$C_6$ alkoxy. Yet, in further embodiments, $R^4$ is $C_2$-$C_6$ alkynyl optionally substituted with one or more $C_1$-$C_6$ thioalkyl. Yet, in further embodiments, $R^4$ is $C_2$-$C_6$ alkynyl optionally substituted with one or more $NH_2$. Yet, in further embodiments, $R^4$ is $C_2$-$C_6$ alkynyl optionally substituted with one or more -NH($C_1$-$C_6$ alkyl). Yet, in further embodiments, $R^4$ is $C_2$-$C_6$ alkynyl optionally substituted with one or more -N($C_1$-$C_6$ alkyl)($C_1$-$C_6$ alkyl).

In further embodiments still of the compounds of the disclosure, or a pharmaceutically acceptable salt thereof, two $R^4$, on adjacent carbons taken together, can combine to form $C_3$-$C_8$ cycloalkyl, heterocycloalkyl, aryl, or heteroaryl. In other embodiments, two $R^4$, on adjacent carbons taken together, can combine to form $C_3$-$C_8$ cycloalkyl, heterocycloalkyl, or aryl. In other embodiments, two $R^4$, on adjacent carbons taken together, can combine to form $C_3$-$C_8$ cycloalkyl or heterocycloalkyl. In other embodiments, two $R^4$, on adjacent carbons taken together, can combine to form $C_3$-$C_8$ cycloalkyl. In other embodiments, two $R^4$, on adjacent carbons taken together, can combine to form heterocycloalkyl. In other embodiments, two $R^4$, on adjacent carbons taken together, can combine to form aryl. In other embodiments, two $R^4$, on adjacent carbons taken together, can combine to form heteroaryl.

In certain embodiments of the compounds of the disclosure, or a pharmaceutically acceptable salt thereof, $R^5$ is independently H, OH, CN, $NO_2$ or $C_1$-$C_4$ alkyl. In further embodiments, $R^5$ is H, OH, CN, or $NO_2$. In further embodiments, $R^5$ is H, OH or CN. In further embodiments, $R^5$ is H or OH. In further embodiments yet, $R^5$ is H. In further embodiments yet, $R^5$ is OH. In further embodiments yet, $R^5$ is CN. In further embodiments yet, $R^5$ is $NO_2$. In further embodiments yet, $R^5$ is $C_1$-$C_4$ alkyl.

In certain embodiments of the compounds of the disclosure, or a pharmaceutically acceptable salt thereof, $R^6$ is independently H.

In some embodiments of the compounds of the disclosure, or a pharmaceutically acceptable salt thereof, $R^{6'}$ is independently H.

In certain embodiments of the compounds of the disclosure, or a pharmaceutically acceptable salt thereof, two $R^6$ can combine to form an oxo group.

In some embodiments of the compounds of the disclosure, or a pharmaceutically acceptable salt thereof, two $R^{6'}$ can combine to form an oxo group.

In some embodiments of the compounds of the disclosure, or a pharmaceutically acceptable salt thereof, $R^7$ is H, halogen, CN, $NO_2$, OH, $NH_2$, $C_1$-$C_6$ alkyl, $C_2$-$C_6$ alkenyl, $C_2$-$C_6$ alkynyl, $C_3$-$C_8$ cycloalkyl, heterocycloalkyl, aryl, or

heteroaryl. In other embodiments, $R^7$ is H, halogen, CN, $NO_2$, OH, $NH_2$, $C_1$-$C_6$ alkyl, $C_2$-$C_6$ alkenyl, $C_2$-$C_6$ alkynyl, $C_3$-$C_8$ cycloalkyl, heterocycloalkyl or aryl. In other embodiments, $R^7$ is H, halogen, CN, $NO_2$, OH, $NH_2$, $C_1$-$C_6$ alkyl, $C_2$-$C_6$ alkenyl, $C_2$-$C_6$ alkynyl, $C_3$-$C_8$ cycloalkyl or heterocycloalkyl. In other embodiments, $R^7$ is H, halogen, CN, $NO_2$, OH, $NH_2$, $C_1$-$C_6$ alkyl, $C_2$-$C_6$ alkenyl, $C_2$-$C_6$ alkynyl or $C_3$-$C_8$ cycloalkyl. In other embodiments, $R^7$ is H, halogen, CN, $NO_2$, OH, $NH_2$, $C_1$-$C_6$ alkyl, $C_2$-$C_6$ alkenyl or $C_2$-$C_6$ alkynyl. In other embodiments, $R^7$ is H, halogen, CN, $NO_2$, OH, $NH_2$, $C_1$-$C_6$ alkyl or $C_2$-$C_6$ alkenyl. In other embodiments, $R^7$ is H, halogen, CN, $NO_2$, OH, $NH_2$ or $C_1$-$C_6$ alkyl. In other embodiments, $R^7$ is H, halogen, CN, $NO_2$, OH or $NH_2$. In other embodiments, $R^7$ is H, halogen, CN, $NO_2$ or OH. In other embodiments, $R^7$ is H, halogen, CN or $NO_2$. In other embodiments, $R^7$ is H, halogen or CN. In other embodiments, $R^7$ is H or halogen. In further embodiments, $R^7$ is H. In other embodiments, $R^7$ is halogen. In further embodiments, $R^7$ is CN. In further embodiments, $R^7$ is $NO_2$. In further embodiments, $R^7$ is OH. In further embodiments, $R^7$ is $NH_2$. In further embodiments, $R^7$ is $C_1$-$C_6$ alkyl. In further embodiments, $R^7$ is $C_2$-$C_6$ alkenyl. In further embodiments, $R^7$ is $C_2$-$C_6$ alkynyl. In further embodiments, $R^7$ is $C_3$-$C_8$ cycloalkyl. In further embodiments, $R^7$ is heterocycloalkyl. In further embodiments, $R^7$ is aryl. In further embodiments, $R^7$ is heteroaryl.

In further embodiments still of the compounds of the disclosure, or a pharmaceutically acceptable salt thereof, two $R^7$, on adjacent carbons taken together, can combine to form $C_3$-$C_8$ cycloalkyl, heterocycloalkyl, aryl, or heteroaryl. In other embodiments, two $R^7$, on adjacent carbons taken together, can combine to form $C_3$-$C_8$ cycloalkyl, heterocycloalkyl, or aryl. In other embodiments, two $R^7$, on adjacent carbons taken together, can combine to form $C_3$-$C_8$ cycloalkyl or heterocycloalkyl. In other embodiments, two $R^7$, on adjacent carbons taken together, can combine to form $C_3$-$C_8$ cycloalkyl. In other embodiments, two $R^7$, on adjacent carbons taken together, can combine to form heterocycloalkyl. In other embodiments, two $R^7$, on adjacent carbons taken together, can combine to form aryl. In other embodiments, two $R^7$, on adjacent carbons taken together, can combine to form heteroaryl.

In further embodiments of the compounds of the instant disclosure, or a pharmaceutically acceptable salt thereof, m is at each occurrence, 0, 1, or 2. In other embodiments, m is 0 or 1. In other embodiments, m is 0 or 2. In other embodiments, m is 1 or 2. In other embodiments, m is 0. In other embodiments, m is 1. In other embodiments, m is 2.

In yet other embodiments of the disclosure, n is, independently at each occurrence, 0, 1, 2, 3 or 4. In yet other embodiments, n is 1 or 2. In yet other embodiments, n is 1 or 3. In yet other embodiments, n is 1 or 4. In yet other embodiments, n is 2 or 3. In yet other embodiments, n is 2 or 4. In yet other embodiments, n is 3 or 4. In yet other embodiments, n is 1. In yet other embodiments, n is 2. In yet other embodiments, n is 3. In yet other embodiments, n is 4. In yet other embodiments, n is 0.

In yet other embodiments of the disclosure, p is, independently at each occurrence, 0, 1, 2, 3, 4, 5, 6, 7 or 8. In yet other embodiments of the disclosure, p is 1 or 2. In yet other embodiments of the disclosure, p is 1 or 3. In yet other embodiments of the disclosure, p is 1 or 4. In yet other embodiments of the disclosure, p is 1 or 5. In yet other embodiments of the disclosure, p is 1 or 6. In yet other embodiments of the disclosure, p is 1 or 7. In yet other embodiments of the disclosure, p is 1 or 8. In yet other embodiments of the disclosure, p is 2 or 3. In yet other embodiments of the disclosure, p is 2 or 4. In yet other embodiments of the disclosure, p is 2 or 5. In yet other embodiments of the disclosure, p is 2 or 6. In yet other embodiments of the disclosure, p is 2 or 7. In yet other embodiments of the disclosure, p is 2 or 8. In yet other embodiments of the disclosure, p is 3 or 4. In yet other embodiments of the disclosure, p is 3 or 5. In yet other embodiments of the disclosure, p is 3 or 6. In yet other embodiments of the disclosure, p is 3 or 7. In yet other embodiments of the disclosure, p is 3 or 8. In yet other embodiments of the disclosure, p is 4 or 5. In yet other embodiments of the disclosure, p is 4 or 6. In yet other embodiments of the disclosure, p is 4 or 7. In yet other embodiments of the disclosure, p is 4 or 8. In yet other embodiments of the disclosure, p is 5 or 6. In yet other embodiments of the disclosure, p is 5 or 7. In yet other embodiments of the disclosure, p is 5 or 8. In yet other embodiments of the disclosure, p is 6 or 7. In yet other embodiments of the disclosure, p is 6 or 8. In yet other embodiments of the disclosure, p is 7 or 8. In yet other embodiments of the disclosure, p is 8. In yet other embodiments of the disclosure, p is 7. In yet other embodiments of the disclosure, p is 6. In yet other embodiments of the disclosure, p is 5. In yet other embodiments of the disclosure, p is 4. In yet other embodiments of the disclosure, p is 3. In yet other embodiments of the disclosure, p is 2. In yet other embodiments of the disclosure, p is 1. In yet other embodiments of the disclosure, p is 0.

In some embodiments, suitable compounds of the disclosure include:

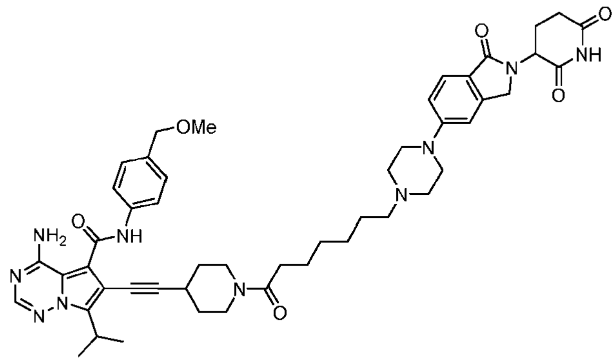

(4-amino-6-((1-(7-(4-(2-(2,6-dioxopiperidin-3-yl)-1-oxoisoindolin-5-yl)piperazin-1-yl)heptanoyl)piperidin-4-yl)ethynyl)-7-isopropyl-N-(4-(methoxymethyl)phenyl)pyrrolo[2,1-f][1,2,4]triazine-5-carboxamide);

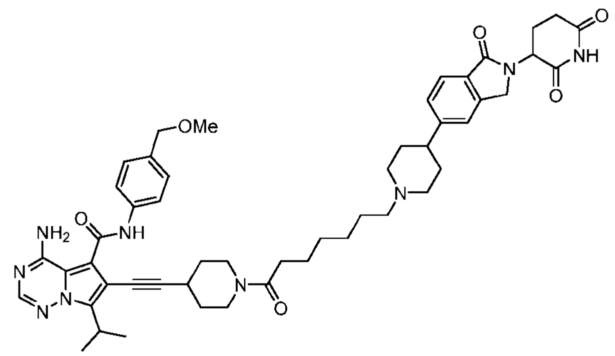

(4-amino-6-((1-(7-(4-(2-(2,6-dioxopiperidin-3-yl)-1-oxoisoindolin-5-yl)piperidin-1-yl)heptanoyl)piperidin-4-yl)ethynyl)-7-isopropyl-N-(4-(methoxymethyl)phenyl)pyrrolo[2,1-f][1,2,4]triazine-5-carboxamide);

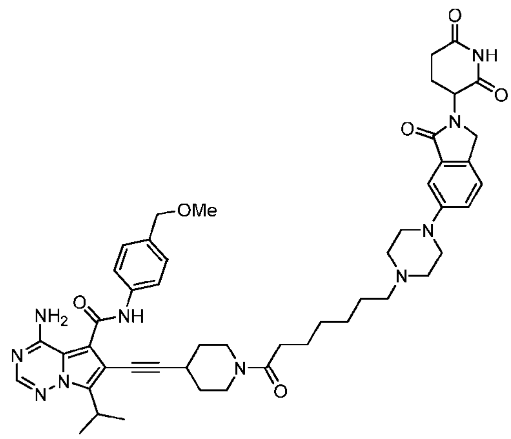

(4-amino-6-((1-(7-(4-(2-(2,6-dioxopiperidin-3-yl)-3-oxoisoindolin-5-yl)piperazin-1-yl)heptanoyl)piperidin-4-yl)ethynyl)-7-isopropyl-N-(4-(methoxymethyl)phenyl)pyrrolo[2,1-f][1,2,4]triazine-5-carboxamide);

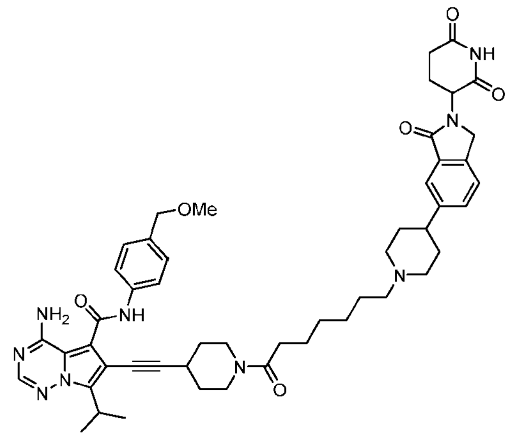

(4-amino-6-((1-(7-(4-(2-(2,6-dioxopiperidin-3-yl)-3-oxoisoindolin-5-yl)piperidin-1-yl)heptanoyl)piperidin-4-yl)ethynyl)-7-isopropyl-N-(4-(methoxymethyl)phenyl)pyrrolo[2,1-f][1,2,4]triazine-5-carboxamide);

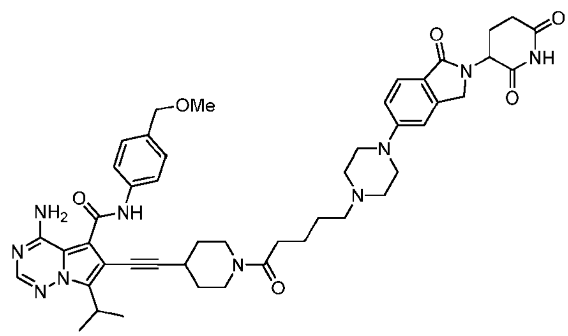

(4-amino-6-((1-(5-(4-(2-(2,6-dioxopiperidin-3-yl)-1-oxoisoindolin-5-yl)piperazin-1-yl)pentanoyl)piperidin-4-yl)ethynyl)-7-isopropyl-N-(4-(methoxymethyl)phenyl)pyrrolo[2,1-f][1,2,4]triazine-5-carboxamide);

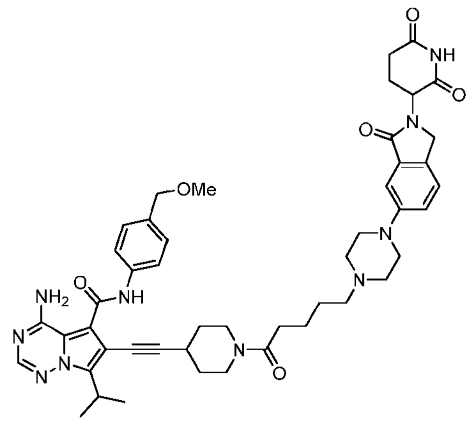

(4-amino-6-((1-(5-(4-(2-(2,6-dioxopiperidin-3-yl)-3-oxoisoindolin-5-yl)piperazin-1-yl)pentanoyl)piperidin-4-yl)ethynyl)-7-isopropyl-N-(4-(methoxymethyl)phenyl)pyrrolo[2,1-f][1,2,4]triazine-5-carboxamide);

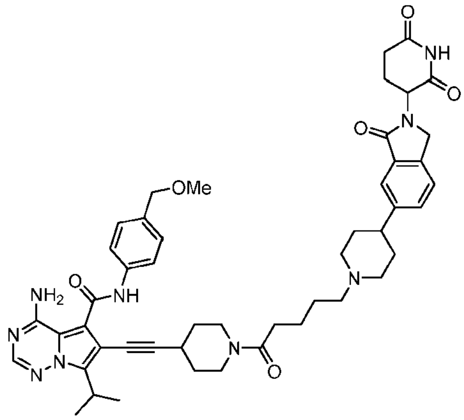

(4-amino-6-((1-(5-(4-(2-(2,6-dioxopiperidin-3-yl)-3-oxoisoindolin-5-yl)piperidin-1-yl)pentanoyl)piperidin-4-yl)ethynyl)-7-isopropyl-N-(4-(methoxymethyl)phenyl)pyrrolo[2,1-f][1,2,4]triazine-5-carboxamide);

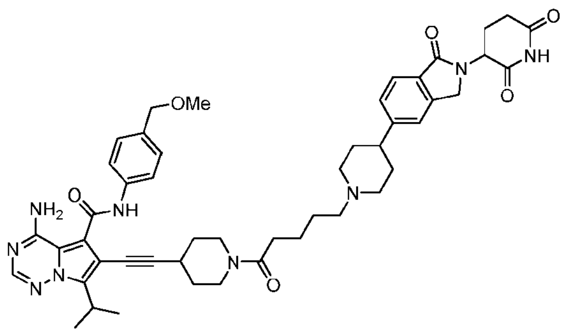

(4-amino-6-((1-(5-(4-(2-(2,6-dioxopiperidin-3-yl)-1-oxoisoindolin-5-yl)piperidin-1-yl)pentanoyl)piperidin-4-yl)ethynyl)-7-isopropyl-N-(4-(methoxymethyl)phenyl)pyrrolo[2,1-f][1,2,4]triazine-5-carboxamide);

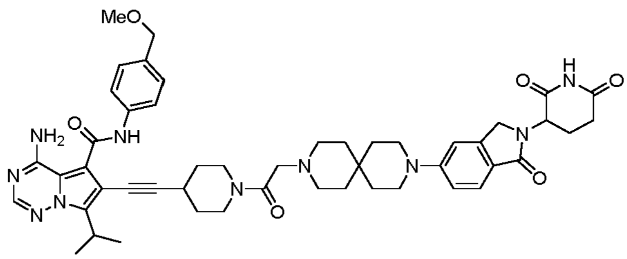

(4-amino-6-((1-(2-(9-(2-(2,6-dioxopiperidin-3-yl)-1-oxoisoindolin-5-yl)-3,9-diazaspiro[5.5]undecan-3-yl)acetyl)piperidin-4-yl)ethynyl)-7-isopropyl-N-(4-(methoxymethyl)phenyl)pyrrolo[2,1-f][1,2,4]triazine-5-carboxamide); or

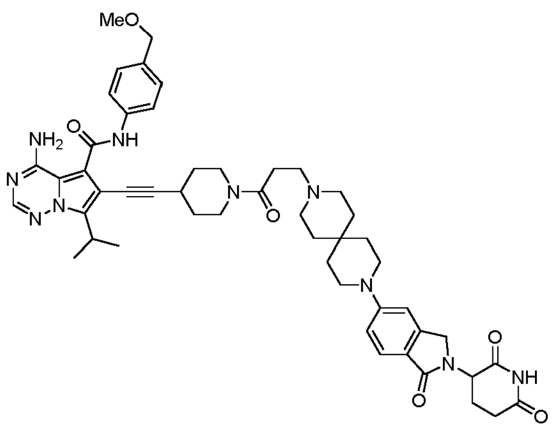

(4-amino-6-((1-(3-(9-(2-(2,6-dioxopiperidin-3-yl)-1-oxoisoindolin-5-yl)-3,9-diazaspiro[5.5]undecan-3-yl)propanoyl)piperidin-4-yl)ethynyl)-7-isopropyl-N-(4-(methoxymethyl)phenyl)pyrrolo[2,1-f][1,2,4]triazine-5-carboxamide), or a pharmaceutically acceptable salt thereof.

In some embodiments, the compound is a pharmaceutically acceptable salt of any one of the compounds described herein.

In some aspects, the present disclosure provides a compound being an isotopic derivative (e.g., isotopically labeled compound) of any one of the compounds of the Formulae disclosed herein.

In some embodiments, the compound is an isotopic derivative of any one of the compounds described in Table 3 and pharmaceutically acceptable salts thereof.

In some embodiments, the compound is an isotopic derivative of any one of the compounds described in Table 3.

It is understood that the isotopic derivative can be prepared using any of a variety of art-recognised techniques. For example, the isotopic derivative can generally be prepared by carrying out the procedures disclosed in the Schemes and/or in the Examples described herein, by substituting an isotopically labeled reagent for a non-isotopically labeled reagent.

In some embodiments, the isotopic derivative is a deuterium labeled compound.

In some embodiments, the isotopic derivative is a deuterium labeled compound of any one of the compounds of the Formulae disclosed herein.

The term "isotopic derivative", as used herein, refers to a derivative of a compound in which one or more atoms are isotopically enriched or labelled. For example, an isotopic derivative of a compound of Formula (I) is isotopically enriched with regard to, or labelled with, one or more isotopes as compared to the corresponding compound of Formula (I). In some embodiments, the isotopic derivative is enriched with regard to, or labelled with, one or more atoms selected from $^{2}H$, $^{13}C$, $^{14}C$, $^{15}N$, $^{18}O$, $^{29}Si$, $^{31}P$, and $^{34}S$. In some embodiments, the isotopic derivative is a deuterium labeled compound (i.e., being enriched with $^{2}H$ with regard to one or more atoms thereof). In some embodiments, the compound is a $^{18}F$ labeled compound. In some embodiments, the compound is a $^{123}I$ labeled compound, a $^{124}I$ labeled compound, a $^{125}I$ labeled compound, a $^{129}I$ labeled compound, a $^{131}I$ labeled compound, a $^{135}I$ labeled compound, or any combination thereof. In some embodiments, the compound is a $^{33}S$ labeled compound, a $^{34}S$ labeled compound, a $^{35}S$ labeled compound, a $^{36}S$ labeled compound, or any combination thereof.

It is understood that the $^{18}F$, $^{123}I$, $^{124}I$, $^{125}I$, $^{129}I$, $^{131}I$, $^{135}I$, $^{32}S$, $^{34}S$, $^{35}S$, and/or $^{36}S$ labeled compound, can be prepared using any of a variety of art-recognised techniques. For example, the deuterium labeled compound can generally be prepared by carrying out the procedures disclosed in the Schemes and/or in the Examples described herein, by substituting a $^{18}F$, $^{125}I$, $^{124}I$, $^{125}I$, $^{129}I$, $^{131}I$, $^{135}I$, $^{32}S$, $^{34}S$, $^{35}S$, and/or $^{36}S$ labeled reagent for a non-isotope labeled reagent.

A compound of the invention or a pharmaceutically acceptable salt thereof that contains one or more of the aforementioned $^{18}F$, $^{123}I$, $^{124}I$, $^{125}I$, $^{129}I$, $^{131}I$, $^{135}I$, $^{32}S$, $^{34}S$, $^{35}S$, and $^{36}S$ atom(s) is within the scope of the invention. Further, substitution with isotope (e.g., $^{18}F$, $^{123}I$, $^{124}I$, $^{125}I$, $^{129}I$, $^{131}I$, $^{135}I$, $^{32}S$, $^{34}S$, $^{35}S$, and/or $^{36}S$) may afford certain therapeutic advantages resulting from greater metabolic stability, e.g., increased in vivo half-life or reduced dosage requirements.

For the avoidance of doubt it is to be understood that, where in this specification a group is qualified by "described herein", the said group encompasses the first occurring and broadest definition as well as each and all of the particular definitions for that group.

A suitable pharmaceutically acceptable salt of a compound of the disclosure is, for example, an acid-addition salt of a compound of the disclosure which is sufficiently basic, for example, an acid-addition salt with, for example, an inorganic or organic acid, for example hydrochloric, hydrobromic, sulfuric, phosphoric, trifluoroacetic, formic, citric methane sulfonate or maleic acid. In addition, a suitable pharmaceutically acceptable salt of a compound of the disclosure which is sufficiently acidic is an alkali metal salt, for example a sodium or potassium salt, an alkaline earth metal salt, for example a calcium or magnesium salt, an ammonium salt or a salt with an organic base which affords a pharmaceutically acceptable cation, for example a salt with methylamine, dimethylamine, diethylamine, trimethylamine, piperidine, morpholine or tris-(2-hydroxyethyl)amine.

It will be understood that the compounds of any one of the Formulae disclosed herein and any pharmaceutically acceptable salts thereof, comprise stereoisomers, mixtures of stereoisomers, polymorphs of all isomeric forms of said compounds.

As used herein, the term "isomerism" means compounds that have identical molecular formulae but differ in the sequence of bonding of their atoms or in the arrangement of their atoms in space. Isomers that differ in the arrangement of their atoms in space are termed "stereoisomers." Stereoisomers that are not mirror images of one another are termed "diastereoisomers," and stereoisomers that are non-superimposable mirror images of each other are termed "enantiomers" or sometimes optical isomers. A mixture containing equal amounts of individual enantiomeric forms of opposite chirality is termed a "racemic mixture."

As used herein, the term "chiral center" refers to a carbon atom bonded to four nonidentical substituents.

As used herein, the term "chiral isomer" means a compound with at least one chiral centre. Compounds with more

than one chiral centre may exist either as an individual diastereomer or as a mixture of diastereomers, termed "diastereomeric mixture." When one chiral centre is present, a stereoisomer may be characterised by the absolute configuration (R or S) of that chiral centre. Absolute configuration refers to the arrangement in space of the substituents attached to the chiral centre. The substituents attached to the chiral centre under consideration are ranked in accordance with the Sequence Rule of Cahn, Ingold and Prelog. (Cahn et al., Angew. Chem. Inter. Edit. 1966, 5, 385; errata 511; Cahn et al., Angew. Chem. 1966, 78, 413. Cahn and Ingold, J. Chem Soc. 1951 (London), 612; Cahn et al., Experientia 1956, 12, 81; Cahn, J. Chem. Educ. 1964, 41, 116).

As used herein, the term "geometric isomer" means the diastereomers that owe their existence to hindered rotation about double bonds or a cycloalkyl linker (e.g., 1,3-cyclobutyl). These configurations are differentiated in their names by the prefixes cis and trans, or Z and E, which indicate that the groups are on the same or opposite side of the double bond in the molecule according to the Cahn-Ingold-Prelog rules.

It is to be understood that the compounds of the present disclosure may be depicted as different chiral isomers or geometric isomers. It is also to be understood that when compounds have chiral isomeric or geometric isomeric forms, all isomeric forms are intended to be included in the scope of the present disclosure, and the naming of the compounds does not exclude any isomeric forms, it being understood that not all isomers may have the same level of activity.

It is to be understood that the structures and other compounds discussed in this disclosure include all atropic isomers thereof. It is also to be understood that not all atropic isomers may have the same level of activity.

As used herein, the term "tautomer" is one of two or more structural isomers that exist in equilibrium and is readily converted from one isomeric form to another. This conversion results in the formal migration of a hydrogen atom accompanied by a switch of adjacent conjugated double bonds. Tautomers exist as a mixture of a tautomeric set in solution. In solutions where tautomerisation is possible, a chemical equilibrium of the tautomers will be reached. The exact ratio of the tautomers depends on several factors, including temperature, solvent and pH. The concept of tautomers that are interconvertible by tautomerisations is called tautomerism. Of the various types of tautomerism, that are possible, two are commonly observed. In keto-enol tautomerism a simultaneous shift of electrons and a hydrogen atom occurs. Ring-chain tautomerism arises as a result of the aldehyde group (-CHO) in a sugar chain molecule reacting with one of the hydroxy groups (-OH) in the same molecule to give it a cyclic (ring-shaped) form as exhibited by glucose.

It is to be understood that the compounds of the present disclosure may be depicted as different tautomers. It should also be understood that when compounds have tautomeric forms, all tautomeric forms are intended to be included in the scope of the present disclosure, and the naming of the compounds does not exclude any tautomer form. It will be understood that certain tautomers may have a higher level of activity than others.

Compounds that have the same molecular formula but differ in the nature or sequence of bonding of their atoms or the arrangement of their atoms in space are termed "isomers". Isomers that differ in the arrangement of their atoms in space are termed "stereoisomers". Stereoisomers that are not mirror images of one another are termed "diastereomers" and those that are non-superimposable mirror images of each other are termed "enantiomers". When a compound has an asymmetric centre, for example, it is bonded to four different groups, a pair of enantiomers is possible. An enantiomer can be characterised by the absolute configuration of its asymmetric centre and is described by the R- and S-sequencing rules of Cahn and Prelog, or by the manner in which the molecule rotates the plane of polarised light and designated as dextrorotatory or levorotatory (i.e., as (+) or (-)-isomers respectively). A chiral compound can exist as either individual enantiomer or as a mixture thereof. A mixture containing equal proportions of the enantiomers is called a "racemic mixture".

The compounds of this disclosure may possess one or more asymmetric centres, such compounds can therefore be produced as individual (R)- or (S)-stereoisomers or as mixtures thereof. Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art (see discussion in Chapter 4 of "Advanced Organic Chemistry", 4th edition J. March, John Wiley and Sons, New York, 2001), for example by synthesis from optically active starting materials or by resolution of a racemic form. Some of the compounds of the disclosure may have geometric isomeric centres (E- and Z-isomers). It is to be understood that the present disclosure encompasses all optical, diastereoisomers and geometric isomers and mixtures thereof that possess inflammasome inhibitory activity.

It is to be understood that the compounds of any Formula described herein include the compounds themselves, as well as their salts, if applicable. A salt, for example, can be formed between an anion and a positively charged group (e.g., amino) on a substituted compound disclosed herein. Suitable anions include chloride, bromide, iodide, sulfate, bisulfate, sulfamate, nitrate, phosphate, citrate, methanesulfonate, trifluoroacetate, glutamate, glucuronate, glutarate, malate, maleate, succinate, fumarate, tartrate, tosylate, salicylate, lactate, naphthalenesulfonate, and acetate (e.g., trifluoroacetate).

As used herein, the term "pharmaceutically acceptable anion" refers to an anion suitable for forming a pharmaceutically acceptable salt. Likewise, a salt can also be formed between a cation and a negatively charged group (e.g., carboxylate) on a substituted compound disclosed herein. Suitable cations include sodium ion, potassium ion,

magnesium ion, calcium ion, and an ammonium cation such as tetramethylammonium ion or diethylamine ion. The substituted compounds disclosed herein also include those salts containing quaternary nitrogen atoms.

It is to be understood that the compounds of the present disclosure, for example, the salts of the compounds, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

As used herein, the term "solvate" means solvent addition forms that contain either stoichiometric or non-stoichiometric amounts of solvent Some compounds have a tendency to trap a fixed molar ratio of solvent molecules in the crystalline solid state, thus forming a solvate. If the solvent is water the solvate formed is a hydrate, and if the solvent is alcohol, the solvate formed is an alcoholate. Hydrates are formed by the combination of one or more molecules of water with one molecule of the substance in which the water retains its molecular state as H2O.

As used herein, the term "analog" refers to a chemical compound that is structurally similar to another but differs slightly in composition (as in the replacement of one atom by an atom of a different element or in the presence of a particular functional group, or the replacement of one functional group by another functional group). Thus, an analog is a compound that is similar or comparable in function and appearance, but not in structure or origin to the reference compound.

As used herein, the term "derivative" refers to compounds that have a common core structure and are substituted with various groups as described herein.

It is also to be understood that certain compounds of any one of the Formulae disclosed herein may exist in solvated as well as unsolvated forms such as, for example, hydrated forms. A suitable pharmaceutically acceptable solvate is, for example, a hydrate such as hemi-hydrate, a mono-hydrate, a di-hydrate or a tri-hydrate. It is to be understood that the disclosure encompasses all such solvated forms that possess inflammasome inhibitory activity.

It is also to be understood that certain compounds of any one of the Formulae disclosed herein may exhibit polymorphism, and that the disclosure encompasses all such forms, or mixtures thereof, which possess inflammasome inhibitory activity. It is generally known that crystalline materials may be analysed using conventional techniques such as X-Ray Powder Diffraction analysis, Differential Scanning Calorimetry, Thermal Gravimetric Analysis, Diffuse Reflectance Infrared Fourier Transform (DRIFT) spectroscopy, Near Infrared (NIR) spectroscopy, solution and/or solid state nuclear magnetic resonance spectroscopy. The water content of such crystalline materials may be determined by Karl Fischer analysis.

Compounds of any one of the Formulae disclosed herein may exist in a number of different tautomeric forms and references to compounds of Formula (I) include all such forms. For the avoidance of doubt, where a compound can exist in one of several tautomeric forms, and only one is specifically described or shown, all others are nevertheless embraced by Formula (I). Examples of tautomeric forms include keto-, enol-, and enolate-forms, as in, for example, the following tautomeric pairs keto/enol, imine/enamine, amide/imino alcohol, amidine/amidine, nitroso/oxime, thioketone/enethiol, and nitro/aci-nitro.

A prodrug may be used to alter the physical properties and/or the pharmacokinetic properties of a compound. A prodrug can be formed when the compound contains a suitable group or substituent to which a property-modifying group can be attached Examples of prodrugs include derivatives containing in vivo cleavable alkyl or acyl substitutents at the ester or amide group.

Accordingly, the present disclosure includes those compounds of any one of the Formulae disclosed herein as defined hereinbefore when made available by organic synthesis and when made available within the human or animal body by way of cleavage of a prodrug thereof. Accordingly, the present disclosure includes those compounds of any one of the Formulae disclosed herein that are produced by organic synthetic means and also such compounds that are produced in the human or animal body by way of metabolism of a precursor compound, that is a compound of any one of the Formulae disclosed herein may be a synthetically-produced compound or a metabolically-produced compound.

A suitable pharmaceutically acceptable prodrug of a compound is one that is based on reasonable medical judgment as being suitable for administration to the human or animal body without undesirable pharmacological activities and without undue toxicity. Various forms of prodrug have been described, for example in the following documents a) Methods in Enzymology, Vol 42, p. 309-396, edited by K. Widder, et al. (Academic Press, 1985); b) Design of Pro-drugs, edited by H. Bundgaard, (Elsevier, 1985); c) A Textbook of Drug Design and Development, edited by Krogsgaard-Larsen and H. Bundgaard, Chapter 5 "Design and Application of Pro-drugs", by H. Bundgaard p. 113-191 (1991); d) H. Bundgaard, Advanced Drug Delivery Reviews, 8, 1-38 (1992); e) H. Bundgaard, et al., Journal of Pharmaceutical Sciences, 77, 285 (1988); f) N Kakeya, et al., Chem. Pharm Bull., 32, 692 (1984); g) T. Higuchi and V. Stella, "Pro-Drugs as Novel Delivery Systems", A.C.S. Symposium Series, Volume 14; and h) E. Roche (editor), "Bioreversible Carriers in Drug Design", Pergamon Press, 1987.

A suitable pharmaceutically acceptable prodrug of a compound that possesses a hydroxy group is, for example, an in vivo cleavable ester or ether thereof. An in vivo cleavable ester or ether of a compound containing a hydroxy group is, for example, a pharmaceutically acceptable ester or ether which is cleaved in the human or animal body to produce the parent hydroxy compound. Suitable pharmaceutically acceptable ester forming groups for a hydroxy group include

inorganic esters such as phosphate esters (including phosphoramidic cyclic esters). Further suitable pharmaceutically acceptable ester forming groups for a hydroxy group include C1-C10, alkanoyl groups such as acetyl, benzoyl, phenylacetyl and substituted benzoyl and phenylacetyl groups, C1-C10 alkoxycarbonyl groups such as ethoxycarbonyl, N,N-(C1-C6 alkyl)2carbamoyl, 2-dialkylaminoacetyl and 2-carboxyacetyl groups. Examples of ring substituents on the phenylacetyl and benzoyl groups include aminomethyl, N-alkylaminomethyl, N,N-dialkylaminomethyl, morpholinomethyl, piperazin-1-ylmethyl and 4-(C1-C4 alkyl)piperazin-1-ylmethyl. Suitable pharmaceutically acceptable ether forming groups for a hydroxy group include $\alpha$-acyloxyalkyl groups such as acetoxymethyl and pivaloyloxymethyl groups.

A suitable pharmaceutically acceptable prodrug of a compound that possesses a carboxy group is, for example, an in vivo cleavable amide thereof, for example an amide formed with an amine such as ammonia, a C1-4alkylamine such as methylamine, a (C1-(C4 alkyl)2-amine such as dimethylamine, N-ethyl-N-methylamine or diethylamine, a C1-C4 alkoxy-C2-C4 alkylamine such as 2-methoxyethylamine, a phenyl-C1-C4 alkylamine such as benzylamine and amino acids such as glycine or an ester thereof.

A suitable pharmaceutically acceptable prodrug of a compound that possesses an amino group is, for example, an in vivo cleavable amide derivative thereof. Suitable pharmaceutically acceptable amides from an amino group include, for example an amide formed with C1-C10 alkanoyl groups such as an acetyl, benzoyl, phenylacetyl and substituted benzoyl and phenylacetyl groups Examples of ring substituents on the phenylacetyl and benzoyl groups include aminomethyl, N-alkylaminomethyl, N,N-dialkylaminomethyl, morpholinomethyl, piperazin-1-ylmethyl and 4-(C1-C4 alkyl)piperazin-1-ylmethyl.

The in vivo effects of a compound of any one of the Formulae disclosed herein may be exerted in part by one or more metabolites that are formed within the human or animal body after administration of a compound of any one of the Formulae disclosed herein.

**Methods of Use**

A compound described herein, or a pharmaceutically acceptable salt thereof, can be used in an effective amount to treat a patient, typically a human, in need thereof, who have a disorder mediated by RET which can be a wild-type RET or mutant RET as described generally herein. In certain embodiments a compound of the present invention degrades an additional protein, for example an aurora kinase or VEGFR2. In certain embodiments a compound of the present invention degrades RET and aurora A kinase (AURKA).

Another aspect of the present invention provides a compound as described herein, or an enantiomer, diastereomer, or stereoisomer thereof, or pharmaceutically acceptable salt, hydrate, or solvate thereof, or a pharmaceutical composition, for use in the manufacture of a medicament for treating or preventing cancer in a patient in need thereof; wherein there is a need of RET inhibition for the treatment or prevention of cancer.

In certain embodiments, the method comprises administering an effective amount of the active compound or its salt as described herein, optionally including a pharmaceutically acceptable excipient, carrier, or adjuvant (i.e., a pharmaceutically acceptable composition), or optionally in combination or alternation with another bioactive agent or combination of agents, to a patient in need thereof.

In certain embodiments, the present invention provides a method of treating any of the disorders described herein, in a patient in need thereof.

In other embodiments, the patient is administered an additional therapeutic agent. In other embodiments, the compound as described herein, and the additional therapeutic agent are administered simultaneously or sequentially.

In certain embodiments, the application provides a method of preventing any of the disorders described herein, in a patient in need thereof.

In certain embodiments, the patient is a human.

Another aspect of the present invention provides a method of treating or preventing a proliferative disease. The method comprises administering an effective amount of a pharmaceutical composition comprising a compound as described herein, or an enantiomer, diastereomer, or stereoisomer thereof, or pharmaceutically acceptable salt, hydrate, or solvate thereof and optionally a pharmaceutically acceptable carrier to a patient in need thereof.

In some embodiments, the disease is mediated by RET, for example, RET plays a role in the initiation or development of the disease.

In certain embodiments, the RET mediated disorder is a benign growth, metastasis, neoplasm, tumor, solid tumor, rhabdoid tumor, carcinoma, leukemia, cancer, abnormal cellular proliferation, an amyloid-based proteinopathy, a proteinopathy, fibrotic disorder, inflammation, arthritis, pulmonary disorders, or immune disorders.

In certain embodiments, the RET mediated disorder is a cancer that has metastasized, for example a cancer that has metastasized to the brain. In certain embodiments the RET mediated disorder is a cancer that has metastasized to the brain, lungs bone, liver, peritoneum, adrenal gland, skin, or muscle.

In certain embodiments a compound of the present invention penetrates the blood brain barrier and can be used for the treatment of a CNS involved cancer or a cancer that has metastasized to the brain.

In certain embodiments, the disease or disorder is cancer or a proliferation disease.

In certain embodiments, the RET mediated disorder is an abnormal cell proliferation, including, but not limited to, a tumor or cancer, or a myelo- or lymphoproliferative disorder such as B- or T-cell lymphomas, multiple myeloma, Waldenstrom's macroglobulinemia, Wiskott-Aldrich syndrome, or a post-transplant lymphoproliferative disorder.

In certain embodiments, the hematological cancer is acute myelogenous leukemia (AML), acute lymphoblastic leukemia (ALL), lymphoblastic T-cell leukemia, chronic myelogenous leukemia (CML), chronic lymphocytic leukemia (CLL), hairy-cell leukemia, chronic neutrophilic leukemia (CNL), acute lymphoblastic T-cell leukemia, acute monocytic leukemia, plasmacytoma, immunoblastic large cell leukemia, mantle cell leukemia, multiple myeloma, megakaryoblastic leukemia, acute megakaryocytic leukemia, promyelocytic leukemia, mixed lineage leukemia (MLL), erythroleukemia, malignant lymphoma, Hodgkins lymphoma, non-Hodgkins lymphoma, lymphoblastic T-cell lymphoma, Burkitt's lymphoma, follicular lymphoma, B cell acute lymphoblastic leukemia, diffuse large B cell lymphoma, Myc and B-Cell Leukemia (BCL)2 and/or BCL6 rearrangements/overexpression [double- and triple-hit lymphoma], myelodysplastic/myeloproliferative neoplasm, mantle cell lymphoma including bortezomib resistant mantle cell lymphoma.

Solid tumors that can be treated with the compounds described herein include, but are not limited to lung cancers, including small cell lung cancer (SCLC) and non-small cell lung cancer (NSCLC), breast cancers including inflammatory breast cancer, ER-positive breast cancer including tamoxifen resistant ER-positive breast cancer, and triple negative breast cancer, colon cancers, midline carcinomas, liver cancers, renal cancers, prostate cancers including castrate resistant prostate cancer (CRPC), brain cancers including gliomas, glioblastomas, neuroblastoma, and medulloblastoma including MYC-amplified medulloblastoma, colorectal cancers, Wilm's tumor, Ewing's sarcoma, rhabdomyosarcomas, ependymomas, head and neck cancers, melanomas, squamous cell carcinomas, ovarian cancers, pancreatic cancers including pancreatic ductal adenocarcinomas (PDAC) and pancreatic neuroendocrine tumors (PanNET), osteosarcomas, giant cell tumors of bone, thyroid cancers, bladder cancers, urothelial cancers, vulval cancers, cervical cancers, endometrial cancers, mesotheliomas, esophageal cancers, salivary gland cancers, gastric cancesr, nasopharangeal cancers, buccal cancers, cancers of the mouth, GIST (gastrointestinal stromal tumors), NUT-midline carcinomas, testicular cancers, squamous cell carcinomas, hepatocellular carcinomas (HCC), MYCN driven solid tumors, and NUT midline carcinomas (NMC).

In further embodiments, the disease or disorder is sarcoma of the bones, muscles, tendons, cartilage, nerves, fat, or blood vessels.

In further embodiments, the disease or disorder is soft tissue sarcoma, bone sarcoma, or osteosarcoma.

In further embodiments, the disease or disorder is angiosarcoma, fibrosarcoma, liposarcoma, leiomyosarcoma, Karposi's sarcoma, osteosarcoma, gastrointestinal stromal tumor, synovial sarcoma, Pleomorphic sarcoma, chondrosarcoma, Ewing's sarcoma, reticulum cell sarcoma, meningiosarcoma, botryoid sarcoma, rhabdomyosarcoma, or embryonal rhabdomyosarcoma.

In further embodiments, the disease or disorder is multiple myeloma.

In other embodiments, the disease or disorder is inflammation, arthritis, rheumatoid arthritis, spondyiarthropathies, gouty arthritis, osteoarthritis, juvenile arthritis, and other arthritic conditions, neuroinflammation, allergy, pain, neuropathic pain, fever, pulmonary disorders, lung inflammation, adult respiratory distress chronic pulmonary inflammatory disease, and chronic obstructive pulmonary disease (COPD), liver disease and nephritis, gastrointestinal conditions, inflammatory bowel disease, Crohn's disease, gastritis, irritable bowel syndrome, ulcerative colitis, ulcerative diseases, gastric ulcers, autoimmune disease, graft vs. host reaction and allograft rejections, cancer, leukemia, lymphoma, colorectal cancer, brain cancer, bone cancer, epithelial call-derived neoplasia (epithelial carcinoma), basal cell carcinoma, adenocarcinoma, gastrointestinal cancer, lip cancer, mouth cancer, esophageal cancer, small bowel cancer, stomach cancer, colon cancer, liver cancer, bladder cancer, pancreas cancer, ovarian cancer, cervical cancer, lung cancer, breast cancer, skin cancer, squamous cell and/or basal cell cancers, prostate cancer, renal cell carcinoma, and other known cancers that affect epithelial cells throughout the body, chronic myelogenous leukemia (CML), acute myeloid leukemia (AML) and acute promyelocytic leukemia (APL), angiogenesis including neoplasia, metastasis, central nervous system disorders, central nervous system disorders having an inflammatory or apoptotic component, peripheral neuropathy, or B-Cell Lymphoma.

In other embodiments, the pharmaceutical composition comprising the compound as described herein and the additional therapeutic agent are administered simultaneously or sequentially.

In other embodiments, the disease or disorder is cancer. In further embodiments, the cancer is lung cancer, colon cancer, breast cancer, prostate cancer, liver cancer, pancreas cancer, brain cancer, kidney cancer, ovarian cancer, stomach cancer, skin cancer, bone cancer, gastric cancer, breast cancer, pancreatic cancer, glioma, glioblastoma, hepatocellular carcinoma, papillary renal carcinoma, head and neck squamous cell carcinoma, leukemias, lymphomas, myelomas, solid tumors, hematological cancers or solid cancers.

In some embodiments, said method is used to treat or prevent a condition selected from autoimmune diseases, inflammatory diseases, proliferative and hyperproliferative diseases, and immunologically-mediated diseases. In other embodiments, said condition is selected from a proliferative disorder.

In certain embodiments, the RET mediated disorder is an immune disorder, including but not limited to, autoimmune disorders such as Addison disease, Celiac disease, dermatomyositis, Graves disease, thyroiditis, multiple sclerosis, pernicious anemia, reactive arthritis, lupus, or type I diabetes.

One aspect of this application provides compounds that are useful for the treatment of diseases, disorders, and conditions characterized by excessive or abnormal cell proliferation. Such diseases include, but are not limited to, a proliferative or hyperproliferative disease. Examples of proliferative and hyperproliferative diseases include, without limitation, cancer. The term "cancer" includes, but is not limited to, the following cancers: breast; ovary; cervix; prostate; testis, genitourinary tract; esophagus; larynx, glioblastoma; neuroblastoma; stomach; skin, keratoacanthoma; lung, epidermoid carcinoma, large cell carcinoma, small cell carcinoma, lung adenocarcinoma; bone; colon; colorectal; adenoma; pancreas, adenocarcinoma; thyroid, follicular carcinoma, undifferentiated carcinoma, papillary carcinoma; seminoma; melanoma; sarcoma; bladder carcinoma; liver carcinoma and biliary passages; kidney carcinoma; myeloid disorders; lymphoid disorders, Hodgkin’s, hairy cells; buccal cavity and pharynx (oral), lip, tongue, mouth, pharynx; small intestine; colonrectum, large intestine, rectum, brain and central nervous system; chronic myeloid leukemia (CML), and leukemia. The term "cancer" includes, but is not limited to, the following cancers: myeloma, lymphoma, or a cancer selected from gastric, renal, or and the following cancers: head and neck, oropharangeal, non-small cell lung cancer (NSCLC), endometrial, hepatocarcinoma, Non-Hodgkins lymphoma, and pulmonary.

The term "cancer" refers to any cancer caused by the proliferation of malignant neoplastic cells, such as tumors, neoplasms, carcinomas, sarcomas, leukemias, lymphomas and the like. For example, cancers include, but are not limited to, mesothelioma, leukemias and lymphomas such as cutaneous T-cell lymphomas (CTCL), noncutaneous peripheral T-cell lymphomas, lymphomas associated with human T-cell lymphotrophic virus (HTLV) such as adult T-cell leukemia/-lymphoma (ATLL), B-cell lymphoma, acute nonlymphocytic leukemias, chronic lymphocytic leukemia, chronic myelogenous leukemia, acute myelogenous leukemia, lymphomas, and multiple myeloma, non-Hodgkin lymphoma, acute lymphatic leukemia (ALL), chronic lymphatic leukemia (CLL), Hodgkin’s lymphoma, Burkitt lymphoma, adult T-cell leukemia lymphoma, acute-myeloid leukemia (AML), chronic myeloid leukemia (CML), or hepatocellular carcinoma. Further examples include myelodisplastic syndrome, childhood solid tumors such as brain tumors, neuroblastoma, retinoblastoma, Wilms’ tumor, bone tumors, and soft-tissue sarcomas, common solid tumors of adults such as head and neck cancers, such as oral, laryngeal, nasopharyngeal and esophageal, genitourinary cancers, such as prostate, bladder, renal, uterine, ovarian, testicular, lung cancer, such as small-cell and non-small cell, breast cancer, pancreatic cancer, melanoma and other skin cancers, stomach cancer, brain tumors, tumors related to Gorlin’s syndrome, such as medulloblastoma or meningioma, and liver cancer.

Additional exemplary forms of cancer include, but are not limited to, cancer of skeletal or smooth muscle, stomach cancer, cancer of the small intestine, rectum carcinoma, cancer of the salivary gland, endometrial cancer, adrenal cancer, anal cancer, rectal cancer, parathyroid cancer, and pituitary cancer.

Additional cancers that the compounds described herein may be useful in preventing, treating and studying are, for example, colon carcinoma, familiary adenomatous polyposis carcinoma and hereditary non-polyposis colorectal cancer, or melanoma. Further, cancers include, but are not limited to, labial carcinoma, larynx carcinoma, hypopharynx carcinoma, tongue carcinoma, salivary gland carcinoma, gastric carcinoma, adenocarcinoma, thyroid cancer (medullary and papillary thyroid carcinoma), renal carcinoma, kidney parenchyma carcinoma, cervix carcinoma, uterine corpus carcinoma, endometrium carcinoma, chorion carcinoma, testis carcinoma, urinary carcinoma, melanoma, brain tumors such as glioblastoma, astrocytoma, meningioma, medulloblastoma and peripheral neuroectodermal tumors, gall bladder carcinoma, bronchial carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma, and plasmocytoma. In one aspect of the application, the present application provides for the use of one or more compound as described herein, in the manufacture of a medicament for the treatment of cancer, including without limitation the various types of cancer disclosed herein. In some embodiments, the compounds of this application are useful for treating cancer, such as colorectal, thyroid, breast, and lung cancer; and myeloproliferative disorders, such as polycythemia vera, thrombocythemia, myeloid metaplasia with myelofibrosis, chronic myelogenous leukemia, chronic myelomonocytic leukemia, hypereosinophilic syndrome, juvenile myelomonocytic leukemia, and systemic mast cell disease. In some embodiments, the compound as described herein is useful for treating hematopoietic disorders, in particular, acute-myelogenous leukemia (AML), chronic-myelogenous leukemia (CML), acute-promyelocytic leukemia, and acute lymphocytic leukemia (ALL). In one embodiment, a compound or its corresponding pharmaceutically acceptable salt, or isotopic derivative, as described herein can be used in an effective amount to treat a host, for example a human, with a lymphoma or lymphocytic or myelocytic proliferation disorder or abnormality. For example, a compound as described herein can be administered to a host suffering from a Hodgkin’s Lymphoma or a Non-Hodgkin’s Lymphoma. For example, the host can be suffering from a Non-Hodgkin’s Lymphoma such as, but not limited to: an AIDS-Related Lymphoma; Anaplastic Large-Cell Lymphoma; Angioimmunoblastic Lymphoma; Blastic NK- Cell Lymphoma; Burkitt’s Lymphoma; Burkitt-like Lymphoma (Small Non-Cleaved Cell Lymphoma); diffuse small-cleaved cell lymphoma (DSCCL); Chronic Lymphocytic Leukemia/Small Lymphocytic Lymphoma; Cutaneous T-Cell Lymphoma; Diffuse Large B-Cell

Lymphoma; Enteropathy-Type T-Cell Lymphoma; Follicular Lymphoma; Hepatosplenic Gamma- Delta T-Cell Lymphoma; Lymphoblastic Lymphoma; Mantle Cell Lymphoma; Marginal Zone Lymphoma; Nasal T-Cell Lymphoma; Pediatric Lymphoma; Peripheral T-Cell Lymphomas; Primary Central Nervous System Lymphoma; T-Cell Leukemias; Transformed Lymphomas; Treatment-Related T-Cell Lymphomas; Langerhans cell histiocytosis; or Waldenstrom's Macrogl obulinemi a .

In another embodiment, a compound or its corresponding pharmaceutically acceptable salt, or isotopic derivative, as described herein can be used in an effective amount to treat a patient, for example a human, with a Hodgkin's lymphoma, such as, but not limited to: Nodular Sclerosis Classical Hodgkin's Lymphoma (CHL); Mixed Cellularity CHL; Lymphocyte-depletion CHL; Lymphocyte-rich CHL; Lymphocyte Predominant Hodgkin's Lymphoma; or Nodular Lymphocyte Predominant HL. This application further embraces the treatment or prevention of cell proliferative disorders such as hyperplasias, dysplasias and pre-cancerous lesions. Dysplasia is the earliest form of pre- cancerous lesion recognizable in a biopsy by a pathologist. The compounds may be administered for the purpose of preventing said hyperplasias, dysplasias or pre-cancerous lesions from continuing to expand or from becoming cancerous. Examples of pre-cancerous lesions may occur in skin, esophageal tissue, breast and cervical intra-epithelial tissue.

In certain embodiments a compound of the present invention is used to treat an abnormal cell proliferation such as a tumor or cancer that has a RET protein with a mutation, wherein the mutation is at one of the below listed amino acid sites. The mutation may, for example, be selected from one of the listed exemplary mutations in Table 1, or may be a different mutation.

**Table 1.**

| Amino Acid Site | Exemplary Mutations |
|---|---|
| G810 | G810R, G810S, G810C, G810N |
| C634 | C634W, C634R |
| M918 | M918T |
| A883 | A883F |
| E762 | E762Q |
| G691 | G691S |
| L790 | L790F |
| R749 | R749T |
| R813 | R813Q |
| S891 | S891A |
| | |
| S904 | S904A, S904F |
| V778 | V778I |
| V804 | V804L, V804M, V804E |
| Y791 | Y791F |
| Y806 | Y806H |

In certain embodiments the RET protein has two mutations selected from Table 1 above. In other embodiments the RET protein has three mutations selected from Table 1 above. In other embodiments the RET protein has four or more mutations, which may optionally be selected from Table 1 above.

In certain embodiments the tumor or cancer has a mutation in a RET protein that is a substantial or partial driver of tumor of cancer cell proliferation. In another embodiment the tumor or cancer has a RET altered protein that is not acting significantly as a driver of abnormal cell proliferation but can be used therapeutically to kill the tumor cell using a selected RET degrader as described herein.

In certain embodiments, a compound of the present invention is used to treat a tumor or cancer with a RET protein V804L mutation. In certain embodiments, a compound of the present invention is used to treat a tumor or cancer with a RET protein V804M mutation. In certain embodiments, a compound of the present invention is used to treat a tumor or cancer with a RET protein M918T mutation. In certain embodiments, a compound of the present invention is used to treat a tumor or cancer with a RET protein S891 A mutation. In certain embodiments, a compound of the present invention is used to

treat a tumor or cancer with a RET protein L790F mutation. In certain embodiments, a compound of the present invention is used treat a tumor or cancer with a RET protein E768D mutation. In certain embodiments, a compound of the present invention is used treat a tumor or cancer with a RET protein C618S mutation. In certain embodiments, a compound of the present invention is used treat a tumor or cancer with a RET protein C618R mutation. In certain embodiments, a compound of the present invention is used to treat a tumor or cancer with a RET protein 634 missense. In certain embodiments, a compound of the present invention is used to treat a tumor or cancer with a RET protein C634R mutation. In certain embodiments, a compound of the present invention is used to treat a tumor or cancer with a RET protein C634Y mutation. In certain embodiments, a compound of the present invention is used to treat a tumor or cancer with a RET protein C634G mutation. In certain embodiments a compound of the present invention, or a pharmaceutically acceptable salt thereof, is used to treat an abnormal cell proliferation such as a tumor or cancer that has a RET protein with a G810R mutation.

In certain embodiments a compound of the present invention, or a pharmaceutically acceptable salt thereof, is used to treat an abnormal cell proliferation such as a tumor or cancer that has a RET protein with a G810S mutation.

In certain embodiments a compound of the present invention, or a pharmaceutically acceptable salt thereof, is used to treat an abnormal cell proliferation such as a tumor or cancer that has a RET protein with a G810C mutation.

In certain embodiments a compound of the present invention, or a pharmaceutically acceptable salt thereof, is used to treat an abnormal cell proliferation such as a tumor or cancer that has a RET protein with a C634W mutation.

In certain embodiments a compound of the present invention, or a pharmaceutically acceptable salt thereof, is used to treat an abnormal cell proliferation such as a tumor or cancer that has a RET protein with a M918T mutation.

In certain embodiments a compound of the present invention, or a pharmaceutically acceptable salt thereof, is used to treat an abnormal cell proliferation such as a tumor or cancer that has a RET protein with a V804L mutation.

In certain embodiments a compound of the present invention, or a pharmaceutically acceptable salt thereof, is used to treat an abnormal cell proliferation such as a tumor or cancer that has a RET protein with a V804M mutation.

In certain embodiments a compound of the present invention, or a pharmaceutically acceptable salt thereof, is used to treat an abnormal cell proliferation such as a tumor or cancer that has a RET protein fused to another protein, for example a fusion selected from CCDC6-RET, NCOA4-RET, KIF5B-RET, PRKAR1A-RET, TRIM24-RET, TRIM33-RET, GOLGA5-RET, HOOK3-RET, KTN1-RET, ERC1-RET, MBD1-RET, TRIM27-RET, BRC-RET, FGFR10P-RET, PCM1-RET, AKAP13-RET, FKBP15-RET, SPECC1L-RET, TBL1XR1-RET, CUX1-RET, KIAA1468-RET, and KIAA1217-RET.

In certain embodiments a compound of the present invention, or a pharmaceutically acceptable salt thereof, is used to treat an abnormal cell proliferation such as a tumor or cancer that has a CCDC6-RET fusion. In certain embodiments a compound of the present invention, or a pharmaceutically acceptable salt thereof, is used to treat an abnormal cell proliferation such as a tumor or cancer that has a NCOA4-RET fusion.

In certain embodiments a compound of the present invention, or a pharmaceutically acceptable salt thereof, is used to treat an abnormal cell proliferation such as a tumor or cancer that has a KIF5B-RET fusion.

In accordance with the foregoing, the present application further provides a method for preventing or treating any of the diseases or disorders described above in a patient in need of such treatment, which method comprises administering to said patient a therapeutically effective amount of a compound as described herein, or an enantiomer, diastereomer, or stereoisomer thereof, or pharmaceutically acceptable salt, hydrate, or solvate thereof. For any of the above uses, the required dosage will vary depending on the mode of administration, the particular condition to be treated and the effect desired.

## Pharmaceutical composition and combination therapy

In some embodiments, a compound of Formula I or a pharmaceutically acceptable salt thereof can be used in an effective amount, either alone or in combination, to treat a patient such as a human with a disorder as described herein or a RET mediated disorder.

The disclosed compounds described herein can be used in an effective amount alone or in combination with another compound of the present invention or another bioactive agent or second therapeutic agent to treat a patient such as a human with a disorder, including but not limited to those described herein.

The term "bioactive agent" is used to describe an agent, other than the selected compound according to the present invention, which can be used in combination or alternation with a compound of the present invention to achieve a desired result of therapy. In one embodiment, the compound of the present invention and the bioactive agent are administered in a manner that they are active in vivo during overlapping time periods, for example, have time-period overlapping Cmax, Tmax, AUC or other pharmacokinetic parameter. In another embodiment, the compound of the present invention and the bioactive agent are administered to a patient in need thereof that do not have overlapping pharmacokinetic parameter, however, one has a therapeutic impact on the therapeutic efficacy of the other.

In one aspect of this embodiment, the bioactive agent is an immune modulator, including but not limited to a checkpoint inhibitor, including as non-limiting examples, a PD-1 inhibitor, PD-L1 inhibitor, PD-L2 inhibitor, CTLA-4 inhibitor, LAG-3 inhibitor, TIM-3 inhibitor, V-domain Ig suppressor of T-cell activation (VISTA) inhibitors, small molecule,

peptide, nucleotide, or other inhibitor. In certain aspects, the immune modulator is an antibody, such as a monoclonal antibody. PD-1 inhibitors that blocks the interaction of PD-1 and PD-L1 by binding to the PD-1 receptor, and in turn inhibit immune suppression include, for example, nivolumab (Opdivo), pembrolizumab (Keytruda), pidilizumab, AMP-224 (AstraZeneca and MedImmune), PF- 06801591 (Pfizer), MED10680 (AstraZeneca), PDR001 (Novartis), REGN2810 (Regeneron), SHR-12-1 (Jiangsu Hengrui Medicine Company and Incyte Corporation), TSR-042 (Tesaro), and the PD-L1/VISTA inhibitor CA-170 (Curis Inc.). PD-L1 inhibitors that block the interaction of PD-1 and PD-L1 by binding to the PD-L1 receptor, and in turn inhibits immune suppression, include for example, atezolizumab (Tecentriq), durvalumab (AstraZeneca and MedImmune), KN035 (Alphamab), and BMS-936559 (Bristol-Myers Squibb). CTLA-4 checkpoint inhibitors that bind to CTLA-4 and inhibits immune suppression include, but are not limited to, ipilimumab, tremelimumab (AstraZeneca and MedImmune), AGEN1884 and AGEN2041 (Agenus). LAG-3 checkpoint inhibitors include, but are not limited to, BMS-986016 (Bristol-Myers Squibb), GSK2831781 (GlaxoSmithKline), IMP321 (Prima BioMed), LAG525 (Novartis), and the dual PD-1 and LAG-3 inhibitor MGD013 (MacroGenics). An example of a TIM-3 inhibitor is TSR- 022 (Tesaro). In certain embodiments the checkpoint inhibitor is selected from nivolumab/OPDIVO®; pembrolizumab/KEYTRUDA®; and pidilizumab/CT-011, MPDL3280A/RG7446; MEDI4736; MSB0010718C; BMS 936559, a PDL2/lg fusion protein such as AMP 224 or an inhibitor of B7- H3 (e.g., MGA271 ), B7-H4, BTLA, HVEM, TIM3, GAL9, LAG 3, VISTA, KIR, 2B4, CD160, CGEN-15049, CHK 1 , CHK2, A2aR, B-7 family ligands, or a combination thereof.

In yet another embodiment, one of the active compounds described herein can be administered in an effective amount for the treatment of abnormal tissue of the female reproductive system such as breast, ovarian, endometrial, or uterine cancer, in combination or alternation with an effective amount of an estrogen inhibitor including, but not limited to, a SERM (selective estrogen receptor modulator), a SERD (selective estrogen receptor degrader), a complete estrogen receptor degrader, or another form of partial or complete estrogen antagonist or agonist. Partial anti-estrogens like raloxifene and tamoxifen retain some estrogen-like effects, including an estrogen-like stimulation of uterine growth, and also, in some cases, an estrogen-like action during breast cancer progression which actually stimulates tumor growth. In contrast, fulvestrant, a complete anti-estrogen, is free of estrogen-like action on the uterus and is effective in tamoxifen-resistant tumors. Non-limiting examples of anti-estrogen compounds are provided in WO 2014/19176 assigned to Astra Zeneca, WO2013/090921, WO 2014/203129, WO 2014/203132, and US2013/0178445 assigned to Olema Pharmaceuticals, and U.S. Patent Nos.9,078,871, 8,853,423, and 8,703, 810, as well as US 2015/0005286, WO 2014/205136, and WO 2014/205138. Additional non-limiting examples of anti-estrogen compounds include: SERMS such as anordrin, bazedoxifene, broparestriol, chlorotrianisene, clomiphene citrate, cyclofenil, lasofoxifene, ormeloxifene, raloxifene, tamoxifen, toremifene, and fulvestratnt; aromatase inhibitors such as aminoglutethimide, testolactone, anastrozole, exemestane, fadrozole, formestane, and letrozole; and antigonadotropins such as leuprorelin, cetrorelix, allylestrenol, chloromadinone acetate, cyproterone acetate, delmadinone acetate, dydrogesterone, medroxyprogesterone acetate, megestrol acetate, nomegestrol acetate, norethisterone acetate, progesterone, and spironolactone. Other estrogenic ligands that can be used according to the present invention are described in U.S. Patent Nos.4,418,068; 5,478,847; 5,393,763; and 5,457,117, WO2011/156518, US Patent Nos. 8,455,534 and 8,299,112, U.S. Patent Nos. 9,078,871; 8,853,423; 8,703,810; US 2015/0005286; and WO 2014/205138, US2016/0175289, US2015/0258080, WO 2014/191726, WO 2012/084711; WO 2002/013802; WO 2002/004418; WO 2002/003992; WO 2002/003991; WO 2002/003990; WO 2002/003989; WO 2002/003988; WO 2002/003986; WO 2002/003977; WO 2002/003976; WO 2002/003975; WO 2006/078834; US 6821989; US 2002/0128276; US 6777424; US 2002/0016340; US 6326392; US 6756401; US 2002/0013327; US 6512002; US 6632834; US 2001/0056099; US 6583170; US 6479535; WO 1999/024027; US 6005102; EP 0802184; US 5998402; US 5780497, US 5880137, WO 2012/048058 and WO 2007/087684.

In another embodiment, an active compound described herein can be administered in an effective amount for the treatment of abnormal tissue of the male reproductive system such as prostate or testicular cancer, in combination or alternation with an effective amount of an androgen (such as testosterone) inhibitor including, but not limited to a selective androgen receptor modulator, a selective androgen receptor degrader, a complete androgen receptor degrader, or another form of partial or complete androgen antagonist. In one embodiment, the prostate or testicular cancer is androgen-resistant. Non-limiting examples of anti-androgen compounds are provided in WO 2011/156518 and US Patent Nos. 8,455,534 and 8,299,112. Additional non-limiting examples of anti-androgen compounds include: enzalutamide, apalutamide, cyproterone acetate, chlormadinone acetate, spironolactone, canrenone, drospirenone, ketoconazole, topilutamide, abiraterone acetate, and cimetidine.

In one embodiment, the bioactive agent is an ALK inhibitor. Examples of ALK inhibitors include but are not limited to Crizotinib, Alectinib, ceritinib, TAE684 (NVP-TAE684), GSK1838705A, AZD3463, ASP3026, PF-06463922, entrectinib (RXDX-101), and AP26113.

In one embodiment, the bioactive agent is an EGFR inhibitor. Examples of EGFR inhibitors include erlotinib (Tarceva), gefitinib (Iressa), afatinib (Gilotrif), rociletinib (CO-1686), osimertinib (Tagrisso), olmutinib (Olita), naquotinib (ASP8273), nazartinib (EGF816), PF-06747775 (Pfizer), icotinib (BPI-2009), neratinib (HKI-272; PB272); avitinib (AC0010), EA1045, tarloxotinib (TH-4000; PR-610), PF-06459988 (Pfizer), tesevatinib (XL647; EXEL-7647; KD-

019), transtinib, WZ-3146, WZ8040, CNX-2006, and dacomitinib (PF-00299804; Pfizer).

In one embodiment, the bioactive agent is an HER-2 inhibitor. Examples of HER-2 inhibitors include trastuzumab, lapatinib, ado-trastuzumab emtansine, and pertuzumab.

In one embodiment, the bioactive agent is a CD20 inhibitor. Examples of CD20 inhibitors include obinutuzumab, rituximab, fatumumab, ibritumomab, tositumomab, and ocrelizumab.

In one embodiment, the bioactive agent is a JAK3 inhibitor. Examples of JAK3 inhibitors include tasocitinib.

In one embodiment, the bioactive agent is a BCL-2 inhibitor. Examples of BCL-2 inhibitors include venetoclax, ABT-199 (4-[4-[[2-(4-Chlorophenyl)-4,4-dimethylcyclohex-1-en-1-yl]methyl]piperazin-1-yl]-N-[[3-nitro-4-[[(tetrahydro-2H-pyran-4-yl)methyl]amino]phenyl]sulfonyl]-2-[(1H- pyrrolo[2,3-b]pyridin-5-yl)oxy]benzamide), ABT-737 (4-[4-[[2-(4-chlorophenyl)phenyl]methyl]piperazin-1-yl]-N-[4- [[(2R)-4-(dimethylamino)-1-phenylsulfanylbutan-2-yl] amino]-3- nitrophenyl]sulfonylbenzamide) (navitoclax), ABT-263 ((R)-4-(4-((4'-chloro-4,4-dimethyl-3,4,5,6-tetrahydro-[l, l'-biphenyl]-2-yl)methyl)piperazin-1-yl)- N-((4-((4-morpholino-1-(phenylthio)butan-2-yl)amino)-3((trifluoromethyl)sulfonyl)phenyl)sulfonyl)benzamide), GX15-070 (obatoclax mesylate, (2Z)-2- [(5Z)-5-[(3,5- dimethyl-lH-pyrrol-2-yl)methylidene]-4-methoxypyrrol-2-ylidene]indole; methanesulfonic acid))), 2-methoxy-antimycin A3, YC137 (4-(4,9-dioxo-4,9-dihydronaphtho[2,3-d]thiazol-2-ylamino)-phenyl ester), pogosin, ethyl 2-amino-6-bromo-4-(1-cyano-2-ethoxy-2-oxoethyl)-4H-chromene-3-carboxylate, Nilotinib-d3, TW-37 (N-[4-[[2-(1,1-Dimethylethyl)phenyl]sulfonyl]phenyl]-2,3,4-trihydroxy-5-[[2-(1-methylethyl)phenyl]methyl]benzamide), Apogossypolone (ApoG2), HA14-1, AT101, sabutoclax, gambogic acid, or G3139 (Oblimersen).

In one embodiment, the bioactive agent is a kinase inhibitor. In one embodiment, the kinase inhibitor is selected from a phosphoinositide 3-kinase (PI3K) inhibitor, a Bruton's tyrosine kinase (BTK) inhibitor, or a spleen tyrosine kinase (Syk) inhibitor, or a combination thereof. Examples of PI3 kinase inhibitors include, but are not limited to, Wortmannin, demethoxyviridin, perifosine, idelalisib, Pictilisib , Palomid 529, ZSTK474, PWT33597, CUDC-907, and AEZS-136, duvelisib, GS-9820, BKM120, GDC-0032 (Taselisib) (2-[4-[2-(2-Isopropyl- 5-methyl-1,2,4-triazol-3-yl)-5,6-dihydroimidazo[1,2-d][1,4]benzoxazepin-9-yl]pyrazol-1-yl]-2- methylpropanamide), MLN-1117 ((2R)-1-Phenoxy-2-butanyl hydrogen (S)-methylphosphonate; or Methyl(oxo) {[(2R)-l-phenoxy-2-butanyl]oxy}phosphonium)), BYL-719 ((2S)-N1-[4-Methyl-5-[2-(2,2,2-trifluoro-1,1-dimethylethyl)-4-pyridinyl]-2-thiazolyl]-1,2-pyrrolidinedicarboxamide), GSK2126458 (2,4-Difluoro-N-{2-(methyloxy)-5-[4-(4-pyridazinyl)-6-quinolinyl]-3- pyridinyl}benzenesulfonamide) (omipalisib), TGX-221 (($\pm$)-7-Methyl-2-(morpholin-4-yl)-9-(l- phenylaminoethyl)-pyrido[l,2-a]-pyrimidin-4-one), GSK2636771 (2-Methyl-1-(2-methyl-3- (trifluoromethyl)benzyl)-6-morpholino-lH-benzo[d]imidazole-4-carboxylic acid dihydrochloride), KIN-193 ((R)-2-((l-(7-methyl-2-morpholino-4-oxo-4H-pyrido[1,2-a]pyrimidin-9- yl)ethyl)amino)benzoic acid), TGR-1202/RP5264, GS-9820 ((S)-l-(4-((2-(2-aminopyrimidin-5- yl)-7-methyl-4-mohydroxypropan- 1 -one), GS-1101 (5-fluoro-3-phenyl-2-([S)]-1-[9H-purin-6- ylamino]-propyl)-3H-quinazolin-4-one), AMG-319, GSK-2269557, SAR245409 (N-(4-(N-(3-((3,5-dimethoxyphenyl)amino)quinoxalin-2-yl)sulfamoyl)phenyl)-3-methoxy-4 methylbenzamide), BAY80-6946 (2-amino-N-(7-methoxy-8-(3-morpholinopropoxy)-2,3- dihydroimidazo[l,2-c]quinaz), AS 252424 (5-[l-[5-(4-Fluoro-2-hydroxy-phenyl)-furan-2-yl]- meth-(Z)-ylidene]-thiazolidine-2,4-dione), CZ 24832 (5-(2-amino-8-fluoro-[l,2,4]triazolo[l,5- a]pyridin-6-yl)-N-tert-butylpyridine-3-sulfonamide), Buparlisib (5-[2,6-Di(4-morpholinyl)-4- pyrimidinyl]-4-(trifluoromethyl)-2-pyridinamine), GDC-0941 (2-(lH-Indazol-4-yl)-6-[[4- (methylsulfonyl)-l-piperazinyl]methyl]-4-(4-morpholinyl)thieno[3,2-d]pyrimidine), GDC-0980 ((S)-1-(4-((2-(2-aminopyrimidin-5-yl)-7-methyl-4-morpholinothieno[3,2-d]pyrimidin-6 yl) methyl)piperazin-l-yl)-2-hydroxypropan-l-one (also known as RG7422)), SF1126 ((8S,14S,17S)-14-(carboxymethyl)-8-(3-guanidinopropyl)-17-(hydroxymethyl)-3,6,9,12,15- pentaoxo-1-(4-(4-oxo-8-phenyl-4H-chromen-2-yl)morpholino-4-ium)-2-oxa-7,10,13,16- tetraazaoctadecan-18-oate), PF-05212384 (N-[4-[[4-(Dimethylamino)-1-piperidinyl] carbonyl]phenyl]-N'-[4-(4,6-di-4-morpholinyl-l,3,5-triazin-2-yl)phenyl]urea) (gedatolisib), LY3023414, BEZ235 (2-Methyl-2-{4-[3-methyl-2-oxo-8-(quinolin-3-yl)-2,3-dihydro-lH-imidazo[4,5-c]quinolin-l-yl]phenyl}propanenitrile) (dactolisib), XL-765 (N-(3-(N-(3- (3,5-dimethoxyphenylamino)quinoxalin-2-yl)sulfamoyl)phenyl)-3-methoxy-4-methylbenzamide), and GSK1059615 (5-[[4-(4-Pyridinyl)-6-quinolinyl]methylene]-2,4-thiazolidenedione), PX886 ([(3aR,6E,9S,9aR,10R,11aS)-6-[[bis(prop-2-enyl)amino]methylidene]-5-hydroxy-9- (methoxymethyl)-9a,11a-dimethyl-1,4,7-trioxo-2,3,3a,9,10,11-hexahydroindeno[4,5h]isochromen- 10-yl] acetate (also known as sonolisib)), LY294002, AZD8186, PF-4989216, pilaralisib, GNE- 317, PI-3065, PI-103, NU7441 (KU-57788), HS 173, VS-5584 (SB2343), CZC24832, TG100- 115, A66, YM201636, CAY10505, PIK-75, PIK-93, AS-605240, BGT226 (NVP-BGT226), AZD6482, voxtalisib, alpelisib, IC-87114, TGI100713, CH5132799, PKI-402, copanlisib (BAY 80-6946), XL 147, PIK-90, PIK-293, PIK-294, 3-MA (3-methyladenine), AS-252424, AS-604850, apitolisib (GDC-0980; RG7422). Examples of BTK inhibitors include ibrutinib (also known as PCI-32765)(Imbruvica™)(1- [(3R)-3-[4-amino-3-(4-phenoxy-phenyl)pyrazolo[3,4-d]pyrimidin-1-yl]piperidin-1-yl]prop-2-en- 1-one), dianilinopyrimidine-based inhibitors such as AVL-101 and AVL-291/292 (N-(3-((5- fluoro-2-((4-(2-methoxyethoxy)phenyl)amino)pyrimidin-4-yl)amino)phenyl)acrylamide) (Avila Therapeutics) (see US Patent Publication No 2011/0117073, incorporated herein in its entirety), Dasatinib ([N-(2-chloro-6-methylphenyl)-2-(6-(4-(2-hydroxyethyl)piperazin-1-yl)-2- methylpyrimidin-4-ylamino)thiazole-5-carboxamide], LFM-A13 (alpha-cyano-beta-hydroxy- beta-methyl-N-(2,5-ibromophenyl) propenamide), GDC-0834 ([R-N-(3-(6-(4-(1,4-dimethyl-3- oxopiperazin-2-yl)phenylamino)-4-methyl-5-oxo-4,5-dihydropyrazin-2-yl)-2-methylphenyl)-

4,5,6,7-tetrahydrobenzo[b]thiophene-2-carboxamide], CGI-560 4-(tert-butyl)-N-(3-(8- (phenylamino)imidazo[1,2-a]pyrazin-6-yl)phenyl)benzamide, CGI-1746 (4-(tert-butyl)-N-(2- methyl-3-(4-methyl-6-((4-(morpholine-4-carbonyl)phenyl) amino)-5-oxo-4,5-dihydropyrazin-2- yl)phenyl)benzamide), CNX-774 (4-(4-((4-((3-acrylamidophenyl)amino)-5-fluoropyrimidin-2- yl)amino)phenoxy)-N-methylpicolinamide), CTA056 (7-benzyl-1-(3-(piperidin-1-yl)propyl)-2- (4-(pyridin-4-yl)phenyl)-1H-imidazo[4,5-g]quinoxalin-6(5H)-one), GDC-0834 ((R)-N-(3-(6-((4- (1,4-dimethyl-3-oxopiperazin-2-yl)phenyl)amino)-4-methyl-5-oxo-4,5-dihydropyrazin-2-yl)-2-methylphenyl)-4,5,6,7-tetrahydrobenzo[b]thiophene-2-carboxamide), GDC-0837 ((R)-N-(3-(6-((4-(1,4-dimethyl-3-oxopiperazin-2-yl)phenyl)amino)-4-methyl-5-oxo-4,5-dihydropyrazin-2-yl)-2-methylphenyl)-4,5,6,7-tetrahydrobenzo[b]thiophene-2-carboxamide), HM-71224, ACP-196, ONO-4059 (Ono Pharmaceuticals), PRT062607 (4-((3-(2H-1,2,3-triazol-2-yl)phenyl)amino)-2-(((1R,2S)-2-aminocyclohexyl)amino)pyrimidine-5-carboxamide hydrochloride), QL-47 (1-(1-acryloylindolin-6-yl)-9-(1-methyl-1H-pyrazol-4-yl)benzo[h][1,6]naphthyridin-2(1H)-one), and RN486 (6-cyclopropyl-8-fluoro-2-(2-hydroxymethyl-3-{1-methyl-5-[5-(4-methyl-piperazin-1-yl)-pyridin-2-ylamino]-6-oxo-1,6-dihydro-pyridin-3-yl}-phenyl)-2H-isoquinolin-1-one), and other molecules capable of inhibiting BTK activity, for example those BTK inhibitors disclosed in Akinleye et ah, Journal of Hematology & Oncology, 2013, 6:59. Syk inhibitors include, but are not limited to, Cerdulatinib (4-(cyclopropylamino)-2-((4- (4-(ethylsulfonyl)piperazin-1-yl)phenyl)amino)pyrimidine-5-carboxamide), entospletinib (6-(1H- indazol-6-yl)-N-(4-morpholinophenyl)imidazo[1,2-a]pyrazin-8-amine), fostamatinib ([6-({5- Fluoro-2-[(3,4,5-trimethoxyphenyl)amino]-4-pyrimidinyl} amino)-2,2-dimethyl-3-oxo-2,3- dihydro-4H-pyrido[3,2-b][1,4]oxazin-4-yl]methyl dihydrogen phosphate), fostamatinib disodium salt (sodium (6-((5-fluoro-2-((3,4,5-trimethoxyphenyl)amino)pyrimidin-4-yl)amino)-2,2- dimethyl-3-oxo-2H-pyrido[3,2-b][1,4]oxazin-4(3H)-yl)methyl phosphate), BAY 61-3606 (2-(7- (3,4-Dimethoxyphenyl)-imidazo[1,2-c]pyrimidin-5-ylamino)-nicotinamide HCl), RO9021 (6-[(1R,2S)-2-Amino-cyclohexylamino]-4-(5,6-dimethyl-pyridin-2-ylamino)-pyridazine-3-carboxylic acid amide), imatinib (Gleevac; 4-[(4-methylpiperazin-1-yl)methyl]-N-(4-methyl-3-{[4-(pyridin-3-yl)pyrimidin-2-yl]amino}phenyl)benzamide), staurosporine, GSK143 (2-(((3R,4R)-3-aminotetrahydro-2H-pyran-4-yl)amino)-4-(p-tolylamino)pyrimidine-5-carboxamide), PP2 (1-(tert-butyl)-3-(4-chlorophenyl)-1H-pyrazolo[3,4-d]pyrimidin-4-amine), PRT-060318 (2-(((1R,2S)-2-aminocyclohexyl)amino)-4-(m-tolylamino)pyrimidine-5-carboxamide), PRT-062607 (4-((3-(2H-1,2,3-triazol-2-yl)phenyl)amino)-2-(((1R,2S)-2-aminocyclohexyl)amino)pyrimidine-5-carboxamide hydrochloride), R112 (3,3'-((5-fluoropyrimidine-2,4-diyl)bis(azanediyl))diphenol), R348 (3-Ethyl-4-methylpyridine), R406 (6-((5-fluoro-2-((3,4,5-trimethoxyphenyl)amino)pyrimidin-4-yl)amino)-2,2-dimethyl-2H-pyrido[3,2-b][1,4]oxazin-3(4H)-one), piceatannol (3-Hydroxyresveratol), YM193306 (see Singh et al. Discovery and Development of Spleen Tyrosine Kinase (SYK) Inhibitors, J. Med. Chem. 2012, 55, 3614-3643), 7-azaindole, piceatannol, ER-27319 (see Singh et al. Discovery and Development of Spleen Tyrosine Kinase (SYK) Inhibitors, J. Med. Chem. 2012, 55, 3614-3643 incorporated in its entirety herein), Compound D (see Singh et al. Discovery and Development of Spleen Tyrosine Kinase (SYK) Inhibitors, J. Med. Chem.2012, 55, 3614-3643 incorporated in its entirety herein), PRT060318 (see Singh et al. Discovery and Development of Spleen Tyrosine Kinase (SYK) Inhibitors, J. Med. Chem.2012, 55, 3614-3643 incorporated in its entirety herein), luteolin (see Singh et al. Discovery and Development of Spleen Tyrosine Kinase (SYK) Inhibitors, J. Med. Chem.2012, 55, 3614-3643 incorporated in its entirety herein), apigenin (see Singh et al. Discovery and Development of Spleen Tyrosine Kinase (SYK) Inhibitors, J. Med. Chem. 2012, 55, 3614-3643 incorporated in its entirety herein), quercetin (see Singh et al. Discovery and Development of Spleen Tyrosine Kinase (SYK) Inhibitors, J. Med. Chem. 2012, 55, 3614-3643 incorporated in its entirety herein), fisetin (see Singh et al. Discovery and Development of Spleen Tyrosine Kinase (SYK) Inhibitors, J. Med. Chem.2012, 55, 3614-3643 incorporated in its entirety herein), myricetin (see Singh et al. Discovery and Development of Spleen Tyrosine Kinase (SYK) Inhibitors, J. Med. Chem. 2012, 55, 3614-3643 incorporated in its entirety herein), morin (see Singh et al. Discovery and Development of Spleen Tyrosine Kinase (SYK) Inhibitors, J. Med. Chem.2012, 55, 3614-3643 incorporated in its entirety herein). In one embodiment, the bioactive agent is a MEK inhibitor. MEK inhibitors are well known, and include, for example, trametinib/GSKl120212 (N-(3-{3-Cyclopropyl-5-[(2-fluoro-4-iodophenyl)amino]-6,8-dimethyl-2,4,7-trioxo-3,4,6,7-tetrahydropyrido[4,3-d]pyrimidin-l(2H-yl}phenyl)acetamide), selumetinib (6-(4-bromo-2-chloroanilino)-7-fluoro-N-(2-hydroxyethoxy)-3-methylbenzimidazole-5-carboxamide), pimasertib/AS703026/MSC 1935369 ((S)-N-(2,3-dihydroxypropyl)-3-((2-fluoro-4- iodophenyl)amino)isonicotinamide), XL-518/GDC-0973 (l-({3,4-difluoro-2-[(2-fluoro-4- iodophenyl) amino]phenyl}carbonyl)-3-[(2S)-piperidin-2-yl]azetidin-3-ol), refametinib/BAY869766/RDEAl 19 (N-(3,4-difluoro-2-(2-fluoro-4-iodophenylamino)-6-methoxyphenyl)-1-(2,3-dihydroxypropyl)cyclopropane-1-sulfonamide), PD-0325901 (N-[(2R)-2,3-Dihydroxypropoxy]-3,4-difluoro-2-[(2-fluoro-4-iodophenyl)amino]-benzamide), TAK733 ((R)-3-(2,3-Dihydroxypropyl)-6-fluoro-5-(2-fluoro-4-iodophenylamino)-8- methylpyrido[2,3-d]pyrimidine-4,7(3H,8H)-dione), MEK162/ARRY438162 (5-[(4-Bromo-2-fluorophenyl)amino]-4-fluoro-N-(2- hydroxyethoxy)-1-methyl-1H-benzimidazole-6-carboxamide), R05126766 (3-[[3-Fluoro-2- (methylsulfamoylamino)-4-pyridyl]methyl]-4-methyl-7-pyrimidin-2-yloxychromen-2-one), WX-554, R04987655/CH4987655 (3,4-difluoro-2-((2-fluoro-4-iodophenyl)amino)-N-(2-hydroxyethoxy)-5-((3-oxo-l,2-oxazinan-2yl)methyl)benzamide), or AZD8330 (2-((2-fluoro-4-iodophenyl)amino)-N-(2 hydroxyethoxy)-1 ,5-dimethyl-6-oxo-l,6-dihydropyridine-3-carboxamide), U0126-EtOH, PD184352 (CI-1040), GDC-0623, BI-847325, cobimetinib, PD98059, BIX 02189, BIX 02188, binimetinib, SL-327, TAK-733, PD318088. In one embodiment, the bioactive agent is a Raf inhibitor. Raf inhibitors are known and include, for example, Vemurafinib (N-[3-[[5-(4-Chlorophenyl)-1H-pyrrolo[2,3-b]pyridin-3- yl]carbonyl]-2,4-difluorophenyl]-1-propanesulfonamide), sorafenib tosylate (4-[4-[[4-chloro-3- (trifluoromethyl)phenyl]carbamoylamino]phenoxy]-N-methylpyridine-2-carboxamide;4- methylbenzenesulfonate), AZ628 (3-(2-cyanopropan-2-yl)-N-(4-methyl-3-(3-methyl-4-oxo-3,4- dihydroquinazolin-6-ylamino)phenyl) benzamide), NVP-BHG712 (4-methyl-3-(1-methyl-6- (pyridin-3-yl)-1H-pyrazolo[3,4-d]pyrimidin-4-ylamino)-N-(3-(trifluoromethyl)phenyl)benzamide), RAF-265 (1-methyl-5-[2-[5-(trifluoromethyl)-1H-imidazol-2-yl]pyridin-4-yl]oxy-N-[4-(trifluoromethyl)phenyl]benzimidazol-2-amine), 2-Bromoaldisine (2-Bromo-6,7-dihydro-1H,5H-pyrrolo[2,3-c]azepine-4,8-dione), Raf Kinase Inhibitor IV (2- chloro-5-(2-phenyl-5-(pyridin-4-yl)-1H-imidazol-4-yl)phenol), Sorafenib N-Oxide (4-[4-[[[[4- Chloro-3(trifluoroMethyl)phenyl]aMino]carbonyl]aMino]phenoxy]-N-Methyl- 2pyridinecarboxaMide 1-Oxide), PLX-4720, dabrafenib (GSK2118436), GDC-0879, RAF265, AZ 628, SB590885, ZM336372, GW5074, TAK-632, CEP-32496, LY3009120, and GX818 (Encorafenib). In one embodiment, the bioactive agent is an AKT inhibitor, including, but not limited to, MK-2206, GSK690693, Perifosine, (KRX-0401), GDC-0068, Triciribine, AZD5363, Honokiol, PF-04691502, and Miltefosine, a FLT-3 inhibitor, including, but not limited to, P406, Dovitinib, Quizartinib (AC220), Amuvatinib (MP-470), Tandutinib (MLN518), ENMD-2076, and KW-2449, or a combination thereof. In one embodiment, the bioactive agent is an mTOR inhibitor. Examples of mTOR inhibitors include, but are not limited to, rapamycin and its analogs, everolimus (Afinitor), temsirolimus, ridaforolimus, sirolimus, and deforolimus. Examples of MEK inhibitors include but are not limited to tametinib/GSKl120212 (N-(3-{3-Cyclopropyl-5-[(2-fluoro-4- iodophenyl)amino]-6,8-dimethyl-2,4,7-trioxo-3,4,6,7-tetrahydropyrido[4,3-d]pyrimidin-l(2H- yl}phenyl)acetamide), selumetinob (6-(4-bromo-2-chloroanilino)-7-fluoro-N-(2-hydroxyethoxy)- 3-methylbenzimidazole-5-carboxamide), pimasertib/AS703026/MSC1935369 ((S)-N-(2,3- dihydroxypropyl)-3-((2-fluoro-4-iodophenyl)amino)isonicotinamide), XL-518/GDC-0973 (l- ({3,4-difluoro-2-[(2-fluoro-4-iodophenyl)amino]phenyl}carbonyl)-3-[(2S)-piperidin-2- yl]azetidin-3-ol) (cobimetinib), refametinib/BAY869766/RDEAl19 (N-(3,4-difluoro-2-(2-fluoro- 4-iodophenylamino)-6-methoxyphenyl)-1-(2,3-dihydroxypropyl)cyclopropane-1-sulfonamide), PD-0325901 (N-[(2R)-2,3-Dihydroxypropoxy]-3,4-difluoro-2-[(2-fluoro-4-iodophenyl)amino]- benzamide), TAK733 ((R)-3-(2,3-Dihydroxypropyl)-6-fluoro-5-(2-fluoro-4-iodophenylamino)-8-methylpyrido[2,3d]pyrimidine-4,7(3H,8H)-dione), MEK162/ARRY438162 (5-[(4-Bromo-2-fluorophenyl) amino]-4-fluoro-N-(2-hydroxyethoxy)-1-methyl-1H-benzimidazole-6 carboxamide), R05126766 (3-[[3-Fluoro-2-(methylsulfamoylamino)-4-pyridyl]methyl]-4-methyl-7-pyrimidin-2-yloxychromen-2-one), WX-554, R04987655/CH4987655 (3,4-difluoro-2-((2-fluoro-4-iodophenyl)amino)-N-(2-hydroxyethoxy)-5-((3-oxo-1,2-oxazinan-2 yl)methyl)benzamide), or AZD8330 (2-((2-fluoro-4-iodophenyl)amino)-N-(2-hydroxyethoxy)-1,5-dimethyl-6-oxo-l,6-dihydropyridine-3-carboxamide). In one embodiment, the bioactive agent is a RAS inhibitor. Examples of RAS inhibitors include but are not limited to Reolysin and siG12D LODER.

In one embodiment, the bioactive agent is a HSP inhibitor. HSP inhibitors include but are not limited to Geldanamycin or 17-N-Allylamino-17-demethoxygeldanamycin (17AAG), and Radicicol.

Additional bioactive compounds include, for example, everolimus, trabectedin, abraxane, TLK 286, AV-299, DN-101, pazopanib, GSK690693, RTA 744, ON 0910.Na, AZD 6244 (ARRY- 142886), AMN-107, TKI-258, GSK461364, AZD 1152, enzastaurin, vandetanib, ARQ-197, MK- 0457, MLN8054, PHA-739358, R-763, AT-9263, a FLT-3 inhibitor, a VEGFR inhibitor, an aurora kinase inhibitor, a PIK-1 modulator, an HDAC inhbitor, a c-MET inhibitor, a PARP inhibitor, a Cdk inhibitor, an IGFR-TK inhibitor, an anti-HGF antibody, a focal adhesion kinase inhibitor, a Map kinase kinase (mek) inhibitor, a VEGF trap antibody, pemetrexed, panitumumab, amrubicin, oregovomab, Lep-etu, nolatrexed, azd2171, batabulin, of atumumab, zanolimumab, edotecarin, tetrandrine, rubitecan, tesmilifene, oblimersen, ticilimumab, ipilimumab, gossypol, Bio 111, 131- I-TM-601, ALT-110, BIO 140, CC 8490, cilengitide, gimatecan, IL13-PE38QQR, INO 1001, IPdR1 KRX-0402, lucanthone, LY317615, neuradiab, vitespan, Rta 744, Sdx 102, talampanel, atrasentan, Xr 311, romidepsin, ADS-100380, sunitinib, 5-fluorouracil, vorinostat, etoposide, gemcitabine, doxorubicin, liposomal doxorubicin, 5'-deoxy-5-fluorouridine, vincristine, temozolomide, ZK-304709, seliciclib; PD0325901, AZD-6244, capecitabine, L-Glutamic acid, N- [4-[2-(2-amino-4,7-dihydro-4-oxo-1H-pyrrolo[2,3-d]pyrimidin-5-yl)ethyl]benzoyl]-, disodium salt, heptahydrate, camptothecin, PEG-labeled irinotecan, tamoxifen, toremifene citrate, anastrazole, exemestane, letrozole, DES(diethylstilbestrol), estradiol, estrogen, conjugated estrogen, bevacizumab, IMC-1C11, CHIR-258); 3-[5-(methylsulfonylpiperadinemethyl)-indolyl- quinolone, vatalanib, AG-013736, AVE-0005, goserelin acetate, leuprolide acetate, triptorelin pamoate, medroxyprogesterone acetate, hydroxyprogesterone caproate, megestrol acetate, raloxifene, bicalutamide, flutamide, nilutamide, megestrol acetate, CP-724714; TAK-165, HKI- 272, erlotinib, lapatanib, canertinib, ABX-EGF antibody, erbitux, EKB-569, PKI-166, GW- 572016, Ionafarnib, BMS-214662, tipifarnib; amifostine, NVP-LAQ824, suberoyl analide hydroxamic acid, valproic acid, trichostatin A, FK-228, SU11248, sorafenib, KRN951, aminoglutethimide, arnsacrine, anagrelide, L-asparaginase, Bacillus Calmette-Guerin (BCG) vaccine, adriamycin, bleomycin, buserelin, busulfan, carboplatin, carmustine, chlorambucil, cisplatin, cladribine, clodronate, cyproterone, cytarabine, dacarbazine, dactinomycin, daunorubicin, diethylstilbestrol, epirubicin, fludarabine, fludrocortisone, fluoxymesterone, flutamide, gleevec, gemcitabine, hydroxyurea, idarubicin, ifosfamide, imatinib, leuprolide, levamisole, lomustine, mechlorethamine, melphalan, 6-mercaptopurine, mesna, methotrexate, mitomycin, mitotane, mitoxantrone, nilutamide, octreotide, oxaliplatin, pamidronate, pentostatin, plicamycin, porfimer, procarbazine, raltitrexed, rituximab, streptozocin, teniposide, testosterone, thalidomide, thioguanine, thiotepa, tretinoin, vindesine, 13-cis-retinoic acid, phenylalanine mustard,

uracil mustard, estramustine, altretamine, floxuridine, 5-deooxyuridine, cytosine arabinoside, 6-mecaptopurine, deoxycoformycin, calcitriol, valrubicin, mithramycin, vinblastine, vinorelbine, topotecan, razoxin, marimastat, COL-3, neovastat, BMS-275291, squalamine, endostatin, SU5416, SU6668, EMD121974, interleukin-12, IM862, angiostatin, vitaxin, droloxifene, idoxyfene, spironolactone, finasteride, cimitidine, trastuzumab, denileukin diftitox, gefitinib, bortezimib, paclitaxel, cremophor-free paclitaxel, docetaxel, epithilone B, BMS-247550, BMS-310705, droloxifene, 4-hydroxytamoxifen, pipendoxifene, ERA-923, arzoxifene, fulvestrant, acolbifene, lasofoxifene, idoxifene, TSE-424, HMR-3339, ZK186619, topotecan, PTK787/ZK 222584, VX-745, PD 184352, rapamycin, 40-O-(2-hydroxyethyl)-rapamycin, temsirolimus, AP- 23573, RAD001, ABT-578, BC-210, LY294002, LY292223, LY292696, LY293684, LY293646, wortmannin, ZM336372, L-779,450, PEG-filgrastim, darbepoetin, erythropoietin, granulocyte colony-stimulating factor, zolendronate, prednisone, cetuximab, granulocyte macrophage colony-stimulating factor, histrelin, pegylated interferon alfa-2a, interferon alfa-2a, pegylated interferon alfa-2b, interferon alfa-2b, azacitidine, PEG-L-asparaginase, lenalidomide, gemtuzumab, hydrocortisone, interleukin-11, dexrazoxane, alemtuzumab, all-transretinoic acid, ketoconazole, interleukin-2, megestrol, immune globulin, nitrogen mustard, methylprednisolone, ibritgumomab tiuxetan, androgens, decitabine, hexamethylmelamine, bexarotene, tositumomab, arsenic trioxide, cortisone, editronate, mitotane, cyclosporine, liposomal daunorubicin, Edwina-asparaginase, strontium 89, casopitant, netupitant, an NK-1 receptor antagonist, palonosetron, aprepitant, diphenhydramine, hydroxyzine, metoclopramide, lorazepam, alprazolam, haloperidol, droperidol, dronabinol, dexamethasone, methylprednisolone, prochlorperazine, granisetron, ondansetron, dolasetron, tropisetron, pegfilgrastim, erythropoietin, epoetin alfa, darbepoetin alfa and mixtures thereof.

In one embodiment, the bioactive agent is selected from, but are not limited to, Imatinib mesylate (Gleevac®), Dasatinib (Sprycel®), Nilotinib (Tasigna®), Bosutinib (Bosulif®), Trastuzumab (Herceptin®), trastuzumab-DM1, Pertuzumab (PerjetaTM), Lapatinib (Tykerb®), Gefitinib (Iressa®), Erlotinib (Tarceva®), Cetuximab (Erbitux®), Panitumumab (Vectibix®), Vandetanib (Caprelsa®), Vemurafenib (Zelboraf®), Vorinostat (Zolinza®), Romidepsin (Istodax®), Bexarotene (Tagretin®), Alitretinoin (Panretin®), Tretinoin (Vesanoid®), Carfilizomib (KyprolisTM), Pralatrexate (Folotyn®), Bevacizumab (Avastin®), Ziv-aflibercept (Zaltrap®), Sorafenib (Nexavar®), Sunitinib (Sutent®), Pazopanib (Votrient®), Regorafenib (Stivarga®), and Cabozantinib (CometriqTM).

In certain aspects, the bioactive agent is an anti-inflammatory agent, a chemotherapeutic agent, a radiotherapeutic, an additional therapeutic agent, or an immunosuppressive agent. Suitable chemotherapeutic bioactive agents include, but are not limited to, a radioactive molecule, a toxin, also referred to as cytotoxin or cytotoxic agent, which includes any agent that is detrimental to the viability of cells, and liposomes or other vesicles containing chemotherapeutic compounds. General anticancer pharmaceutical agents include: Vincristine (Oncovin®) or liposomal vincristine (Marqibo®), Daunorubicin (daunomycin or Cerubidine®) or doxorubicin (Adriamycin®), Cytarabine (cytosine arabinoside, ara-C, or Cytosar®), L-asparaginase (Elspar®) or PEG-L-asparaginase (pegaspargase or Oncaspar®), Etoposide (VP-16), Teniposide (Vumon®), 6-mercaptopurine (6-MP or Purinethol®), Methotrexate, Cyclophosphamide (Cytoxan®), Prednisone, Dexamethasone (Decadron), imatinib (Gleevec®), dasatinib (Sprycel®), nilotinib (Tasigna®), bosutinib (Bosulif®), and ponatinib (Iclusig™). Examples of additional suitable chemotherapeutic agents include, but are not limited to 1-dehydrotestosterone, 5-fluorouracil decarbazine, 6-mercaptopurine, 6-thioguanine, actinomycin D, adriamycin, aldesleukin, an alkylating agent, allopurinol sodium, altretamine, amifostine, anastrozole, anthramycin (AMC)), an anti-mitotic agent, cis-dichlorodiamine platinum (II) (DDP) cisplatin), diamino dichloro platinum, anthracycline, an antibiotic, an antimetabolite, asparaginase, BCG live (intravesical), betamethasone sodium phosphate and betamethasone acetate, bicalutamide, bleomycin sulfate, busulfan, calcium leucouorin, calicheamicin, capecitabine, carboplatin, lomustine (CCNU), carmustine (BSNU), Chlorambucil, Cisplatin, Cladribine, Colchicin, conjugated estrogens, Cyclophosphamide, Cyclothosphamide, Cytarabine, Cytarabine, cytochalasin B, Cytoxan, Dacarbazine, Dactinomycin, dactinomycin (formerly actinomycin), daunirubicin HCL, daunorucbicin citrate, denileukin diftitox, Dexrazoxane, Dibromomannitol, dihydroxy anthracin dione, Docetaxel, dolasetron mesylate, doxorubicin HCL, dronabinol, E. coli L-asparaginase, emetine, epoetin-$\alpha$, Erwinia L-asparaginase, esterified estrogens, estradiol, estramustine phosphate sodium, ethidium bromide, ethinyl estradiol, etidronate, etoposide citrororum factor, etoposide phosphate, filgrastim, floxuridine, fluconazole, fludarabine phosphate, fluorouracil, flutamide, folinic acid, gemcitabine HCL, glucocorticoids, goserelin acetate, gramicidin D, granisetron HCL, hydroxyurea, idarubicin HCL, ifosfamide, interferon $\alpha$-2b, irinotecan HCL, letrozole, leucovorin calcium, leuprolide acetate, levamisole HCL, lidocaine, lomustine, maytansinoid, mechlorethamine HCL, medroxyprogesterone acetate, megestrol acetate, melphalan HCL, mercaptipurine, mesna, methotrexate, methyltestosterone, mithramycin, mitomycin C, mitotane, mitoxantrone, nilutamide, octreotide acetate, ondansetron HCL, paclitaxel, pamidronate disodium, pentostatin, pilocarpine HCL, plimycin, polifeprosan 20 with carmustine implant, porfimer sodium, procaine, procarbazine HCL, propranolol, rituximab, sargramostim, streptozotocin, tamoxifen, taxol, teniposide, tenoposide, testolactone, tetracaine, thioepa chlorambucil, thioguanine, thiotepa, topotecan HCL, toremifene citrate, trastuzumab, tretinoin, valrubicin, vinblastine sulfate, vincristine sulfate, and vinorelbine tartrate.

In some embodiments, the compound of the present invention is administered in combination with a chemotherapeutic agent (e.g., a cytotoxic agent or other chemical compound useful in the treatment of cancer). Examples of chemotherapeutic agents include alkylating agents, antimetabolites, folic acid analogs, pyrimidine analogs, purine

analogs and related inhibitors, vinca alkaloids, epipodopyyllotoxins, antibiotics, L-Asparaginase, topoisomerase inhibitors, interferons, platinum coordination complexes, anthracenedione substituted urea, methyl hydrazine derivatives, adrenocortical suppressant, adrenocorticosteroides, progestins, estrogens, antiestrogen, androgens, antiandrogen, and gonadotropin-releasing hormone analog. Also included is 5 -fluorouracil (5-FU), leucovorin (LV), irenotecan, oxaliplatin, capecitabine, paclitaxel, and doxetaxel. Non-limiting examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; cally statin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1 ); eleutherobin; pancrati statin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gammall and calicheamicin omegall (see, e.g., Agnew, Chem. Inti. Ed Engl. 33: 183- 186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo- 5-oxo-L-norleucine, ADRIAMYCIN® (doxorubicin, including morpholino-doxorubicin, cyanomorpholino- doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5 -fluorouracil (5-FU); folic acid analogues such as denopterin, methotrexate, pteropterin, trimetrexate; purine analogs such as fludarabine, 6- mercaptopurine, thiamiprine, thioguanine; pyrimidine analogs such as ancitabine, azacitidine, 6- azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex (JHS Natural Products, Eugene, OR); razoxane; rhizoxin; sizofuran; spirogermanium; tenuazonic acid; triaziquone; 2,2',2"-trichlorotriethylamine; trichothecenes (especially T- 2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL® (paclitaxel; Bristol-Myers Squibb Oncology, Princeton, NJ), ABRAXANE®, cremophor-free, albumin-engineered nanoparticle formulation of paclitaxel (American Pharmaceutical Partners, Schaumberg, IL), and TAXOTERE® doxetaxel (Rhone-Poulenc Rorer, Antony, France); chloranbucil; GEMZAR® gemcitabine; 6-thioguanine; mercaptopurine; methotrexate; platinum coordination complexes such as cisplatin, oxaliplatin and carboplatin; vinblastine; platinum; etoposide (VP- 16); ifosfamide; mitoxantrone; vincristine; NAVELBINE® vinorelbine; novantrone; teniposide; edatrexate; daunomycin; aminopterin; xeloda; ibandronate; irinotecan (e.g., CPT-1 1 ); topoisomerase inhibitor RFS 2000; difluoromethylornithine (DMFO); retinoids such as retinoic acid; capecitabine; and pharmaceutically acceptable salts, acids or derivatives of any of the above. Two or more chemotherapeutic agents can be used in a cocktail to be administered in combination with the compound of the present invention. Suitable dosing regimens of combination chemotherapies are known in the ar. For example combination dosing regimes are described in Saltz et al., Proc. Am. Soc. Clin. Oncol. 18:233a (1999) and Douillard et al., Lancet 355(9209): 1041 -1047 (2000). Additional therapeutic agents that can be administered in combination with a Compound disclosed herein can include bevacizumab, sutinib, sorafenib, 2-methoxyestradiol or 2ME2, finasunate, vatalanib, vandetanib, aflibercept, volociximab, etaracizumab (MEDI-522), cilengitide, erlotinib, cetuximab, panitumumab, gefitinib, trastuzumab, dovitinib, figitumumab, atacicept, rituximab, alemtuzumab, aldesleukine, atlizumab, tocilizumab, temsirolimus, everolimus, lucatumumab, dacetuzumab, HLL1, huN901-DM1, atiprimod, natalizumab, bortezomib, carfilzomib, marizomib, tanespimycin, saquinavir mesylate, ritonavir, nelfinavir mesylate, indinavir sulfate, belinostat, panobinostat, mapatumumab, lexatumumab, dulanermin, ABT-737, oblimersen, plitidepsin, talmapimod, P276-00, enzastaurin, tipifarnib, perifosine, imatinib, dasatinib, lenalidomide, thalidomide, simvastatin, celecoxib, bazedoxifene, AZD4547, rilotumumab, oxaliplatin (Eloxatin), PD0332991, ribociclib (LEE011), amebaciclib (LY2835219), HDM201, fulvestrant (Faslodex), exemestane (Aromasin), PIM447, ruxolitinib (INC424), BGJ398, necitumumab, pemetrexed (Alimta), and ramucirumab (IMC-1121B). In one embodiment, the additional therapy is a monoclonal antibody (MAb). Some MAbs stimulate an immune response that destroys cancer cells. Similar to the antibodies produced naturally by B cells, these MAbs may "coat" the cancer cell surface, triggering its

destruction by the immune system. For example, bevacizumab targets vascular endothelial growth factor (VEGF), a protein secreted by tumor cells and other cells in the tumor's microenvironment that promotes the development of tumor blood vessels. When bound to bevacizumab, VEGF cannot interact with its cellular receptor, preventing the signaling that leads to the growth of new blood vessels. Similarly, cetuximab and panitumumab target the epidermal growth factor receptor (EGFR), and trastuzumab targets the human epidermal growth factor receptor 2 (HER-2). MAbs that bind to cell surface growth factor receptors prevent the targeted receptors from sending their normal growth-promoting signals. They may also trigger apoptosis and activate the immune system to destroy tumor cells. In one aspect of the present invention, the bioactive agent is an immunosuppressive agent. The immunosuppressive agent can be a calcineurin inhibitor, e.g. a cyclosporin or an ascomycin, e.g. Cyclosporin A (NEORAL®), FK506 (tacrolimus), pimecrolimus, a mTOR inhibitor, e.g. rapamycin or a derivative thereof, e.g. Sirolimus (RAPAMUNE®), Everolimus (Certican®), temsirolimus, zotarolimus, biolimus-7, biolimus-9, a rapalog, e.g.ridaforolimus, azathioprine, campath 1H, a S1P receptor modulator, e.g. fingolimod or an analogue thereof, an anti IL-8 antibody, mycophenolic acid or a salt thereof, e.g. sodium salt, or a prodrug thereof, e.g. Mycophenolate Mofetil (CELLCEPT®), OKT3 (ORTHOCLONE OKT3®), Prednisone, ATGAM®, THYMOGLOBULIN®, Brequinar Sodium, OKT4, T10B9.A-3A, 33B3.1, 15- deoxyspergualin, tresperimus, Leflunomide ARAVA®, CTLAI-Ig, anti-CD25, anti-IL2R, Basiliximab (SIMULECT®), Daclizumab (ZENAPAX®), mizorbine, methotrexate, dexamethasone, ISAtx-247, SDZ ASM 981 (pimecrolimus, Elidel®), CTLA4lg (Abatacept), belatacept, LFA3lg,, etanercept (sold as Enbrel® by Immunex), adalimumab (Humira®), infliximab (Remicade®), an anti-LFA-1 antibody, natalizumab (Antegren®), Enlimomab, gavilimomab, antithymocyte immunoglobulin, siplizumab, Alefacept efalizumab, pentasa, mesalazine, asacol, codeine phosphate, benorylate, fenbufen, naprosyn, diclofenac, etodolac and indomethacin, aspirin and ibuprofen. In some embodiments, the bioactive agent is a therapeutic agent which is a biologic such a cytokine (e.g., interferon or an interleukin (e.g., IL-2)) used in cancer treatment. In some embodiments the biologic is an anti-angiogenic agent, such as an anti-VEGF agent, e.g., bevacizumab (AVASTIN®). In some embodiments the biologic is an immunoglobulin-based biologic, e.g., a monoclonal antibody (e.g., a humanized antibody, a fully human antibody, an Fc fusion protein or a functional fragment thereof) that agonizes a target to stimulate an anti-cancer response, or antagonizes an antigen important for cancer. Such agents include RITUXAN® (rituximab); ZENAPAX® (daclizumab); SIMULECT® (basiliximab); SYNAGIS® (palivizumab); REMICADE® (infliximab); HERCEPTIN® (trastuzumab); MYLOTARG® (gemtuzumab ozogamicin); CAMPATH® (alemtuzumab); ZEVALIN® (ibritumomab tiuxetan); HUMIRA® (adalimumab); XOLAIR® (omalizumab); BEXXAR® (tositumomab-l- 131 ); RAPTIVA® (efalizumab); ERBITUX® (cetuximab); AVASTIN® (bevacizumab); TYSABRI® (natalizumab); ACTEMRA® (tocilizumab); VECTIBIX® (panitumumab); LUCENTIS® (ranibizumab); SOURIS® (eculizumab); CIMZIA® (certolizumab pegol); SIMPONI® (golimumab); ILARIS® (canakinumab); STELARA® (ustekinumab); ARZERRA® (ofatumumab); PROLIA® (denosumab); NUMAX® (motavizumab); ABTHRAX® (raxibacumab); BENLYSTA® (belimumab); YERVOY® (ipilimumab); ADCETRIS® (brentuximab vedotin); PERJETA® (pertuzumab); KADCYLA® (ado- trastuzumab emtansine); and GAZYVA® (obinutuzumab). Also included are antibody-drug conjugates. The combination therapy may include a therapeutic agent which is a non-drug treatment. For example, the compound could be administered in addition to radiation therapy, cryotherapy, hyperthermia, and/or surgical excision of tumor tissue. In certain embodiments the first and second therapeutic agents are administered simultaneously or sequentially, in either order. The first therapeutic agent may be administered immediately, up to 1 hour, up to 2 hours, up to 3 hours, up to 4 hours, up to 5 hours, up to 6 hours, up to 7 hours, up to, 8 hours, up to 9 hours, up to 10 hours, up to 11 hours, up to 12 hours, up to 13 hours, 14 hours, up to hours 16, up to 17 hours, up 18 hours, up to 19 hours up to 20 hours, up to 21 hours, up to 22 hours, up to 23 hours up to 24 hours or up to 1-7, 1-14, 1-21 or 1-30 days before or after the second therapeutic agent. In certain embodiments the second therapeutic agent is administered on a different dosage schedule than the compound of the present invention. For example the second therapeutic agent may have a treatment holiday of 1 day, 2 days, 3 days, 4 days, 5 days, 6 days, 7 days, 8 days, 9 days, 10 days, 11 days, 12 days, 13 days, or 14 days per treatment cycle. In another embodiment the first therapeutic agent has a treatment holiday. For example the first therapeutic agent may have a treatment holiday of 1 day, 2 days, 3 days, 4 days, 5 days, 6 days, 7 days, 8 days, 9 days, 10 days, 11 days, 12 days, 13 days, or 14 days per treatment cycle. In certain embodiments both the first and second therapeutic have a treatment holiday.

**Dosage Forms and Administration**

A compound of the present disclosure, e.g., a compound of Formula I, I(a), I(b), I(c), I(d), I(e), I(f), I(g), and/or I(h), or its pharmaceutically acceptable salt thereof, as described herein can be administered as the neat chemical, but is more typically administered as a pharmaceutical composition, that includes an effective amount for a patient, typically a human, in need of such treatment for any of the disorders described herein. Accordingly, the disclosure provides pharmaceutical compositions comprising an effective amount of compound or pharmaceutically acceptable salt together with at least one pharmaceutically acceptable carrier for any of the uses described herein. The pharmaceutical composition may contain a compound or salt as the only active agent, or, in an alternative embodiment, the compound and at least one additional active agent.

In general, the compositions of the disclosure will be administered in a therapeutically effective amount by any of the accepted modes of administration. Suitable dosage ranges depend upon numerous factors such as the severity of the disease to be treated, the age and relative health of the subject, the potency of the compound used, the route and form of administration, the indication towards which the administration is directed, and the preferences and experience of the medical practitioner involved. One of ordinary skill in the art of treating such diseases will be able, without undue experimentation and in reliance upon personal knowledge and the disclosure of this application, to ascertain a therapeutically effective amount of the compositions of the disclosure for a given disease.

In certain embodiments the pharmaceutical composition is in a dosage form that contains from about 0.1 mg to about 2000 mg, from about 10 mg to about 1000 mg, from about 100 mg to about 800 mg, or from about 200 mg to about 600 mg of the active compound and optionally from about 0.1 mg to about 2000 mg, from about 10 mg to about 1000 mg, from about 100 mg to about 800 mg, or from about 200 mg to about 600 mg of an additional active agent in a unit dosage form. Examples are dosage forms with at least about 0.1, 1, 5, 10, 25, 50, 100, 200, 250, 300, 400, 500, 600, 700, or 750 mg of active compound, or its salt.

In certain embodiments the patient can be treated with low dosage therapy with a compound of the present invention. For example, the pharmaceutical composition can be in a dosage form that contains from about 0.1 μg to about 2000 μg, from about 10 μg to about 1000 μg, from about 100 μg to about 800 μg, or from about 200 μg to about 600 μg of the active compound. Examples are dosage forms with at least about 0.1, 1, 5, 10, 25, 50, 100, 200, 250, 300, 400, 500, 600, 700, or 750 μg of active compound, or its salt.

In certain embodiments the dose ranges from about 0.01-100 mg/kg of patient body weight, for example at least about 0.01 mg/kg, at least about 0.05 mg/kg, at least about 0.1 mg/kg, at least about 0.5 mg/kg, at least about 1 mg/kg, at least about 1.5 mg/kg, at least about 2 mg/kg, at least about 2.5 mg/kg, at least about 3 mg/kg, at least about 3.5 mg/kg, at least about 4 mg/kg, at least about 4.5 mg/kg, at least about 5 mg/kg, at least about 10 mg/kg, at least about 15 mg/kg, at least about 20 mg/kg, at least about 25 mg/kg, at least about 30 mg/kg, at least about 35 mg/kg, at least about 40 mg/kg, at least about 45 mg/kg, at least about 50 mg/kg, at least about 55 mg/kg, at least about 60 mg/kg, at least about 65 mg/kg, at least about 70 mg/kg, at least about 75 mg/kg, at least about 80 mg/kg, at least about 85 mg/kg, at least about 90 mg/kg, at least about 95 mg/kg, or at least about 100 mg/kg.

A pharmaceutically or therapeutically effective amount of the composition will be delivered to the patient. The precise effective amount will vary from patient to patient, and will depend upon the species, age, the subject's size and health, the nature and extent of the condition being treated, recommendations of the treating physician, and the therapeutics or combination of therapeutics selected for administration. The effective amount for a given situation can be determined by routine experimentation. For purposes of the disclosure, a therapeutic amount may for example be in the range of about 0.01 mg/kg to about 250 mg/kg body weight, more typically about 0.1 mg/kg to about 10 mg/kg, in at least one dose. The subject can be administered as many doses as is required to reduce and/or alleviate the signs, symptoms, or causes of the disorder in question, or bring about any other desired alteration of a biological system. When desired, formulations can be prepared with enteric coatings adapted for sustained or controlled release administration of the active ingredient.

In some embodiments, compounds disclosed herein or used as described are administered once a day (QD), twice a day (BID), or three times a day (TID). In some embodiments, compounds disclosed herein or used as described are administered at least once a day for at least 1 day, at least 2 days, at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 26 days, at least 27 days, at least 28 days, at least 29 days, at least 30 days, at least 31 days, at least 35 days, at least 45 days, at least 60 days, at least 75 days, at least 90 days, at least 120 days, at least 150 days, at least 180 days, or longer.

In certain embodiments the compound of the present invention is administered once a day, twice a day, three times a day, or four times a day. In certain embodiments the compound of the present invention is administered orally once a day. In certain embodiments the compound of the present invention is administered orally twice a day. In certain embodiments the compound of the present invention is administered orally three times a day. In certain embodiments the compound of the present invention is administered orally four times a day.

In certain embodiments the compound of the present invention is administered intravenously once a day. In certain embodiments the compound of the present invention is administered intravenously twice a day. In certain embodiments the compound of the present invention is administered intravenously three times a day. In certain embodiments the compound of the present invention is administered intravenously four times a day.

In some embodiments the compound of the present invention is administered with a treatment holiday in between treatment cycles. For example, the compound may have a treatment holiday of 1 day, 2 days, 3 days, 4 days, 5 days, 6 days, 7 days, 8 days, 9 days, 10 days, 11 days, 12 days, 13 days, or 14 days per treatment cycle.

The pharmaceutical composition may also include a molar ratio of the active compound and an additional active agent. For example, the pharmaceutical composition may contain a molar ratio of about 0.5:1, about 1 : 1, about 2: 1, about

3:1 or from about 1.5: 1 to about 4: 1 of an antiinflammatory or immunosuppressing agent.

These compositions can contain any amount of active compound that achieves the desired result, for example between 0.1 and 99 weight % (wt. %) of the compound and usually at least about 5 wt. % of the compound. Some embodiments contain from about 25 wt. % to about 50 wt. % or from about 5 wt. % to about 75 wt. % of the compound.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

In certain embodiments the compound is administered as a pharmaceutically acceptable salt. Non-limiting examples of pharmaceutically acceptable salts include: acetate, adipate, alginate, ascorbate, aspartate, benzenesulfonate, benzoate, bisulfate, borate, butyrate, camphorate, camphorsulfonate, citrate, cyclopentanepropionate, digluconate, dodecyl sulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3 -phenylpropionate, phosphate, picrate, pivalate, propionate, stearate, succinate, sulfate, tartrate, thiocyanate, toluenesulfonate, undecanoate, and valerate salts. Representative alkali or alkaline earth metal salts include sodium, lithium, potassium, calcium, and magnesium, as well as nontoxic ammonium, quaternary ammonium, and amine cations, including, but not limited to ammonium, tetramethylammonium, tetraethylammonium, methylamine, dimethylamine, trimethylamine, triethylamine, and ethylamine.

Thus, the composition of the disclosure can be administered as a pharmaceutical formulation including one suitable for oral (including buccal and sub-lingual), rectal, nasal, topical, transdermal, pulmonary, vaginal or parenteral (including intramuscular, intra-arterial, intrathecal, subcutaneous and intravenous), injections, inhalation or spray, intra-aortal, intracranial, subdermal, intraperitioneal, subcutaneous, or by other means of administration containing conventional pharmaceutically acceptable carriers. A typical manner of administration is oral, topical or intravenous, using a convenient daily dosage regimen which can be adjusted according to the degree of affliction.

Depending on the intended mode of administration, the pharmaceutical compositions can be in the form of solid, semi-solid or liquid dosage forms, such as, for example, tablets, suppositories, pills, capsules, powders, liquids, syrup, suspensions, creams, ointments, lotions, paste, gel, spray, aerosol, foam, or oil, injection or infusion solution, a transdermal patch, a subcutaneous patch, an inhalation formulation, in a medical device, suppository, buccal, or sublingual formulation, parenteral formulation, or an ophthalmic solution, or the like, preferably in unit dosage form suitable for single administration of a precise dosage.

Some dosage forms, such as tablets and capsules, are subdivided into suitably sized unit doses containing appropriate quantities of the active components, e.g., an effective amount to achieve the desired purpose. The compositions will include an effective amount of the selected drug in combination with a pharmaceutically acceptable carrier and, in addition, can include other pharmaceutical agents, adjuvants, diluents, buffers, and the like. Carriers include excipients and diluents and must be of sufficiently high purity and sufficiently low toxicity to render them suitable for administration to the patient being treated. The carrier can be inert or it can possess pharmaceutical benefits of its own. The amount of carrier employed in conjunction with the compound is sufficient to provide a practical quantity of material for administration per unit dose of the compound. Classes of carriers include, but are not limited to adjuvants, binders, buffering agents, coloring agents, diluents, disintegrants, excipients, emulsifiers, flavorants, gels, glidents, lubricants, preservatives, stabilizers, surfactants, solubilizer, tableting agents, wetting agents or solidifying material. Some carriers may be listed in more than one class, for example vegetable oil may be used as a lubricant in some formulations and a diluent in others. Exemplary pharmaceutically acceptable carriers include sugars, starches, celluloses, powdered tragacanth, malt, gelatin; talc, petroleum jelly, lanoline, polyethylene glycols, alcohols, transdermal enhancers and vegetable oils. Optional active agents may be included in a pharmaceutical composition, which do not substantially interfere with the activity of the compound of the present invention. Some excipients include, but are not limited, to liquids such as water, saline, glycerol, polyethylene glycol, hyaluronic acid, ethanol, and the like. The compound can be provided, for example, in the form of a solid, a liquid, spray dried material, a microparticle, nanoparticle, controlled release system, etc., as desired according to the goal of the therapy. Suitable excipients for non-liquid formulations are also known to those of skill in the art. A thorough discussion of pharmaceutically acceptable excipients and salts is available in Remington's Pharmaceutical Sciences, 18th Edition (Easton, Pennsylvania: Mack Publishing Company, 1990). Additionally, auxiliary substances, such as wetting or emulsifying agents, biological buffering substances, surfactants, and the like, can be present in such vehicles. A biological buffer can be any solution which is pharmacologically acceptable, and which provides the formulation with the desired pH, i.e., a pH in the physiologically acceptable range. Examples of buffer solutions include saline, phosphate buffered saline, Tris buffered saline, Hank's buffered saline, and the like. For solid compositions, conventional nontoxic solid carriers include, for example, pharmaceutical grades of mannitol, lactose, starch, magnesium stearate, sodium saccharin, talc, cellulose, glucose, sucrose, magnesium carbonate, and the like.

Liquid pharmaceutically administrable compositions can, for example, be prepared by dissolving, dispersing, and the like, an active compound as described herein and optional pharmaceutical adjuvants in an excipient, such as, for example, water, saline, aqueous dextrose, glycerol, ethanol, and the like, to thereby form a solution or suspension. If desired, the pharmaceutical composition to be administered can also contain minor amounts of nontoxic auxiliary substances such as wetting or emulsifying agents, pH buffering agents and the like, for example, sodium acetate, sorbitan monolaurate, triethanolamine sodium acetate, triethanolamine oleate, and the like. Actual methods of preparing such dosage forms are known, or will be apparent, to those skilled in this art; for example, see Remington's Pharmaceutical Sciences, referenced above. In yet another embodiment provided is the use of permeation enhancer excipients including polymers such as: polycations (chitosan and its quaternary ammonium derivatives, poly-L-arginine, aminated gelatin); polyanions (N-carboxymethyl chitosan, poly-acrylic acid); and, thiolated polymers (carboxymethyl cellulose-cysteine, polycarbophil-cysteine, chitosan-thiobutylamidine, chitosan-thioglycolic acid, chitosan-glutathione conjugates). The pharmaceutical compositions/combinations can be formulated for oral administration. For oral administration, the composition will generally take the form of a tablet, capsule, a softgel capsule or can be an aqueous or nonaqueous solution, suspension or syrup. Tablets and capsules are typical oral administration forms. Tablets and capsules for oral use can include one or more commonly used carriers such as lactose and corn starch. Lubricating agents, such as magnesium stearate, are also typically added. Typically, the compositions of the disclosure can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like. Moreover, when desired or necessary, suitable binders, lubricants, disintegrating agents, and coloring agents can also be incorporated into the mixture. Suitable binders include starch, gelatin, natural sugars such as glucose or beta-lactose, corn sweeteners, natural and synthetic gums such as acacia, tragacanth, or sodium alginate, carboxymethylcellulose, polyethylene glycol, waxes, and the like. Lubricants used in these dosage forms include sodium oleate, sodium stearate, magnesium stearate, sodium benzoate, sodium acetate, sodium chloride, and the like. Disintegrators include, without limitation, starch, methyl cellulose, agar, bentonite, xanthan gum, and the like. When liquid suspensions are used, the active agent can be combined with any oral, nontoxic, pharmaceutically acceptable inert carrier such as ethanol, glycerol, water, and the like and with emulsifying and suspending agents. If desired, flavoring, coloring and/or sweetening agents can be added as well. Other optional components for incorporation into an oral formulation herein include, but are not limited to, preservatives, suspending agents, thickening agents, and the like.

For ocular delivery, the compound can be administered, as desired, for example, via intravitreal, intrastromal, intracameral, sub-tenon, sub-retinal, retro-bulbar, peribulbar, suprachorodial, conjunctival, subconjunctival, episcleral, periocular, transscleral, retrobulbar, posterior juxtascleral, circumcorneal, or tear duct injections, or through a mucus, mucin, or a mucosal barrier, in an immediate or controlled release fashion or via an ocular device.

Parenteral formulations can be prepared in conventional forms, either as liquid solutions or suspensions, solid forms suitable for solubilization or suspension in liquid prior to injection, or as emulsions. Typically, sterile injectable suspensions are formulated according to techniques known in the art using suitable carriers, dispersing or wetting agents and suspending agents. The sterile injectable formulation can also be a sterile injectable solution or a suspension in a acceptably nontoxic parenterally acceptable diluent or solvent. Among the acceptable vehicles and solvents that can be employed are water, Ringer's solution and isotonic sodium chloride solution. In addition, sterile, fixed oils, fatty esters or polyols are conventionally employed as solvents or suspending media. In addition, parenteral administration can involve the use of a slow release or sustained release system such that a constant level of dosage is maintained.

Parenteral administration includes intraarticular, intravenous, intramuscular, intradermal, intraperitoneal, and subcutaneous routes, and include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. Administration via certain parenteral routes can involve introducing the formulations of the disclosure into the body of a patient through a needle or a catheter, propelled by a sterile syringe or some other mechanical device such as a continuous infusion system. A formulation provided by the disclosure can be administered using a syringe, injector, pump, or any other device recognized in the art for parenteral administration. Preparations according to the disclosure for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms can also contain adjuvants such as preserving, wetting, emulsifying, and dispersing agents. They can be sterilized by, for example, filtration through a bacteria retaining filter, by incorporating sterilizing agents into the compositions, by irradiating the compositions, or by heating the compositions. They can also be manufactured using sterile water, or some other sterile injectable medium, immediately before use.

Sterile injectable solutions are prepared by incorporating one or more of the compounds of the disclosure in the required amount in the appropriate solvent with various of the other ingredients enumerated above, as required, followed by filtered sterilization. Generally, dispersions are prepared by incorporating the various sterilized active ingredients into a

sterile vehicle which contains the basic dispersion medium and the required other ingredients from those enumerated above. In the case of sterile powders for the preparation of sterile injectable solutions, typical methods of preparation are vacuum-drying and freeze-drying techniques which yield a powder of the active ingredient plus any additional desired ingredient from a previously sterile- filtered solution thereof. Thus, for example, a parenteral composition suitable for administration by injection is prepared by stirring 1.5% by weight of active ingredient in 10% by volume propylene glycol and water. The solution is made isotonic with sodium chloride and sterilized.

Alternatively, the pharmaceutical compositions of the disclosure can be administered in the form of suppositories for rectal administration. These can be prepared by mixing the agent with a suitable nonirritating excipient which is solid at room temperature but liquid at the rectal temperature and therefore will melt in the rectum to release the drug. Such materials include cocoa butter, beeswax and polyethylene glycols.

The pharmaceutical compositions of the disclosure can also be administered by nasal aerosol or inhalation. Such compositions are prepared according to techniques well-known in the art of pharmaceutical formulation and can be prepared as solutions in saline, employing benzyl alcohol or other suitable preservatives, absorption promoters to enhance bioavailability, propellants such as fluorocarbons or nitrogen, and/or other conventional solubilizing or dispersing agents. Formulations for buccal administration include tablets, lozenges, gels and the like. Alternatively, buccal administration can be effected using a transmucosal delivery system as known to those skilled in the art. The compounds of the disclosure can also be delivered through the skin or muscosal tissue using conventional transdermal drug delivery systems, i.e., transdermal "patches" wherein the agent is typically contained within a laminated structure that serves as a drug delivery device to be affixed to the body surface. In such a structure, the drug composition is typically contained in a layer, or "reservoir," underlying an upper backing layer. The laminated device can contain a single reservoir, or it can contain multiple reservoirs. In one embodiment, the reservoir comprises a polymeric matrix of a pharmaceutically acceptable contact adhesive material that serves to affix the system to the skin during drug delivery. Examples of suitable skin contact adhesive materials include, but are not limited to, polyethylenes, polysiloxanes, polyisobutylenes, polyacrylates, polyurethanes, and the like.

Alternatively, the drug-containing reservoir and skin contact adhesive are present as separate and distinct layers, with the adhesive underlying the reservoir which, in this case, can be either a polymeric matrix as described above, or it can be a liquid or gel reservoir, or can take some other form. The backing layer in these laminates, which serves as the upper surface of the device, functions as the primary structural element of the laminated structure and provides the device with much of its flexibility. The material selected for the backing layer should be substantially impermeable to the active agent and any other materials that are present.

The compositions of the disclosure can be formulated for aerosol administration, particularly to the respiratory tract and including intranasal administration. The compound may, for example generally have a small particle size for example of the order of 5 microns or less. Such a particle size can be obtained by means known in the art, for example by micronization. The active ingredient is provided in a pressurized pack with a suitable propellant such as a chlorofluorocarbon (CFC) for example dichlorodifluoromethane, tri chlorofluoromethane, or dichlorotetrafluoroethane, carbon dioxide or other suitable gas. The aerosol can conveniently also contain a surfactant such as lecithin. The dose of drug can be controlled by a metered valve.

Alternatively, the active ingredients can be provided in a form of a dry powder, for example a powder mix of the compound in a suitable powder base such as lactose, starch, starch derivatives such as hydroxypropylmethyl cellulose and polyvinylpyrrolidine (PVP). The powder carrier will form a gel in the nasal cavity. The powder composition can be presented in unit dose form for example in capsules or cartridges of e.g., gelatin or blister packs from which the powder can be administered by means of an inhaler.

Formulations suitable for rectal administration are typically presented as unit dose suppositories. These may be prepared by admixing the active compound with one or more conventional solid carriers, for example, cocoa butter, and then shaping the resulting mixture.

In certain embodiments, the pharmaceutical composition is suitable for topical application to the skin using a mode of administration and defined above.

In certain embodiments, the pharmaceutical composition is suitable for transdermal administration may be presented as discrete patches adapted to remain in intimate contact with the epidermis of the recipient for a prolonged period of time. Formulations suitable for transdermal administration may also be delivered by iontophoresis (see, for example, Pharmaceutical Research 3 (6):318 (1986)) and typically take the form of an optionally buffered aqueous solution of the active compound.

In one embodiment, microneedle patches or devices are provided for delivery of drugs across or into biological tissue, particularly the skin. The microneedle patches or devices permit drug delivery at clinically relevant rates across or into skin or other tissue barriers, with minimal or no damage, pain, or irritation to the tissue.

Formulations suitable for administration to the lungs can be delivered by a wide range of passive breath driven and active power driven single/-multiple dose dry powder inhalers (DPI). The devices most commonly used for respiratory delivery include nebulizers, metered-dose inhalers, and dry powder inhalers. Several types of nebulizers are available,

including jet nebulizers, ultrasonic nebulizers, and vibrating mesh nebulizers. Selection of a suitable lung delivery device depends on parameters, such as nature of the drug and its formulation, the site of action, and pathophysiology of the lung.

## EXAMPLES

The compounds described herein can be made using conventional organic syntheses and commercially available starting materials, or the methods provided herein. By way of example and not limitation, compounds of formula I, can be prepared as outlined in the examples set forth herein. It should be noted that one skilled in the art would know how to modify the procedures set forth in the examples to arrive at the desired products.

### Analytical Procedures

NMR

The following conditions were used for obtaining proton nuclear magnetic resonance (NMR) spectra: NMR spectra were taken in either 400 MHz or 500 MHz Bruker instrument using either DMSO-$d_6$ or $CDCl_3$ as solvent and internal standard. The crude NMR data was analyzed by using either ACD Spectrus version 2015-01 by ADC Labs or MestReNova software.

Chemical shifts are reported in parts per million (ppm) downfield from internal tetramethylsilane (TMS) or from the position of TMS inferred by the deuterated NMR solvent. Apparent multiplicities are reported as: singlet-s, doublet-d, triplet-t, quartet-q, or multiplet-m. Peaks that exhibit broadening are further denoted as br. Integrations are approximate. It should be noted that integration intensities, peak shapes, chemical shifts and coupling constants can be dependent on solvent, concentration, temperature, pH, and other factors. Further, peaks that overlap with or exchange with water or solvent peaks in the NMR spectrum may not provide reliable integration intensities. In some cases, NMR spectra may be obtained using water peak suppression, which may result in overlapping peaks not being visible or having altered shape and/or integration.

### Liquid chromatography

The following preparative and/or analytical (LC/MS) liquid chromatography methods were used.

Method A: Column: XBridge C18, 2.1 mm x 50 mm, 1.7 μm particles; Mobile Phase A: ACN/$H_2O$ (5:95) with 10 mM AA; Mobile Phase B: ACN/$H_2O$ (95:5) with 10 mM AA; Temperature: 50 °C; Gradient: 0-100 %B (0.0-3.0 min), 100 %B (3.0-3.5 min); Flow: 1.0 mL/min; Detection: UV (220 nm) and MS (ESI +).

Method B: Column: XBridge C18, 2.1 mm x 50 mm, 1.7 μm particles; Mobile Phase A: ACN/$H_2O$ (5:95) with 0.05 % TFA; Mobile Phase B: ACN/$H_2O$ (95:5) with 0.05 % TFA; Temperature: 50 °C; Gradient: 0-100 %B (0.0-3.0 min), 100 %B (3.0-3.5 min); Flow: 1.0 mL/min; Detection: UV (220 nm) and MS (ESI +).

Method Column 6: Column: Waters Acquity BEH C18 2.1 x 50 mm 1.7 μm particles; Mobile Phase A: 95:5 acetonitrile: water with 0.05% TFA; Mobile Phase B: 95:5 acetonitrile: water with 0.05% TFA; Temperature: 50 °C; Gradient: 0 %B to 100 %B over 1.00 min, then a 0.50 min hold at 100 %B; Flow: 1.0 mL/min; Detection: MS and UV (254 nm).

UHPLC Method D: Column: Waters Acquity BEH C18 2.1 x 50 mm 1.7 μm particles; Mobile Phase A: 95:5 acetonitrile: water with 0.05% TFA; Mobile Phase B: 95:5 acetonitrile: water with 0.05% TFA; Temperature: 50 °C; Gradient: 0 %B to 100 %B over 3.00 min, then a 0.50 min hold at 100 %B; Flow: 1.0 mL/min; Detection: MS and UV (254 nm).

Method P: Mode: Binary gradient, Pump A: LC-20ADXR, Pump B: LC-20ADXR, Total Flow: 1.5000 mL/min, B Conc.: 30.0 %, Oven Temperature: 40 °C, PDA Model: SPD-M20A, Lamp: D2, Start Wavelength: 190 nm, End Wavelength: 400 nm, Column Name: XBridge BEH Shield RP18, Length: 30 mm, Internal Diameter: 4.6 mm, Description: 2.5 um particles, Mobile Phase A: Water/5mM $NH_4HCO_3$, Mobile Phase B: Acetonitrile; Acquisition Mode: Scan, Polarity: Positive.

Method Q: Mode: Binary gradient, Pump A: LC-40D XR, Pump B: LC-40D XR; Oven Temperature: 40 °C; PDA Model: SPD-M20A, Lamp: D2, Start Wavelength: 190 nm, End Wavelength: 400 nm; Column Name: ACE Excel 2C18, Length: 30 mm, Internal Diameter: 3.0 mm, Column Particle Size: 2.0 um, Mobile phase A: Water + 0.05% TFA, Mobile phase B:Acetonitrile + 0.05% TFA, Start Time: 0.00 min, End Time: 3.00 min, Acquisition Mode: Scan, Polarity: Positive; LCMS5: Waters Acquity BEH C18 2.1 x 50 mm 1.7 μm particles; Mobile Phase A: 95:5 water: acetonitrile with 0.05% TFA; Mobile Phase B: 95:5 acetonitrile: water with 0.05% TFA; Temperature: 50 °C; Gradient: 0 %B to 100 %B over 2.00 min, then a 0.50 min hold at 100 %B; Flow: 1.0 mL/min; Detection: MS and UV (254 nm)

## Acronyms and Abbreviations

Table 2 provides a list of acronyms and abbreviations used in this specification, along with their meanings.

Table 2

| ACRONYM OR ABBREVIATION | MEANING OR DEFINITION |
|---|---|
| Ac | Acetyl |
| ACN, MeCN | Acetonitrile |
| Aq. | Aqueous |
| Boc | *t*-Butyloxycarbonyl |
| BOP | (benzotriazol-1-yloxytris(dimethylamino)phosphonium hexafluorophosphate |
| DCM | Dichloromethane |
| DIPEA, DIEA | N,N-diisopropylethylamine, also known as Hünig's base |
| DMA | N,N-Dimethylacetamide |
| DMAP | 4-(Dimethylamino)pyridine |
| DMF | N,N-dimethylformamide |
| DMSO | Dimethyl sulfoxide |
| EA, EtOAc | Ethyl acetate |
| h | Hour |
| HPLC | High pressure liquid chromatography |
| Hunig's base | See DIPEA, DIEA |
| IBX | 2-Iodoxybenzoic acid |
| LCMS, LC-MS, LC/MS | Liquid chromatography-mass spectrometry |
| min | Minute |
| MS | Mass spectrometry |
| NBS | N-bromosuccinimide |
| NIS | N-iodosuccinimide |
| NMM | N-methylmorpholine |
| NMO | N-Methylmorpholine N-oxide |
| NMR | Nuclear magnetic resonance |
| PE | Petroleum ether |
| Prep | Preparative |
| RT (in context of liquid chromatography) | Retention time, in min |
| rt or RT (in the context of reaction conditions) | Room (ambient) temperature, circa 25 °C |
| Sat. | Saturated |
| Soln | Solution |
| T3P | Propanephosphonic acid anhydride |
| TCFH | N,N,N',N-tetramethylchloroformamidinium hexafluorophosphate |
| TEA | Triethylamine |
| TFA | Trifluoroacetic acid |
| THF | Tetrahydrofuran |

**Example 1: SYNTHESIS OF 4-amino-6-((1-(7-(4-(2-(2,6-dioxopiperidin-3-yl)-1-oxoisoindolin-5-yl)piperazin-1-yl)heptanoyl)piperidin-4-yl)ethynyl)-7-isopropyl-N-(4-(methoxymethyl)phenyl)pyrrolo[2,1-f][1,2,4]triazine-5-carboxamide.**

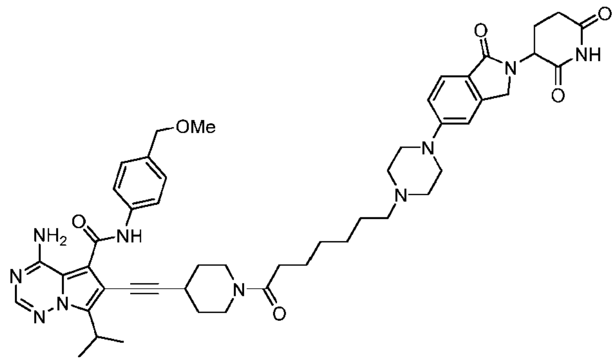

To a sealable reaction vial containing the TFA salt of 7-(4-(2-(2,6-dioxopiperidin-3-yl)-1-oxoisoindolin-5-yl)piperazin-1-yl)heptanoic acid (12.1 mg, 0.021 mmol) in DMF (500 μl) was added DIPEA (15 μl, 0.085 mmol) followed by the addition of BOP (14.07 mg, 0.032 mmol). The reaction mixture was stirred at room temperature for 1 min after which the TFA salt of 4-amino-7-isopropyl-N-(4-(methoxymethyl)phenyl)-6-(piperidin-4-ylethynyl)pyrrolo[2,1-f][1,2,4]triazine-5-carboxamide (19.87 mg, 0.032 mmol) was added to the solution and stirred for 2 hours. The reaction was then diluted with 1 mL of DMF followed by the addition of acetic acid (6.07 μl, 0.106 mmol). The crude material then was purified via preparative Reverse Phase chromatography with the following conditions: Column: XBridge C18, 19 mm x 200 mm, 5 μm particles; Flow Rate: 20 mL/min; Column Temperature: 25 °C. Mobile Phase A: ACN/H2O (5:95) with 0.05 % TFA; Mobile Phase B: ACN/H2O (95:5) with 0.05 % TFA. Fraction collection was triggered by MS (ESI +). Fractions containing the desired product were combined and dried via centrifugal evaporation to yield the desired compound as the mono TFA salt in 77% yield (16.3 mg) . $^{1}H$ NMR (500 MHz, DMSO-$d_6$) δ 10.99 - 10.91 (m, 1H), 10.09 - 10.00 (m, 1H), 9.11 - 8.92 (m, 1H), 8.36 - 8.18 (m, 1H), 8.05 - 7.98 (m, 1H), 7.70 - 7.64 (m, 2H), 7.63 - 7.57 (m, 1H), 7.35 - 7.29 (m, 2H), 7.19 - 7.12 (m, 2H), 5.09 - 4.99 (m, 1H), 4.42 - 4.20 (m, 4H), 3.85 - 3.52 (m, 2H), 3.30 - 3.27 (m, 2H), 3.26 - 3.01 (m, 5H), 2.95 - 2.82 (m, 1H), 2.44 - 2.22 (m, 3H), 2.02 - 1.64 (m, 5H), 1.58 - 1.45 (m, 4H), 1.44 - 1.39 (m, 6H), 1.34 - 1.28 (m, 4H). Integration low due to overlap with suppressed water and obscurement by DMSO signal. Analytical LCMS ESI-MS(+) m/z 885.3 $[M+H]^+$. Analytical Reverse Phase chromatography was used to determine the final purity. Injection 1 conditions: Column: XBridge C18, 2.1 mm x 50 mm, 1.7 μm particles; Mobile Phase A: ACN/H2O (5:95) with 0.05 % TFA; Mobile PhaseB: ACN/H2O (95:5) with 0.05 % TFA; Temperature: 50 °C; Gradient: 0-100 %B (0.0-3.0 min), 100 %B (3.0-3.5min); Flow: 1.0 mL/min; Detection: UV (220 nm) and MS (ESI +). Injection 1 results: Purity: 98.2%; Observed Mass: 885.3; Retention Time: 1.65 min. Injection 2 conditions: Column: XBridge C18, 2.1 mm x 50 mm, 1.7 μm particles; Mobile Phase A: ACN/H2O (5:95) with 10 mM AA; Mobile Phase B: ACN/H2O (95:5) with 10 mM AA; Temperature: 50 °C; Gradient: 0-100 %B (0.0-3.0 min), 100 %B (3.0-3.5 min); Flow: 1.0 mL/min; Detection: UV (220 nm) and MS (ESI +). Injection 2 results: Purity: 98.9%; Observed Mass: 885.3; Retention Time: 2.09 min.

**Example 2. SYNTHESIS OF 4-amino-6-((1-(7-(4-(2-(2,6-dioxopiperidin-3-yl)-1-oxoisoindolin-5-yl)piperidin-1-yl)heptanoyl)piperidin-4-yl)ethynyl)-7-isopropyl-N-(4-(methoxymethyl)phenyl)pyrrolo[2,1-f][1,2,4]triazine-5-carboxamide**

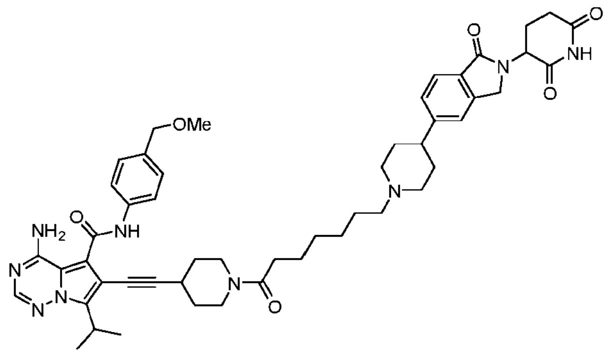

To a sealable reaction vial containing the TFA salt of 7-(4-(2-(2,6-dioxopiperidin-3-yl)-1-oxoisoindolin-5-yl)piperidin-1-yl)heptanoic acid (12.3 mg, 0.022 mmol) in DMF (500 µl) was added DIPEA (15 µl, 0.086 mmol) followed by the addition of BOP (14.33 mg, 0.032 mmol). The reaction mixture was stirred at room temperature for 1 min after which the TFA salt of 4-amino-7-isopropyl-N-(4-(methoxymethyl)phenyl)-6-(piperidin-4-ylethynyl)pyrrolo[2,1-f][1,2,4]triazine-5-carboxamide (20.2 mg, 0.032 mmol) was added to the solution and stirred for 2 hours. The reaction was then diluted with 1 mL of DMF followed by the addition of acetic acid (6.18 µl, 0.108 mmol). The crude material then was purified via preparative Reverse Phase chromatography with the following conditions: Column: XBridge C18, 19 mm x 200 mm, 5 µm particles; Flow Rate: 20 mL/min; Column Temperature: 25 °C. Mobile Phase A: ACN/H2O (5:95) with 0.05 % TFA; Mobile Phase B: ACN/H2O (95:5) with 0.05 % TFA. Fraction collection was triggered by MS (ESI +). Fractions containing the desired product were combined and dried via centrifugal evaporation to yield the desired compound as the mono TFA salt in 69% yield (14.8 mg). $^1$H NMR (500 MHz, DMSO-$d_6$) δ 11.05 - 10.89 (m, 1H), 10.10 - 9.97 (m, 1H), 9.05 - 8.93 (m, 1H), 8.31 - 8.13 (m, 1H), 8.05 - 7.97 (m, 1H), 7.75 - 7.69 (m, 1H), 7.69 - 7.64 (m, 2H), 7.48 (s, 1H), 7.44 - 7.38 (m, 1H), 7.36 - 7.29 (m, 2H), 5.12 - 5.03 (m, 1H), 4.48 - 4.29 (m, 4H), 3.85 - 3.45 (m, 4H), 3.30 - 3.26 (m, 3H), 3.17 - 2.83 (m, 8H), 2.44 - 2.36 (m, 1H), 2.31 - 2.19 (m, 2H), 2.09 - 1.96 (m, 3H), 1.96 - 1.72 (m, 4H), 1.71 - 1.61 (m, 2H), 1.58 - 1.45 (m, 4H), 1.44 - 1.41 (m, 6H), 1.34 - 1.27 (m, 4H). Integration low due to overlap with suppressed water and obscurement by DMSO signal. Analytical LCMS ESI-MS(+) m/z 884.4 [M+H]$^+$. Analytical Reverse Phase chromatography was used to determine the final purity. Injection 1 conditions: Column:XBridge C18, 2.1 mm x 50 mm, 1.7 µm particles; Mobile Phase A: ACN/H2O (5:95) with 0.05 % TFA; Mobile PhaseB: ACN/H2O (95:5) with 0.05 % TFA; Temperature: 50 °C; Gradient: 0-100 %B (0.0-3.0 min), 100 %B (3.0-3.5min); Flow: 1.0 mL/min; Detection: UV (220 nm) and MS (ESI +). Injection 1 results: Purity: 100%; Observed Mass: 884.4; Retention Time: 1.92 min. Injection 2 conditions: Column: XBridge C18, 2.1 mm x 50 mm, 1.7 µm particles; Mobile Phase A: ACN/H2O (5:95) with 10 mM AA; Mobile Phase B: ACN/H2O (95:5) with 10 mM AA; Temperature: 50 °C; Gradient: 0-100 %B (0.0-3.0 min), 100 %B (3.0-3.5 min); Flow: 1.0 mL/min; Detection: UV (220 nm) and MS (ESI +). Injection 2 results: Purity: 100%; Observed Mass: 884.4; Retention Time: 1.6 min.

**Example 3. SYNTHESIS OF 4-amino-6-((1-(7-(4-(2-(2,6-dioxopiperidin-3-yl)-3-oxoisoindolin-5-yl)piperazin-1-yl)heptanoyl)piperidin-4-yl)ethynyl)-7-isopropyl-N-(4-(methoxymethyl)phenyl)pyrrolo[2,1-f][1,2,4]triazine-5-carboxamide**

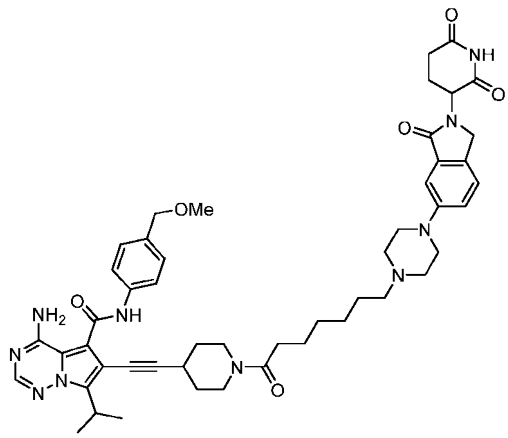

To a sealable reaction vial containing the TFA salt of 7-(4-(2-(2,6-dioxopiperidin-3-yl)-3-oxoisoindolin-5-yl)piperazin-1-yl)heptanoic acid (14.7 mg, 0.026 mmol)in DMF (500 μl) was added DIPEA (18 μl, 0.103 mmol) followed by the addition of BOP (17.09 mg, 0.039 mmol). The reaction mixture was stirred at room temperature for 1 min after which the TFA salt of 4-amino-7-isopropyl-N-(4-(methoxymethyl)phenyl)-6-(piperidin-4-ylethynyl)pyrrolo[2,1-f][1,2,4]triazine-5-carboxamide (24.1 mg, 0.039 mmol) was added to the solution and stirred for 2 hours. The reaction was then diluted with 1 mL of DMF followed by the addition of acetic acid (7.37 μl, 0.129 mmol). The crude material then was purified via preparative Reverse Phase chromatography with the following conditions: Column: XBridge C18, 19 mm x 200 mm, 5 μm particles; Flow Rate: 20 mL/min; Column Temperature: 25 °C. Mobile Phase A: ACN/H2O (5:95) with 0.05 % TFA; Mobile Phase B: ACN/H2O (95:5) with 0.05 % TFA. Fraction collection was triggered by MS (ESI +). Fractions containing the desired product were combined and dried via centrifugal evaporation to yield the desired compound as the mono TFA salt in 50% yield (12.9 mg). $^1$H NMR (500 MHz, DMSO-$d_6$) δ 10.10 - 9.98 (m, 1H), 9.11 - 8.88 (m, 1H), 8.24 - 8.11 (m, 1H), 8.03 - 7.98 (m, 1H), 7.70 - 7.64 (m, 2H), 7.44 - 7.39 (m, 1H), 7.35 - 7.28 (m, 2H), 7.27 - 7.19 (m, 1H), 7.19 - 7.11 (m, 1H), 5.14 - 5.03 (m, 1H), 4.43 - 4.16 (m, 4H), 3.87 - 3.58 (m, 2H), 3.31 - 3.27 (m, 2H), 3.26 - 2.96 (m, 6H), 2.95 - 2.78 (m, 2H), 2.41 - 2.21 (m, 5H), 2.04 - 1.95 (m, 1H), 1.88 - 1.74 (m, 2H), 1.56 - 1.40 (m, 12H), 1.32 - 1.25 (m, 4H). Integration low due to overlap with suppressed water and obscurement by DMSO signal. Analytical LCMS ESI-MS(+) m/z 885.8 [M+H]$^+$. Analytical Reverse Phase chromatography was used to determine the final purity. Injection 1 conditions: Column: XBridge C18, 2.1 mm x 50 mm, 1.7 μm particles; Mobile Phase A: ACN/H2O (5:95) with 0.05 % TFA; Mobile PhaseB: ACN/H2O (95:5) with 0.05 % TFA; Temperature: 50 °C; Gradient: 0-100 %B (0.0-3.0 min), 100 %B (3.0-3.5min); Flow: 1.0 mL/min; Detection: UV (220 nm) and MS (ESI +). Injection 1 results: Purity: 98.2%; Observed Mass: 885.1; Retention Time: 2.06 min. Injection 3 conditions: Column: XBridge C18, 2.1 mm x 50 mm, 1.7 μm particles; Mobile Phase A: ACN/H2O (5:95) with 10 mM AA; Mobile Phase B: ACN/H2O (95:5) with 10 mM AA; Temperature: 50 °C; Gradient: 0-100 %B (0.0-3.0 min), 100 %B (3.0-3.5 min); Flow: 1.0 mL/min; Detection: UV (220 nm) and MS (ESI +). Injection 3 results: Purity: 100%; Observed Mass: 885.8; Retention Time: 1.59 min.

**Example 4. SYNTHESIS OF 4-amino-6-((1-(7-(4-(2-(2,6-dioxopiperidin-3-yl)-3-oxoisoindolin-5-yl)piperidin-1-yl) heptanoyl)piperidin-4-yl)ethynyl)-7-isopropyl-N-(4-(methoxymethyl)phenyl)pyrrolo[2,1-f][1,2,4]triazine-5-carboxamide**

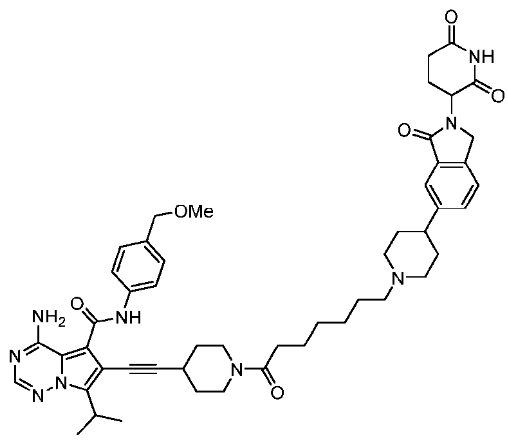

To a sealable reaction vial containing the TFA salt of 7-(4-(2-(2,6-dioxopiperidin-3-yl)-3-oxoisoindolin-5-yl) piperidin-1-yl)heptanoic acid (13.3 mg, 0.023 mmol) in DMF (500 μl) was added DIPEA (16 μl, 0.93 mmol) followed by the addition of BOP (15.4 mg, 0.035 mmol). The reaction mixture was stirred at room temperature for 1 min after which the TFA salt of 4-amino-7-isopropyl-N-(4-(methoxymethyl)phenyl)-6-(piperidin-4-ylethynyl)pyrrolo[2,1-f][1,2,4]triazine-5-carboxamide (21.9 mg, 0.035 mmol) was added to the solution and stirred for 2 hours. The reaction was then diluted with 1 mL of DMF followed by the addition of acetic acid (6.7 μl, 0.117 mmol). The crude material then was purified via preparative Reverse Phase chromatography with the following conditions: Column: XBridge C18, 19 mm x 200 mm, 5 μm particles; Flow Rate: 20 mL/min; Column Temperature: 25 °C. Mobile Phase A: ACN/H2O (5:95) with 0.05 % TFA; Mobile Phase B: ACN/H2O (95:5) with 0.05 % TFA. Fraction collection was triggered by MS (ESI +). Fractions containing the desired product were combined and dried via centrifugal evaporation to yield the desired compound as the mono TFA salt in 58% yield (13.5 mg). $^1$H NMR (500 MHz, DMSO-$d_6$) δ 10.07 - 9.98 (m, 1H), 9.10 - 8.93 (m, 1H), 8.22 - 8.10 (m, 1H), 8.04 - 7.98 (m, 1H), 7.72 - 7.63 (m, 2H), 7.59 - 7.48 (m, 3H), 7.38 - 7.24 (m, 2H), 5.13 - 5.04 (m, 1H), 4.45 - 4.22 (m, 4H), 3.92 - 3.42 (m, 3H), 3.31 - 3.28 (m, 2H), 3.26 - 2.88 (m, 6H), 2.46 - 2.35 (m, 1H), 2.32 - 2.19 (m, 4H), 2.06 - 1.94 (m, 3H), 1.81 - 1.59 (m, 5H), 1.57 - 1.40 (m, 12H), 1.31 - 1.24 (m, 4H). Integration low due to overlap with suppressed water and obscurement by DMSO

signal. Analytical LCMS ESI-MS(+) m/z 884.5 $[M+H]^+$. Analytical Reverse Phase chromatography was used to determine the final purity. Injection 1 conditions: Column:XBridge C18, 2.1 mm x 50 mm, 1.7 μm particles; Mobile Phase A: ACN/H2O (5:95) with 0.05 % TFA; Mobile PhaseB: ACN/H2O (95:5) with 0.05 % TFA; Temperature: 50 °C; Gradient: 0-100 %B (0.0-3.0 min), 100 %B (3.0-3.5min); Flow: 1.0 mL/min; Detection: UV (220 nm) and MS (ESI +). Injection 1 results: Purity: 99.1 %; Observed Mass: 884.3; Retention Time: 1.91 min. Injection 2 conditions: Column: XBridge C18, 2.1 mm x 50 mm, 1.7 μm particles; Mobile Phase A: ACN/H2O (5:95) with 10 mM AA; Mobile Phase B: ACN/H2O (95:5) with 10 mM AA; Temperature: 50 °C; Gradient: 0-100 %B (0.0-3.0 min), 100 %B (3.0-3.5 min); Flow: 1.0 mL/min; Detection: UV (220 nm) and MS (ESI +). Injection 2 results: Purity: 99.3%; Observed Mass: 884.4; Retention Time: 1.61 min.

**Example 5. SYNTHESIS OF 4-amino-6-((1-(5-(4-(2-(2,6-dioxopiperidin-3-yl)-1-oxoisoindolin-5-yl)piperazin-1-yl) pentanoyl)piperidin-4-yl)ethynyl)-7-isopropyl-N-(4-(methoxymethyl)phenyl)pyrrolo[2,1-f][1,2,4]triazine-5-carboxamide**

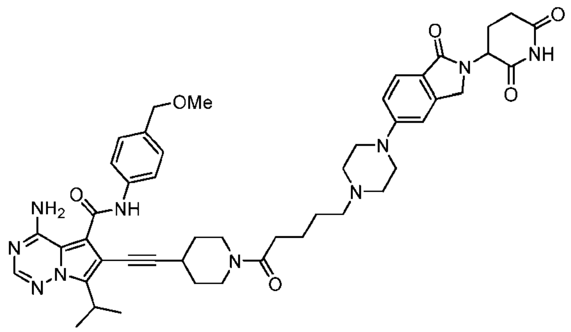

To a sealable reaction vial containing the TFA salt of 5-(4-(2-(2,6-dioxopiperidin-3-yl)-1-oxoisoindolin-5-yl) piperazin-1-yl)pentanoic acid (12 mg, 0.022 mmol) in DMF (500 μl) was added DIPEA (15 μl, 0.088 mmol) followed by the addition of BOP (14.7 mg, 0.033 mmol). The reaction mixture was stirred at room temperature for 1 min after which the TFA salt of 4-amino-7-isopropyl-N-(4-(methoxymethyl)phenyl)-6-(piperidin-4-ylethynyl)pyrrolo[2,1-f][1,2,4]triazine-5-carboxamide (20.7 mg, 0.033 mmol) was added to the solution and stirred for 3 hours. The reaction was then diluted with 1 mL of DMF followed by the addition of acetic acid (6.3 μl, 0.111 mmol). The crude material then was purified via preparative Reverse Phase chromatography with the following conditions: Column: XBridge C18, 19 mm x 200 mm, 5 μm particles; Flow Rate: 20 mL/min; Column Temperature: 25 °C. Mobile Phase A: ACN/H2O (5:95) with 0.05 % TFA; Mobile Phase B: ACN/H2O (95:5) with 0.05 % TFA. Fraction collection was triggered by MS (ESI +). Fractions containing the desired product were combined and dried via centrifugal evaporation to yield the desired compound as the mono TFA salt in 36% yield (7.7 mg). $^1$H NMR (500 MHz, DMSO-$d_6$) δ 10.99 - 10.86 (m, 1H), 10.06 - 9.97 (m, 1H), 9.09 - 8.85 (m, 1H), 8.24 - 8.10 (m, 1H), 8.04 - 7.96 (m, 1H), 7.70 - 7.62 (m, 2H), 7.55 - 7.48 (m, 1H), 7.35 - 7.28 (m, 2H), 7.10 - 6.99 (m, 2H), 5.07 - 4.97 (m, 1H), 4.42 - 4.36 (m, 2H), 4.36 - 4.27 (m, 1H), 4.27 - 4.17 (m, 1H), 3.86 - 3.61 (m, 2H), 3.56 - 3.35 (m, 1H), 3.32 - 3.22 (m, 5H), 3.18 - 3.08 (m, 1H), 3.07 - 3.00 (m, 1H), 2.92 - 2.83 (m, 1H), 2.41 - 2.25 (m, 5H), 2.01 - 1.92 (m, 1H), 1.89 - 1.74 (m, 2H), 1.59 - 1.44 (m, 6H), 1.41 (d, J=7.1 Hz, 6H). Integration low due to overlap with suppressed water and obscurement by DMSO signal. Analytical LCMS ESI-MS(+) m/z 857.4 $[M+H]^+$. Analytical Reverse Phase chromatography was used to determine the final purity. Injection 1 conditions: Column: XBridge C18, 2.1 mm x 50 mm, 1.7 μm particles; Mobile Phase A: ACN/H2O (5:95) with 10 mM AA; Mobile Phase B: ACN/H2O (95:5) with 10 mM AA; Temperature: 50 °C; Gradient: 0-100 %B (0.0-3.0 min), 100 %B (3.0-3.5 min); Flow: 1.0 mL/min; Detection: UV (220 nm) and MS (ESI +). Injection 1 results: Purity: 100%; Observed Mass: 857.3; Retention Time: 1.96 min. Injection 4 conditions: Column: XBridge C18, 2.1 mm x 50 mm, 1.7 μm particles; Mobile Phase A: ACN/H2O (5:95) with 0.05 % TFA; Mobile Phase B: ACN/H2O (95:5) with 0.05 % TFA; Temperature: 50 °C; Gradient: 0-100 %B (0.0-3.0 min), 100 %B (3.0-3.5 min); Flow: 1.0 mL/min; Detection: UV (220 nm) and MS (ESI +). Injection 4 results: Purity: 100%; Observed Mass: 857.4; Retention Time: 1.49 min.

Example 6. SYNTHESIS OF 4-amino-6-((1-(5-(4-(2-(2,6-dioxopiperidin-3-yl)-3-oxoisoindolin-5-yl)piperazin-1-yl)pentanoyl)piperidin-4-yl)ethynyl)-7-isopropyl-N-(4-(methoxymethyl)phenyl)pyrrolo[2,1-f][1,2,4]triazine-5-carboxamide

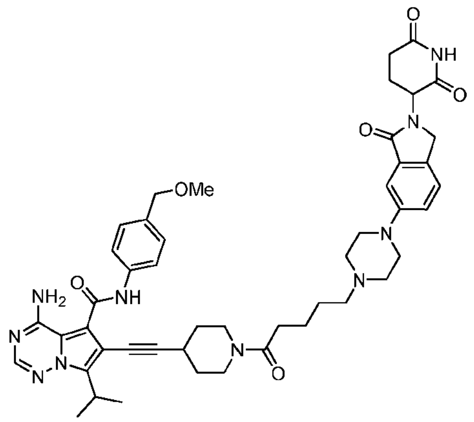

To a sealable reaction vial containing the TFA salt of 5-(4-(2-(2,6-dioxopiperidin-3-yl)-3-oxoisoindolin-5-yl) piperazin-1-yl)pentanoic acid, (10 mg, 0.018 mmol) in DMF (500 µl) was added DIPEA (13 µl, 0.074 mmol) followed by the addition of BOP (12.3 mg, 0.028 mmol). The reaction mixture was stirred at room temperature for 1 min after which the TFA salt of 4-amino-7-isopropyl-N-(4-(methoxymethyl)phenyl)-6-(piperidin-4-ylethynyl)pyrrolo[2,1-f][1,2,4]triazine-5-carboxamide (17.7 mg, 0.028 mmol) was added to the solution and stirred for 3 hours. The reaction was then diluted with 1 mL of DMF followed by the addition of acetic acid (5.3 µl, 0.092 mmol). The crude material then was purified via preparative Reverse Phase chromatography with the following conditions: Column: XBridge C18, 19 mm x 200 mm, 5 µm particles; Flow Rate: 20 mL/min; Column Temperature: 25 °C. Mobile Phase A: ACN/H2O (5:95) with 0.05 % TFA; Mobile Phase B: ACN/H2O (95:5) with 0.05 % TFA. Fraction collection was triggered by MS (ESI +). Fractions containing the desired product were combined and dried via centrifugal evaporation to yield the desired compound as the mono TFA salt in 65% yield (11.6 mg). $^1H$ NMR (500 MHz, DMSO-$d_6$) δ 11.08 - 10.86 (m, 1H), 10.13 - 9.95 (m, 1H), 9.12 - 8.90 (m, 1H), 8.40 - 8.16 (m, 1H), 8.06 - 7.99 (m, 1H), 7.71 - 7.65 (m, 2H), 7.54 - 7.47 (m, 1H), 7.37 - 7.27 (m, 4H), 5.12 - 5.05 (m, 1H), 4.42 - 4.33 (m, 3H), 4.28 - 4.22 (m, 1H), 3.97 - 3.40 (m, 5H), 3.33 - 3.24 (m, 3H), 3.20 - 2.88 (m, 6H), 2.46 - 2.26 (m, 3H), 2.06 - 1.94 (m, 1H), 1.94 - 1.74 (m, 2H), 1.74 - 1.65 (m, 2H), 1.62 - 1.18 (m, 12H). Integration low due to overlap with suppressed water and obscurement by DMSO signal. Analytical LCMS ESI-MS(+) m/z 857.3 $[M+H]^+$. Analytical Reverse Phase chromatography was used to determine the final purity. Injection 1 conditions: Column: XBridge C18, 2.1 mm x 50 mm, 1.7 µm particles; Mobile Phase A: ACN/H2O (5:95) with 10 mM AA; Mobile Phase B: ACN/H2O (95:5) with 10 mM AA; Temperature: 50 °C; Gradient: 0-100 %B (0.0-3.0 min), 100 %B (3.0-3.5 min); Flow: 1.0 mL/min; Detection: UV (220 nm) and MS (ESI +). Injection 1 results: Purity: 100%; Observed Mass: 857.3; Retention Time: 1.93 min. Injection 5 conditions: Column: XBridge C18, 2.1 mm x 50 mm, 1.7 µm particles; Mobile Phase A: ACN/H2O (5:95) with 0.05 % TFA; Mobile Phase B: ACN/H2O (95:5) with 0.05 % TFA; Temperature: 50 °C; Gradient: 0-100 %B (0.0-3.0 min), 100 %B (3.0-3.5 min); Flow: 1.0 mL/min; Detection: UV (220 nm) and MS (ESI +). Injection 5 results: Purity: 100%; Observed Mass: 857.3; Retention Time: 1.5 min.

**Example 7. SYNTHESIS OF 4-amino-6-((1-(5-(4-(2-(2,6-dioxopiperidin-3-yl)-3-oxoisoindolin-5-yl)piperidin-1-yl) pentanoyl)piperidin-4-yl)ethynyl)-7-isopropyl-N-(4-(methoxymethyl)phenyl)pyrrolo[2,1-f][1,2,4]triazine-5-carboxamide**

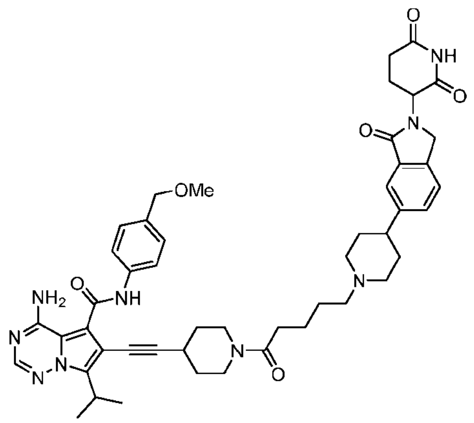

To a sealable reaction vial containing the TFA salt of 5-(4-(2-(2,6-dioxopiperidin-3-yl)-3-oxoisoindolin-5-yl)piperidin-1-yl)pentanoic acid (11.3 mg, 0.021 mmol) in DMF (500 μl) was added DIPEA (15 μl, 0.083 mmol) followed by the addition of BOP (13.8 mg, 0.031mmol). The reaction mixture was stirred at room temperature for 1 min after which the TFA salt of 4-amino-7-isopropyl-N-(4-(methoxymethyl)phenyl)-6-(piperidin-4-ylethynyl)pyrrolo[2,1-f][1,2,4]triazine-5-carboxamide (19.5 mg, 0.031 mmol) was added to the solution and stirred for 3 hours. The reaction was then diluted with 1 mL of DMF followed by the addition of acetic acid (6 μl, 0.104 mmol). The crude material then was purified via preparative Reverse Phase chromatography with the following conditions: Column: XBridge C18, 19 mm x 200 mm, 5 μm particles; Flow Rate: 20 mL/min; Column Temperature: 25 °C. Mobile Phase A: ACN/H2O (5:95) with 0.05 % TFA; Mobile Phase B: ACN/H2O (95:5) with 0.05 % TFA. Fraction collection was triggered by MS (ESI +). Fractions containing the desired product were combined and dried via centrifugal evaporation to yield the desired compound as the mono TFA salt in 65% yield (13.2 mg). $^1H$ NMR (500 MHz, DMSO-$d_6$) δ 11.04 - 10.92 (m, 1H), 10.13 - 9.96 (m, 1H), 9.28 - 8.93 (m, 1H), 8.28 - 8.12 (m, 1H), 8.05 - 7.99 (m, 1H), 7.69 - 7.64 (m, 2H), 7.64 - 7.57 (m, 2H), 7.55 - 7.49 (m, 1H), 7.37 - 7.29 (m, 2H), 5.15 - 5.02 (m, 1H), 4.50 - 4.27 (m, 4H), 3.86 - 3.51 (m, 3H), 3.34 - 3.27 (m, 3H), 3.22 - 2.81 (m, 8H), 2.45 - 2.25 (m, 3H), 2.12 - 1.73 (m, 7H), 1.73 - 1.47 (m, 6H), 1.44 - 1.38 (m, 6H). Integration low due to overlap with suppressed water and obscurement by DMSO signal. Analytical LCMS ESI-MS(+) m/z 856.5 $[M+H]^+$. Analytical LCMS ESI-MS(+) m/z 857.3 $[M+H]^+$. Analytical Reverse Phase chromatography was used to determine the final purity. Injection 1 conditions: Column: XBridge C18, 2.1 mm x 50 mm, 1.7 μm particles; Mobile Phase A: ACN/H2O (5:95) with 10 mM AA; Mobile Phase B: ACN/H2O (95:5) with 10 mM AA; Temperature: 50 °C; Gradient: 0-100 %B (0.0-3.0 min), 100 %B (3.0-3.5 min); Flow: 1.0 mL/min; Detection: UV (220 nm) and MS (ESI +). Injection 1 results: Purity: 98.3%; Observed Mass: 856.3; Retention Time: 1.53 min. Injection 5 conditions: Column: XBridge C18, 2.1 mm x 50 mm, 1.7 μm particles; Mobile Phase A: ACN/H2O (5:95) with 0.05 % TFA; Mobile Phase B: ACN/H2O (95:5) with 0.05 % TFA; Temperature: 50 °C; Gradient: 0-100 %B (0.0-3.0 min), 100 %B (3.0-3.5 min); Flow: 1.0 mL/min; Detection: UV (220 nm) and MS (ESI +). Injection 2 results: Purity: 98.4%; Observed Mass: 856.4; Retention Time: 1.82 min.

**Example 8. SYNTHESIS OF 4-amino-6-((1-(5-(4-(2-(2,6-dioxopiperidin-3-yl)-1-oxoisoindolin-5-yl)piperidin-1-yl)pentanoyl)piperidin-4-yl)ethynyl)-7-isopropyl-N-(4-(methoxymethyl)phenyl)pyrrolo[2,1-f][1,2,4]triazine-5-carboxamide**

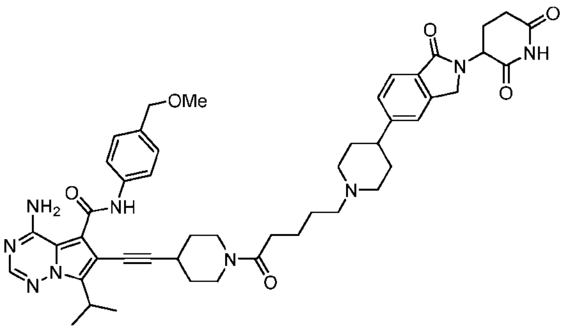

To a sealable reaction vial containing the TFA salt 5-(4-(2-(2,6-dioxopiperidin-3-yl)-1-oxoisoindolin-5-yl)piperidin-1-yl)pentanoic acid (12 mg, 0.022 mmol) of in DMF (500 μl) was added DIPEA (15 μl, 0.088 mmol) followed by the addition of BOP (14.7 mg, 0.033 mmol). The reaction mixture was stirred at room temperature for 1 min after which the TFA salt of 4-amino-7-isopropyl-N-(4-(methoxymethyl)phenyl)-6-(piperidin-4-ylethynyl)pyrrolo[2,1-f][1,2,4]triazine-5-carboxamide (20.7 mg, 0.033 mmol) was added to the solution and stirred for 3 hours. The reaction was then diluted with 1 mL of DMF followed by the addition of acetic acid (6.3 μl, 0.111 mmol). The crude material then was purified via preparative Reverse Phase chromatography with the following conditions: Column: XBridge C18, 19 mm x 200 mm, 5 μm particles; Flow Rate: 20 mL/min; Column Temperature: 25 °C. Mobile Phase A: ACN/H2O (5:95) with 0.05 % TFA; Mobile Phase B: ACN/H2O (95:5) with 0.05 % TFA. Fraction collection was triggered by MS (ESI +). Fractions containing the desired product were combined and dried via centrifugal evaporation to yield the desired compound in as the mono TFA salt 36% yield (7.7 mg). $^1H$ NMR (500 MHz, DMSO-$d_6$) δ 10.10 - 9.95 (m, 1H), 9.09 - 8.88 (m, 1H), 8.22 - 8.12 (m, 1H), 8.04 - 7.98 (m, 1H), 7.70 - 7.61 (m, 3H), 7.49 - 7.43 (m, 1H), 7.40 - 7.36 (m, 1H), 7.34 - 7.27 (m, 2H), 5.14 - 5.03 (m, 1H), 4.43 - 4.20 (m, 4H), 3.93 - 3.57 (m, 3H), 3.32 - 3.18 (m, 3H), 3.18 - 2.85 (m, 5H), 2.77 - 2.60 (m, 2H), 2.41 - 2.24 (m, 5H), 2.02 - 1.92 (m, 3H), 1.81 - 1.66 (m, 4H), 1.60 - 1.40 (m, 12H). Integration low due to overlap with suppressed water and obscurement by DMSO signal. Analytical LCMS ESI-MS(+) m/z 856.5 $[M+H]^+$. Analytical Reverse Phase chromatography was used to determine the final purity. Injection 1 conditions: Column: XBridge C18, 2.1 mm x 50 mm, 1.7 μm particles; Mobile Phase A: ACN/H2O

(5:95) with 0.05 % TFA; Mobile Phase B: ACN/H2O (95:5) with 0.05 % TFA; Temperature: 50 °C; Gradient: 0-100 %B (0.0-3.0 min), 100 %B (3.0-3.5 min); Flow: 1.0 mL/min; Detection: UV (220 nm) and MS (ESI +). Injection 1 results: Purity: 97.1%; Observed Mass: 856.1; Retention Time: 1.66 min. Injection 2 conditions: Column: XBridge C18, 2.1 mm x 50 mm, 1.7 μm particles; Mobile Phase A: ACN/H2O (5:95) with 10 mM AA; Mobile Phase B: ACN/H2O (95:5) with 10 mM AA; Temperature: 50 °C; Gradient: 0-100 %B (0.0-3.0 min), 100 %B (3.0-3.5 min); Flow: 1.0 mL/min; Detection: UV (220 nm) and MS (ESI +/-). Injection 2 results: Purity: 97.4%; Observed Mass: 856.5; Retention Time: 2 min.

**Example 9. SYNTHESIS OF 4-amino-6-((1-(2-(9-(2-(2,6-dioxopiperidin-3-yl)-1-oxoisoindolin-5-yl)-3,9-diazaspiro[5.5]undecan-3-yl)acetyl)piperidin-4-yl)ethynyl)-7-isopropyl-N-(4-(methoxymethyl)phenyl)pyrrolo[2,1-f][1,2,4]triazine-5-carboxamide**

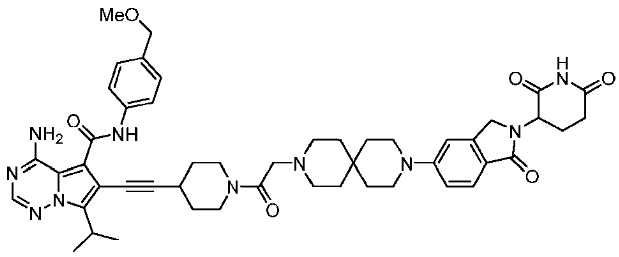

**A. tert-butyl 2-(9-(2-(2,6-dioxopiperidin-3-yl)-1-oxoisoindolin-5-yl)-3,9-diazaspiro[5.5]undecan-3-yl)acetate.** To a stirred solution of 3-(1-oxo-5-(3,9-diazaspiro[5.5]undecan-3-yl)isoindolin-2-yl)piperidine-2,6-dione, HCl (0.020 g, 0.046 mmol) in acetonitrile (0.5 mL) was added tert-butyl 2-bromoacetate (0.012 g, 0.060 mmol), sodium iodide (3.46 mg, 0.023 mmol) and DIPEA (0.020 mL, 0.115 mmol) and the reaction mixture was heated to 70 °C. After 2 hours the solution was cooled to rroom temperature and diluted with 10 ml of dichloromethane. The solution was washed with water and the organic layer was dried over sodium sulfate and evaporated to yield the crude mixture which was directly carried over to the next step without further purification. Analytical LCMS ESI-MS(+) m/z 511.3 $[M+H]^+$.

**B. 2-(9-(2-(2,6-dioxopiperidin-3-yl)-1-oxoisoindolin-5-yl)-3,9-diazaspiro[5.5]undecan-3-yl)acetic acid.** To a sealable reaction vial containing a stirred solution of tert-butyl 2-(9-(2-(2,6-dioxopiperidin-3-yl)-1-oxoisoindolin-5-yl)-3,9-diazaspiro[5.5]undecan-3-yl)acetate (26 mg, 0.051 mmol) in DCM (550 μl) was added 2,2,2-trifluoroacetic acid (0.097 mL, 1.273 mmol) at 0 °C and the reaction mixture was then slowly warmed to room temperature. The reaction was monitored by LCMS and was deemed complete after 6.5 hours. The solvent was then evaporated and the crude reaction mixture was dried overnight in vacuum to get rid of excess TFA. The resulting mono TFA salt of the product was directly used for synthesis of the LDDs without further purification.

**C. 4-amino-6-((1-(2-(9-(2-(2,6-dioxopiperidin-3-yl)-1-oxoisoindolin-5-yl)-3,9-diazaspiro[5.5]undecan-3-yl)acetyl)piperidin-4-yl)ethynyl)-7-isopropyl-N-(4-(methoxymethyl)phenyl)pyrrolo[2,1-f][1,2,4]triazine-5-carboxamide.** To a sealable reaction vial containing the TFA salt of 2-(9-(2-(2,6-dioxopiperidin-3-yl)-1-oxoisoindolin-5-yl)-3,9-diazaspiro[5.5]undecan-3-yl)acetic acid (0.023 g, 0.040 mmol)in DMF (1 ml) was added DIPEA (28 μl, 0.160 mmol) followed by the addition of BOP (27 mg, 0.060 mmol). The reaction mixture was stirred at room temperature for 1 min after which the TFA salt of 4-amino-7-isopropyl-N-(4-(methoxymethyl)phenyl)-6-(piperidin-4-ylethynyl)pyrrolo[2,1-f][1,2,4]triazine-5-carboxamide (37 mg, 0.060 mmol) was added to the solution and stirred for 3 hours. The crude material then was purified via preparative Reverse Phase chromatography with the following conditions: Column: XBridge C18, 19 mm x 200 mm, 5 μm particles; Flow Rate: 20 mL/min; Column Temperature: 25 °C. Mobile Phase A: ACN/H2O (5:95) with 0.05 % TFA; Mobile Phase B: ACN/H2O (95:5) with 0.05 % TFA. Fraction collection was triggered by MS (ESI +). Fractions containing the desired product were combined and dried via centrifugal evaporation to yield the desired compound as the mono TFA salt in 5% yield (2 mg). $^1H$ NMR (500 MHz, DMSO-$d_6$) δ 10.99 - 10.82 (m, 1H), 10.14 - 9.93 (m, 1H), 9.15 - 8.92 (m, 1H), 8.38 - 8.13 (m, 1H), 8.06 - 7.96 (m, 1H), 7.76 - 7.64 (m, 2H), 7.56 - 7.47 (m, 1H), 7.41 - 7.27 (m, 2H), 7.13 - 6.88 (m, 1H), 5.08 - 4.97 (m, 1H), 4.45 - 4.15 (m, 4H), 3.93 - 3.67 (m, 2H), 3.31 - 3.03 (m, 4H), 2.95 - 2.85 (m, 1H), 2.43 - 2.31 (m, 1H), 2.01 - 1.76 (m, 3H), 1.73 - 1.30 (m, 15H). Integration low due to overlap with suppressed water and obscurement by DMSO signal. Analytical LCMS ESI-MS(+) m/z 883.3 $[M+H]^+$. $]^+$. Analytical Reverse Phase chromatography was used to determine the final purity. Injection 1 conditions: Column: XBridge C18, 2.1 mm x 50 mm, 1.7 μm particles; Mobile Phase A: ACN/H2O (5:95) with 10 mM AA; Mobile Phase B: ACN/H2O (95:5) with 10 mM AA; Temperature: 50 °C; Gradient: 0-100 %B (0.0-3.0 min), 100 %B (3.0-3.5 min); Flow:

1.0 mL/min; Detection: UV (220 nm) and MS (ESI +). Injection 1 results: Purity: 100%; Observed Mass: 883.3; Retention Time: 1.46 min. Injection 2 conditions: Column: XBridge C18, 2.1 mm x 50 mm, 1.7 μm particles; Mobile Phase A: ACN/H2O (5:95) with 0.05 % TFA; Mobile Phase B: ACN/H2O (95:5) with 0.05 % TFA; Temperature: 50 °C; Gradient: 0-100 %B (0.0-3.0 min), 100 %B (3.0-3.5 min); Flow: 1.0 mL/min; Detection: UV (220 nm) and MS (ESI +). Injection 2 results: Purity: 100%; Observed Mass: 883.6; Retention Time: 2.08 min.

**Example 10. SYNTHESIS OF 4-amino-6-((1-(3-(9-(2-(2,6-dioxopiperidin-3-yl)-1-oxoisoindolin-5-yl)-3,9-diazaspiro[5.5]undecan-3-yl)propanoyl)piperidin-4-yl)ethynyl)-7-isopropyl-N-(4-(methoxymethyl)phenyl)pyrrolo[2,1-f][1,2,4]triazine-5-carboxamide**

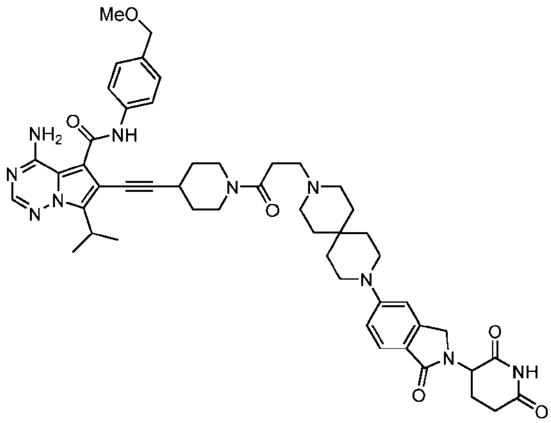

To a sealable reaction vial containing the TFA salt of 3-(9-(2-(2,6-dioxopiperidin-3-yl)-1-oxoisoindolin-5-yl)-3,9-diazaspiro[5.5]undecan-3-yl)propanoic acid (35 mg, 0.050 mmol) in DMF (500 μl) was added DIPEA (35 μl, 0.198 mmol) followed by the addition of BOP (33 mg, 0.074mmol). The reaction mixture was stirred at room temperature for 1 min after which the TFA salt of 4-amino-7-isopropyl-N-(4-(methoxymethyl)phenyl)-6-(piperidin-4-ylethynyl)pyrrolo[2,1-f][1,2,4] triazine-5-carboxamide (46.4 mg, 0.074 mmol) was added to the solution and stirred for 3 hours. The reaction was then diluted with 1 mL of DMF followed by the addition of acetic acid (14 μl, 0.248 mmol). The crude material then was purified via preparative Reverse Phase chromatography with the following conditions: Column: XBridge C18, 19 mm x 200 mm, 5 μm particles; Flow Rate: 20 mL/min; Column Temperature: 25 °C. Mobile Phase A: ACN/H2O (5:95) with 0.05 % TFA; Mobile Phase B: ACN/H2O (95:5) with 0.05 % TFA. Fraction collection was triggered by MS (ESI +). Fractions containing the desired product were combined and dried via centrifugal evaporation to yield the desired compound as the mono TFA salt in 12% yield (5.8 mg). $^1$H NMR (500 MHz, DMSO-$d_6$) δ 10.07 - 9.98 (m, 1H), 9.08 - 8.90 (m, 1H), 8.23 - 8.10 (m, 1H), 8.04 - 7.98 (m, 1H), 7.68 - 7.64 (m, 2H), 7.52 - 7.46 (m, 1H), 7.34 - 7.29 (m, 2H), 7.04 - 6.97 (m, 2H), 5.07 - 4.98 (m, 1H), 4.42 - 4.36 (m, 2H), 4.36 - 4.28 (m, 1H), 4.23 - 4.16 (m, 1H), 3.86 - 3.63 (m, 2H), 3.19 - 2.99 (m, 2H), 2.93 - 2.85 (m, 1H), 2.46 - 2.29 (m, 7H), 2.01 - 1.92 (m, 1H), 1.82 - 1.76 (m, 1H), 1.62 - 1.55 (m, 1H), 1.53 - 1.41 (m, 15H). Integration low due to overlap with suppressed water and obscurement by DMSO signal. Analytical LCMS ESI-MS(+) m/z 856.5 $[M+H]^+$. Analytical Reverse Phase chromatography was used to determine the final purity. Injection 1 conditions: Column: XBridge C18, 2.1 mm x 50 mm, 1.7 μm particles; Mobile Phase A: ACN/H2O (5:95) with 10 mM AA; Mobile Phase B: ACN/H2O (95:5) with 10 mM AA; Temperature: 50 °C; Gradient: 0-100 %B (0.0-3.0 min), 100 %B (3.0-3.5 min); Flow: 1.0 mL/min; Detection: UV (220 nm) and MS (ESI +). Injection 1 results: Purity: 94%; Observed Mass: 897.3; Retention Time: 1.53 min. Injection 2 conditions: Column: XBridge C18, 2.1 mm x 50 mm, 1.7 μm particles; Mobile Phase A: ACN/H2O (5:95) with 0.05 % TFA; Mobile Phase B: ACN/H2O (95:5) with 0.05 % TFA; Temperature: 50 °C; Gradient: 0-100 %B (0.0-3.0 min), 100 %B (3.0-3.5 min); Flow: 1.0 mL/min; Detection: UV (220 nm) and MS (ESI +). Injection 2 results: Purity: 94.1%; Observed Mass: 897.5; Retention Time: 1.89 min.

**Cellular RET Degradation Assay**

Cellular RET degradation compound potency was determined using the bead-based luminescent amplification assay - AlphaLISA Surefire Ultra Detection Kit (Perkin-Elmer). The CCDC6-RET fusion expressing papillary thyroid carcinoma (TPC-1) cell line was selected for screening of potential degraders: Parental (TPC-1/CCDC6-RET). Cells were plated in 384 well culture plates (Greiner Bio-One) at 10,000 cells/well in 50ul/well DMEM (Fisher Scientific) with 2% FBS serum (Gibco). After cellular adhesion (4 hrs), cells were treated with compound by dispensing 25nl of serially diluted

compound (1:3 dilution: 11point) directly into the cell culture wells using an Echo-655 acoustic liquid dispenser (Beckman). Low normalization wells were media only and high normalization wells were cells treated with DMSO only. Treated cell plates were placed in an incubator at 37C and 5% $CO_2$ for 24 hrs. At 24 hrs, cell media was removed by a FELIX automated liquid hander (Analytik Jena) and 25ul of cell lysis buffer (provided in AlphaLisa kit) was added to each well. 5ul cell lysate were transferred into a 384-well microplate (Perkin Elmer) before addition of 2.5ul AlphaLISA Acceptor Bead mix. The plates were sealed and kept at room temperature for 1hr before addition of donor bead mixture. Donor bead addition was performed in the dark and incubated for >1 hr before AlphaLISA luminescence was measured on an Envision plate reader with an Alphascreen aperture at RT (excitation 680 nm, emission 615 nm). RET protein abundance (and therefore degradation) directly corresponds to luminescence intensity.

Intensities were normalized relative to the average values from 16 wells containing media alone (blank-low) and 16 wells containing cells +DMSO (high). % Degradation for each well was determined using the standard equation, where $\%\ Degradation = \frac{total-well\ count}{total-blank} \times 100$. $DC_{50}$ (IC50) values for each compound tested are calculated by analyzing plots of % degradation versus compound concentration, and fitting to the standard 4-parameter logistic curve where, $\%\ Degradation = Bottom + \frac{[Compound]^N \times (Top-Bottom)}{Halfmax^N + [Compound]^N}$. Top and bottom are the % degradation at infinitely large and infinitely small compound concentrations, respectively, while Halfmax is the compound concentration yielding % degradation corresponding to ½ Top-Bottom, and N is the Hill coefficient. $DC_{50}$ values are calculated according to the relationship, $DC_{50} = Halfmax\left(\sqrt[N]{\frac{50-Bottom}{Top-50}}\right)$. Compound replicates were performed in independent assays. Maximum degradation (YMax) was defined as the maximal % degradation value from the 4-parameter logistic equation, as previously described.

**Activity Table.**

Each of the compounds in Table 3, was tested in one or more of the degradation assays shown above, for example, the tRET AlphaLISA Degradation Assay with the parental RET TPC-1 cell line, and was found to have activity therein. The half-maximal degradation concentration ($DC_{50}$) and % degradation response (Ymax) are included in Table 3.

**Table 3**

| **Example #** | Cell Density TPC-1/CCDC6-RET IC50 (nM) | Cell Density TPC-1/CCDC6-RET YMax (%) |
|---|---|---|
| **1** | 28.399 | 84.517 |
| **2** | 11.159 | 87.449 |
| **3** | 228.784 | 91.826 |
| **4** | 37.422 | 97.608 |
| **5** | 18.639 | 79.940 |
| **6** | 6.249 | 97.308 |
| **7** | 11.164 | 90.064 |
| **8** | 23.482 | 86.273 |
| **9** | 17.971 | 79.133 |
| **10** | 11.929 | 78.921 |

**Claims**

**1.** A compound of Formula I:

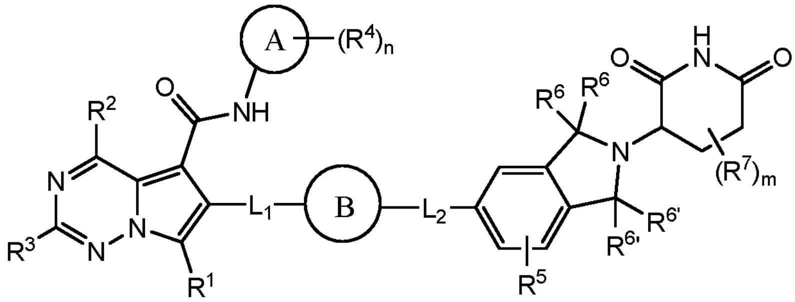

(I),

or a pharmaceutically acceptable salt thereof,
wherein

$R^1$ is $C_1$-$C_6$ alkyl, $C_3$-$C_8$ cycloalkyl, heterocycloalkyl, aryl, or heteroaryl, wherein the alkyl, cycloalkyl, heterocycloalkyl, aryl or heteroaryl is optionally substituted with one or more $R^8$;
A is aryl or heteroaryl, wherein the aryl or heteroaryl is optionally substituted with one or more $R^9$;
B is heterocycloalkyl or $C_3$-$C_8$ cycloalkyl;
$L_1$ is $C_1$-$C_6$ alkyl, $C_2$-$C_6$ alkenyl, or $C_2$-$C_6$ alkynyl;
$L_2$ is -C(O)-$(CH_2)_p$-(4- to 12-heterocycloalkyl)-; -C(O)-$(OCH_2CH_2)_p$-(4- to 12-heterocycloalkyl)-; -C(O)NH-$(CH_2)_p$-(4- to 12-heterocycloalkyl)-; -C(O)NH-$(CH_2CH_2O)_p$-(4- to 12-heterocycloalkyl)-; -C(O)-$(CH_2CH_2O)_p$-(4- to 12-heterocycloalkyl)-; -$(OCH_2CH_2)_p$-(4- to 12-heterocycloalkyl)-; -C(O)-$(CH_2)_p$-(4- to 12-heterocycloalkyl)-C(O)-; or -$(CH_2)_p$-(4- to 12-heterocycloalkyl)-C(O)-;
$R^2$ is $NR^{10}R^{11}$;
$R^3$ is H, halogen, $NH_2$, or $C_1$-$C_4$ alkyl;
each $R^4$ is independently $C_1$-$C_6$ alkyl, $C_2$-$C_6$ alkenyl, or $C_2$-$C_6$ alkynyl, wherein the alkyl, alkenyl, or alkynyl is optionally substituted with one or more $C_1$-$C_6$ alkoxy, $C_1$-$C_6$ thioalkyl, $NH_2$, -NH($C_1$-$C_6$ alkyl), or -N($C_1$-$C_6$ alkyl)($C_1$-$C_6$ alkyl); or
two $R^4$, on adjacent carbons taken together, can combine to form $C_3$-$C_8$ cycloalkyl, heterocycloalkyl, aryl, or heteroaryl;
$R^5$ is H, OH, CN, $NO_2$. or $C_1$-$C_4$ alkyl;
each $R^6$ or $R^{6'}$ is H; or
two $R^6$ can combine to form an oxo group; or
two $R^{6'}$ can combine to form an oxo group;
$R^7$, $R^8$, and $R^9$ are each independently H, halogen, CN, $NO_2$, OH, $NH_2$, $C_1$-$C_6$ alkyl, $C_2$-$C_6$ alkenyl, $C_2$-$C_6$ alkynyl, $C_3$-$C_8$ cycloalkyl, heterocycloalkyl, aryl, or heteroaryl; or
two $R^7$ on adjacent carbons taken together, can combine to form $C_3$-$C_8$ cycloalkyl, heterocycloalkyl, aryl, or heteroaryl;
$R^{10}$ and $R^{11}$ are independently H or $C_1$-$C_4$ alkyl;
m is an integer from 0 to 2;
n is an integer from 0 to 4; and
p is an integer from 1 to 8.

**2.** The compound of claim 1, or a pharmaceutically acceptable salt thereof, wherein the compound has Formula I(a):

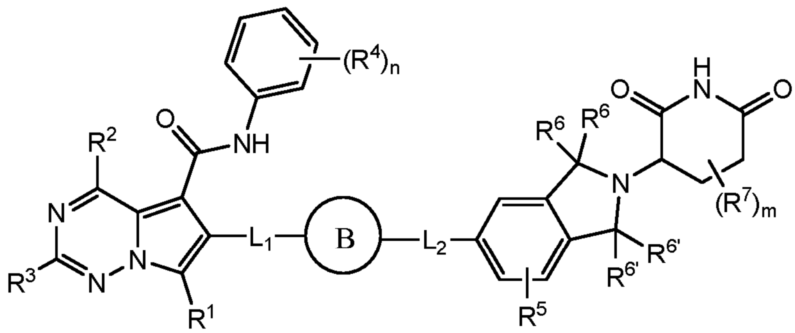

I(a);

or wherein the compound has Formula I(b):

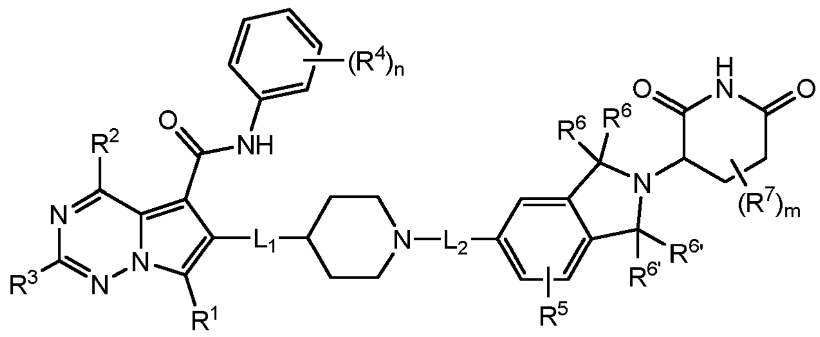

I(b);

or wherein the compound has Formula I(c):

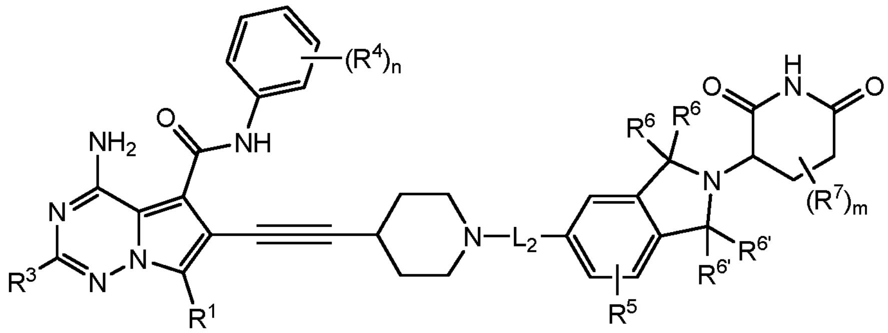

I(c);

or wherein the compound has Formula I(d):

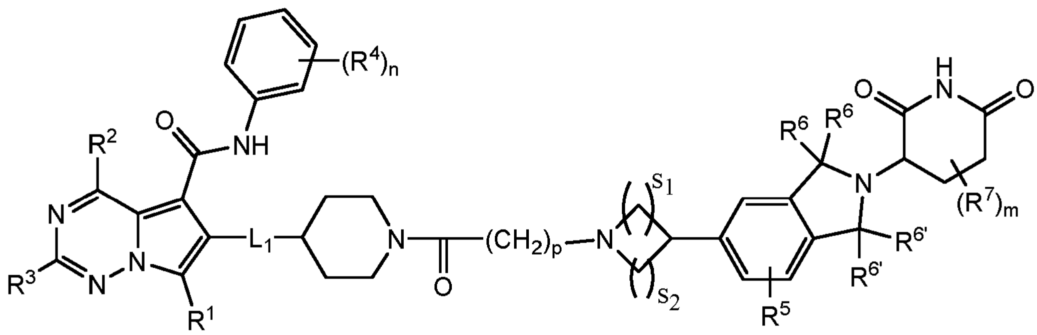

I(d),

wherein $s_1$ and $s_2$ are independently integers from 1 to 2; or

wherein the compound has Formula I(e):

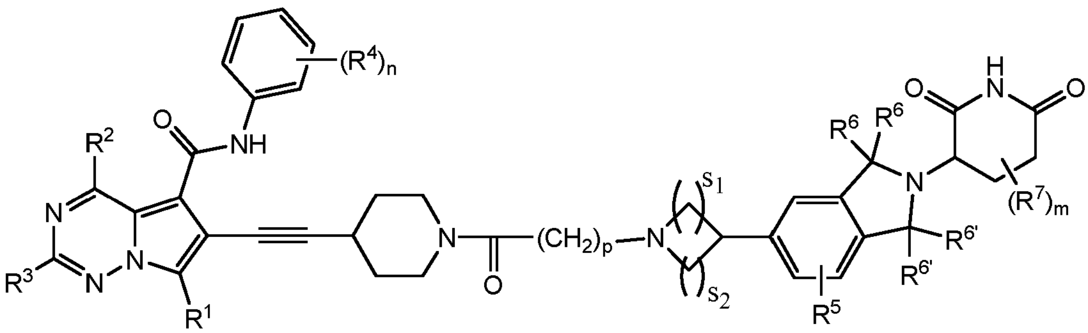

I(e),

wherein $s_1$ and $s_2$ are independently integers from 1 to 2; or wherein the compound has Formula I(f):

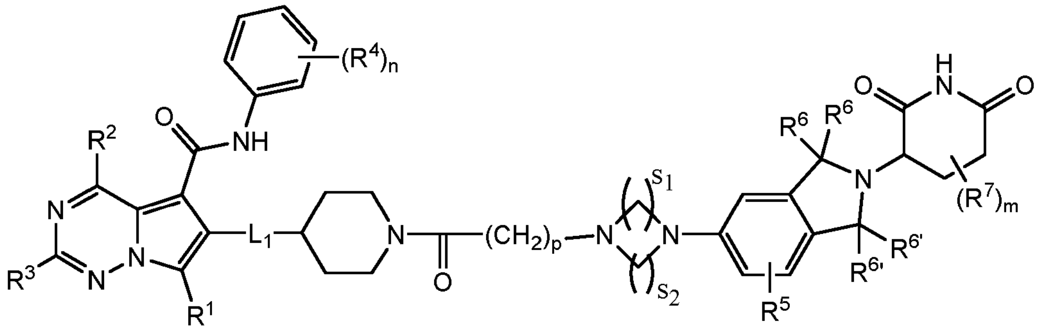

I(f),

wherein $s_1$ and $s_2$ are independently integers from 1 to 2; or wherein the compound has Formula I(g):

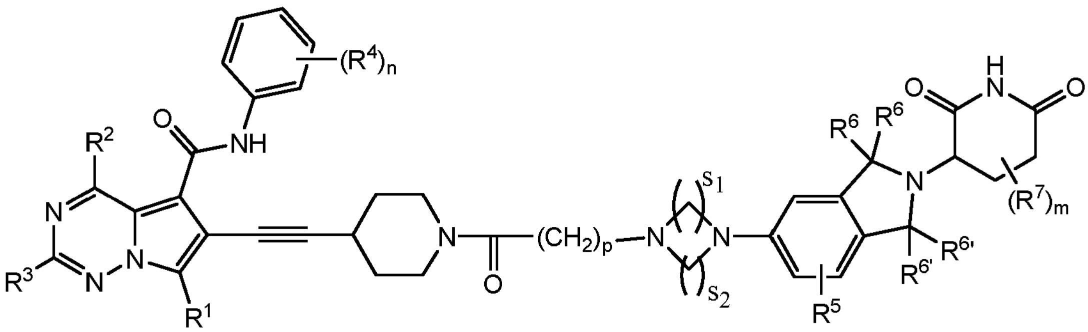

I(g),

wherein $s_1$ and $s_2$ are independently integers from 1 to 2; or wherein the compound has Formula I(h):

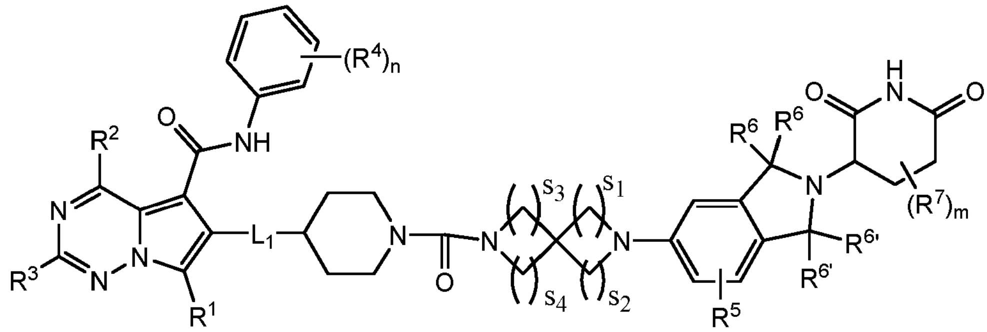

I(h),

wherein $s_1$, $s_2$, $s_3$, and $s_4$ are independently integers from 1 to 2.

**3.** The compound of claim 1 or 2, or a pharmaceutically acceptable salt thereof, wherein $R^1$ is $C_1$-$C_6$ alkyl.

**4.** The compound of any one of claims 1-3, or a pharmaceutically acceptable salt thereof, wherein $R^1$ is isopropyl.

**5.** The compound of any one of claims 1-4, or a pharmaceutically acceptable salt thereof, wherein $R^3$ is H.

**6.** The compound of any one of claims 1-5, or a pharmaceutically acceptable salt thereof, wherein $R^4$ is $C_1$-$C_6$ alkyl substituted with one or more $C_1$-$C_6$ alkoxy.

**7.** The compound of any one of claims 1-6, or a pharmaceutically acceptable salt thereof, wherein $R^4$ is methoxymethyl.

**8.** The compound of any one of claims 1-7, or a pharmaceutically acceptable salt thereof, wherein $R^6$ is H.

**9.** The compound of any one of claims 1-8, or a pharmaceutically acceptable salt thereof, wherein the compound is of the structure:

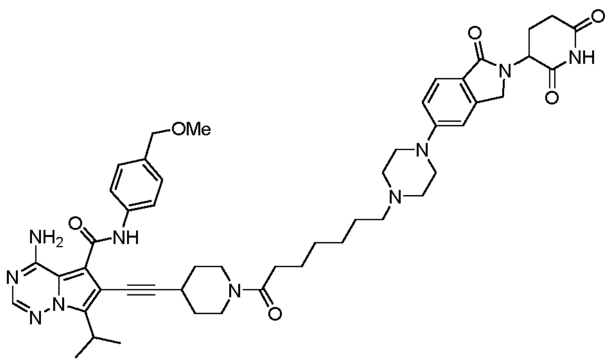

,

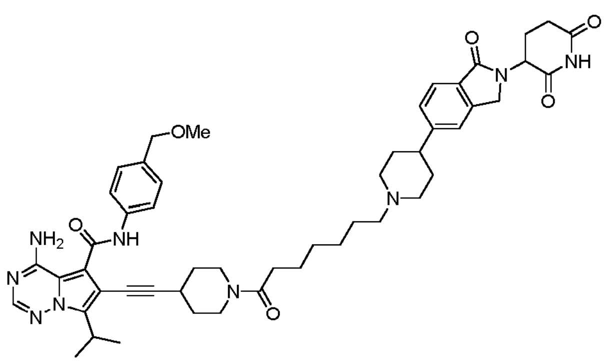

,

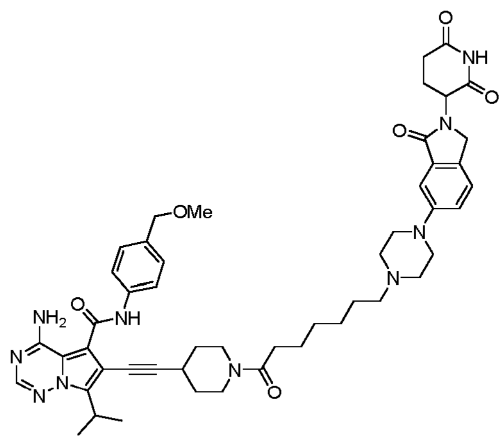

,

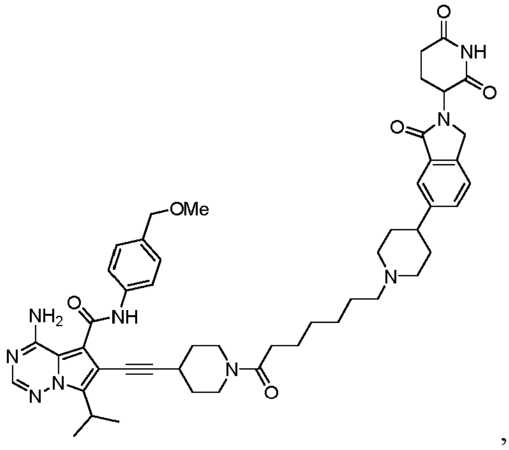

,

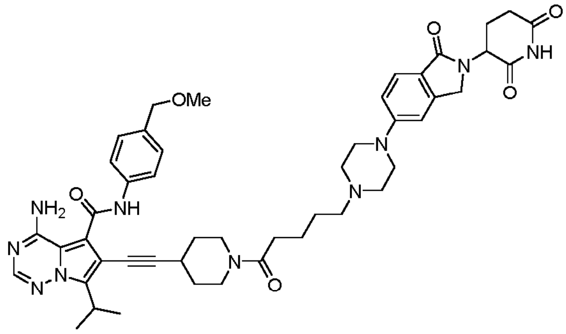

,

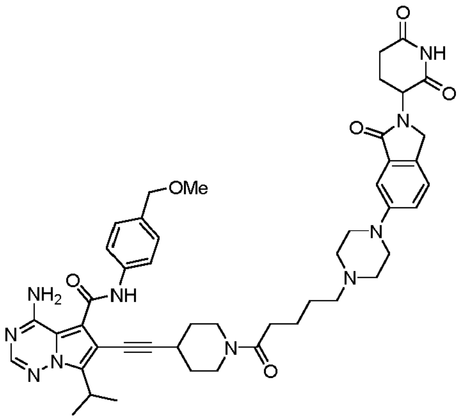

,

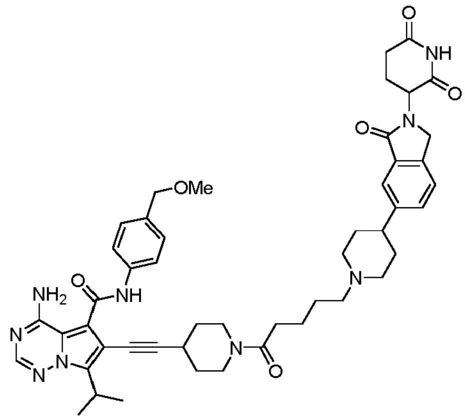

,

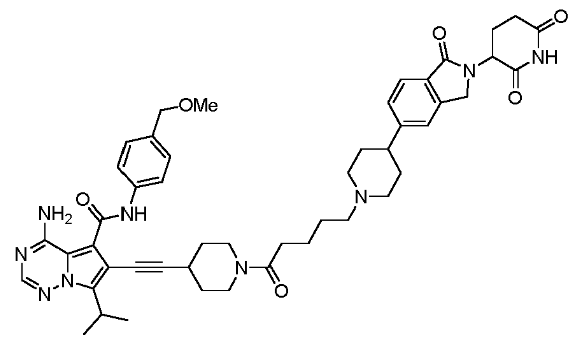

,

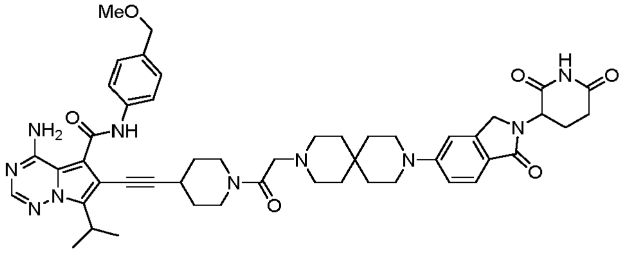

,

or

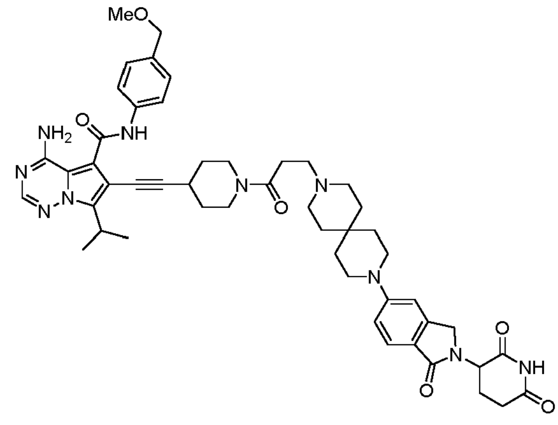

10. A pharmaceutical composition comprising a compound of any one of claims 1-9 or a pharmaceutically acceptable salt thereof, and a pharmaceutically acceptable excipient.

11. A compound of any one of claims 1-9 or a pharmaceutically acceptable salt thereof, or the pharmaceutical composition of claim 10, for use in the treatment of a RET mediated disorder.

12. The compound or pharmaceutical composition for use according to claim 11, wherein the RET mediated disorder is cancer, and optionally:

(i) wherein the cancer is non-small cell lung cancer, and/or
(ii) wherein the cancer has metastasized to the brain, and/or
(iii) wherein the cancer is relapsed or refractory cancer.

13. The compound or pharmaceutical composition for use according to claim 11 or 12, wherein the RET mediated disorder is mediated by a mutant RET.

**Patentansprüche**

1. Verbindung von Formel I:

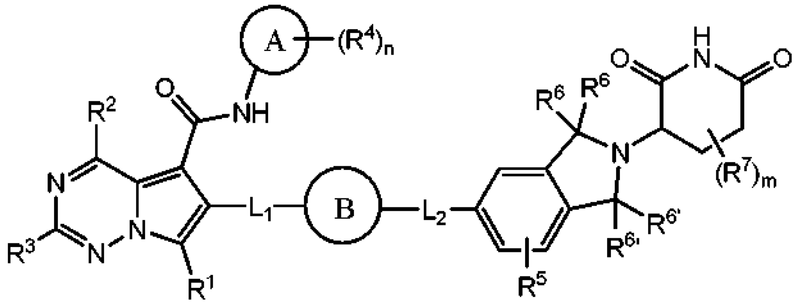

(I),

oder pharmazeutisch verträgliches Salz davon,
wobei

$R^1$ $C_1$-$C_6$ Alkyl, $C_3$-$C_8$ Cycloalkyl, Heterocycloalkyl, Aryl oder Heteroaryl ist, wobei das Alkyl, Cycloalkyl, Heterocycloalkyl, Aryl oder Heteroaryl optional mit einem oder mehreren $R^8$substituiert ist;
A Aryl oder Heteroaryl ist, wobei das Aryl oder Heteroaryl optional mit einem oder mehreren $R^9$ substituiert ist;
B Heterocycloalkyl oder $C_3$-$C_8$ Cycloalkyl ist;
$L_1$ $C_1$-$C_6$ Alkyl, $C_2$-$C_6$ Alkenyl oder $C_2$-$C_6$ Alkinyl ist;
$L_2$ -C(O)-$(CH_2)_p$-(4- bis 12-Heterocycloalkyl)-; -C(O)-$(OCH_2CH_2)_p$-(4- bis 12-Heterocycloalkyl)-; -C(O)NH-$(CH_2)_p$-(4- bis 12-Heterocycloalkyl)-; -C(O)NH-$(CH_2CH_2O)_p$,-(4-bis 12-Heterocycloalkyl)-; -C(O)-$(CH_2CH_2O)_p$-(4- bis 12-Heterocycloalkyl)-; -$(OCH_2CH_2)_p$-(4-bis 12-Heterocycloalkyl)-; -C(O)-$(CH_2)_p$-(4-

bis 12-Heterocycloalkyl)-C(O)-; oder -$(CH_2)_p$-(4-bis 12-Heterocycloalkyl)-C(O)- ist;
$R^2$ $NR^{10}R^{11}$ ist;
$R^3$ H, Halogen, $NH_2$, oder $C_1$-$C_4$ Alkyl ist;
jedes $R^4$ unabhängig $C_1$-$C_6$ Alkyl, $C_2$-$C_6$ Alkenyl oder $C_2$-$C_6$ Alkinyl ist, wobei das Alkyl, Alkenyl oder Alkinyl optional mit einem oder mehreren $C_1$-$C_6$ Alkoxy, $C_1$-$C_6$ Thioalkyl, $NH_2$, -NH($C_1$-$C_6$ Alkyl) oder -N($C_1$-$C_6$ Alkyl)($C_1$-$C_6$ Alkyl) substituiert ist; oder
zwei $R^4$an benachbarten Kohlenstoffatomen zusammen sich verbinden können, um $C_3$-$C_8$ Cycloalkyl, Heterocycloalkyl, Aryl oder Heteroaryl zu bilden;
$R^5$ H, OH, CN, $NO_2$. oder $C_1$-$C_4$ Alkyl ist;
jedes $R^6$ oder $R^6$ H ist; oder
zwei $R^6$ sich verbinden können, um eine Oxogruppe zu bilden; oder
zwei $R^6$ sich verbinden können, um eine Oxogruppe zu bilden;
$R^7$, $R^8$ und $R^9$jeweils unabhängig voneinander H, Halogen, CN, $NO_2$, OH, $NH_2$, $C_1$-$C_6$ Alkyl, $C_2$-$C_6$ Alkenyl, $C_2$-$C_6$ Alkinyl, $C_3$-$C_8$ Cycloalkyl, Heterocycloalkyl, Aryl oder Heteroaryl sind; oder
zwei $R^7$ an benachbarten Kohlenstoffatomen zusammen sich verbinden können, um $C_3$-$C_8$ Cycloalkyl, Heterocycloalkyl, Aryl oder Heteroaryl zu bilden;
$R^{10}$ und $R^{11}$ unabhängig voneinander H oder $C_1$-$C_4$ Alkyl sind;
m eine ganze Zahl von 0 bis 2 ist;
n eine ganze Zahl von 0 bis 4 ist; und
p eine ganze Zahl von 1 bis 8 ist.

**2.** Verbindung nach Anspruch 1 oder pharmazeutisch verträgliches Salz davon, wobei die Verbindung die Formel I(a) hat:

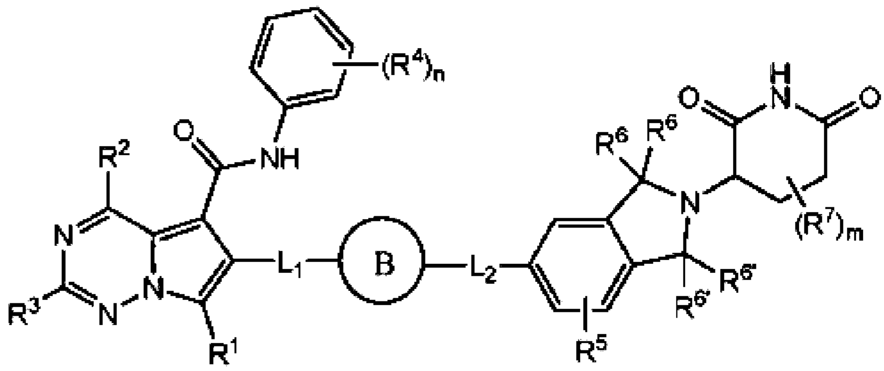

I(a);

oder

wobei die Verbindung die Formel I(b) hat:

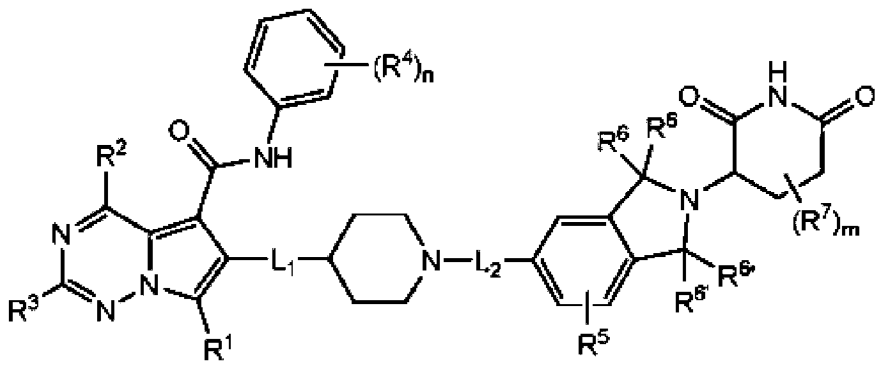

I(b);

oder
wobei die Verbindung die Formel I(c) hat:

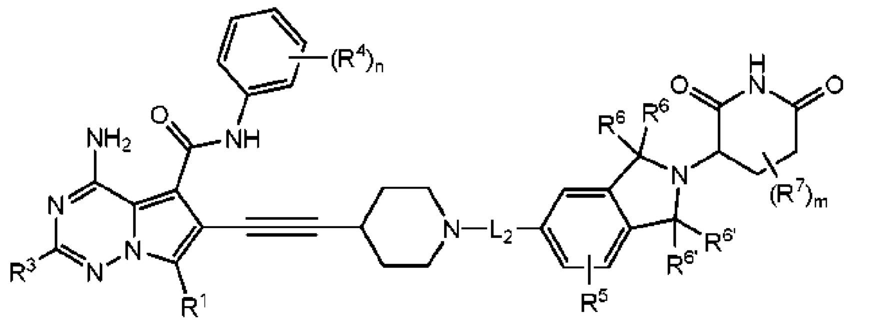

I(c);

oder

wobei die Verbindung die Formel I(d) hat:

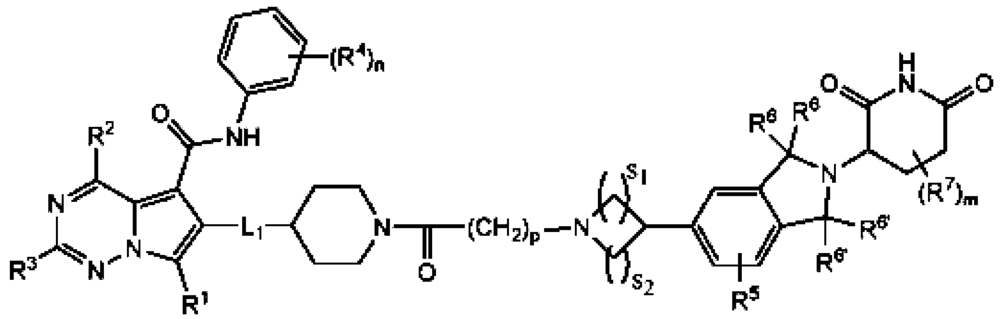

I(d),

wobei $s_1$ und $s_2$ unabhängig voneinander ganze Zahlen von 1 bis 2 sind; oder

wobei die Verbindung die Formel I(e) hat:

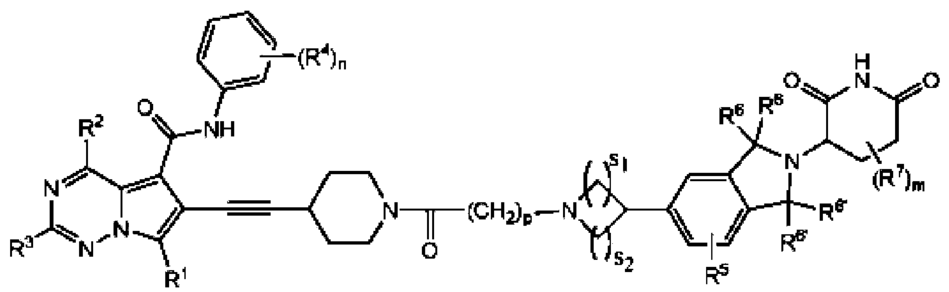

I(e),

wobei $s_1$ und $s_2$ unabhängig voneinander ganze Zahlen von 1 bis 2 sind; oder

wobei die Verbindung die Formel I(f) hat:

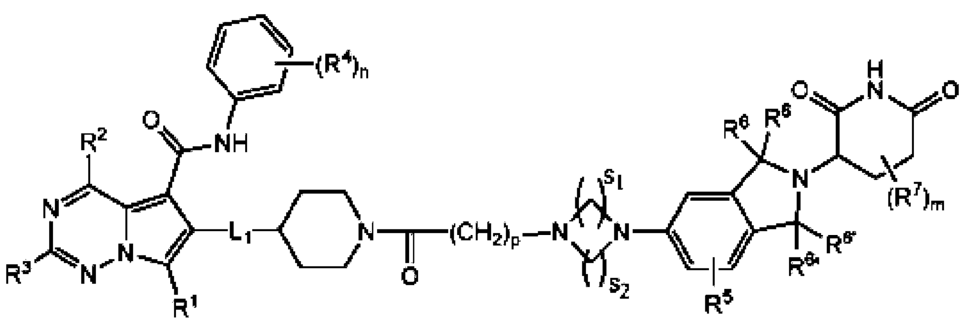

I(f),

wobei $s_1$ und $s_2$ unabhängig voneinander ganze Zahlen von 1 bis 2 sind; oder

wobei die Verbindung die Formel I(g) hat:

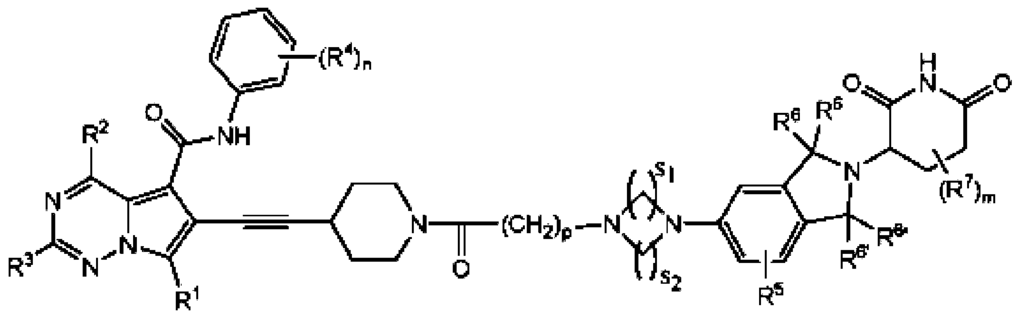

I(g),

wobei $s_1$ und $s_2$ unabhängig voneinander ganze Zahlen von 1 bis 2 sind; oder wobei die Verbindung die Formel I(h) hat:

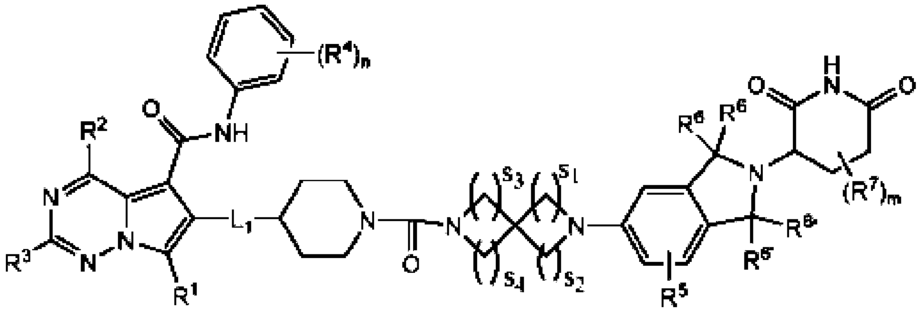

I(h),

wobei $s_1$, $s_2$, $s_3$ und $s_4$ unabhängig voneinander ganze Zahlen von 1 bis 2 sind.

**3.** Verbindung nach Anspruch 1 oder 2 oder pharmazeutisch verträgliches Salz davon, wobei $R^1$ $C_1$-$C_6$ Alkyl ist.

**4.** Verbindung nach einem der Ansprüche 1 bis 3 oder pharmazeutisch verträgliches Salz davon, wobei $R^1$ Isopropyl ist.

**5.** Verbindung nach einem der Ansprüche 1 bis 4 oder pharmazeutisch verträgliches Salz davon, wobei $R^3$ H ist.

**6.** Verbindung nach einem der Ansprüche 1 bis 5 oder pharmazeutisch verträgliches Salz davon, wobei $R^4$ $C_1$-$C_6$ Alkyl ist, das mit einem oder mehreren $C_1$-$C_6$ Alkoxy substituiert ist.

**7.** Verbindung nach einem der Ansprüche 1 bis 6 oder pharmazeutisch verträgliches Salz davon, wobei $R^4$ Methoxymethyl ist.

**8.** Verbindung nach einem der Ansprüche 1 bis 7 oder pharmazeutisch verträgliches Salz davon, wobei $R^6$ H ist.

**9.** Verbindung nach einem der Ansprüche 1 bis 8 oder pharmazeutisch verträgliches Salz davon, wobei die Verbindung folgende Struktur hat:

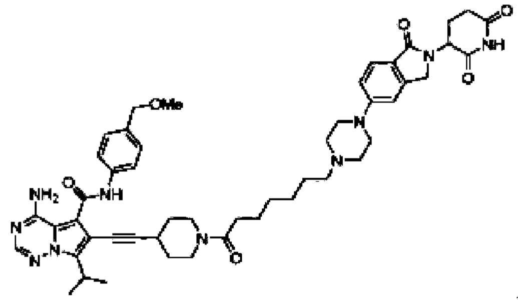

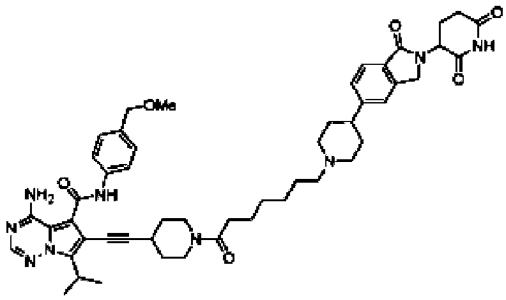
,
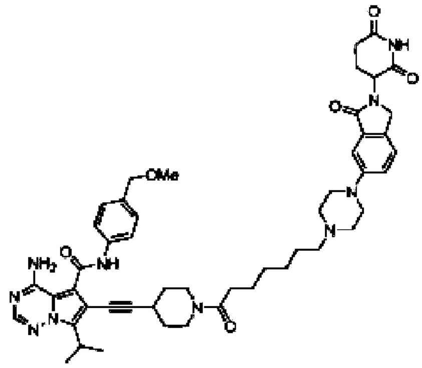
,
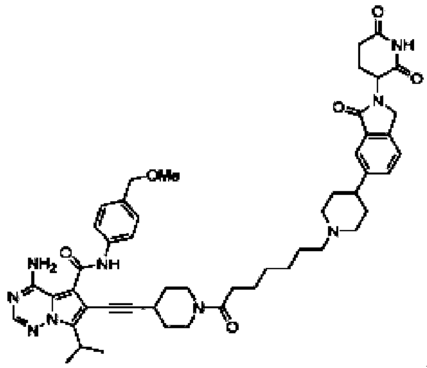
,
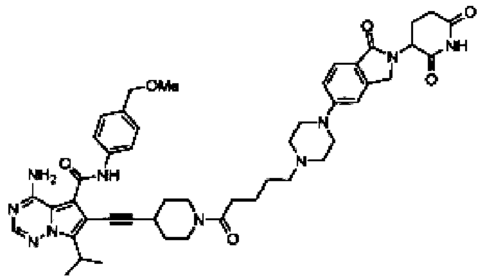
,
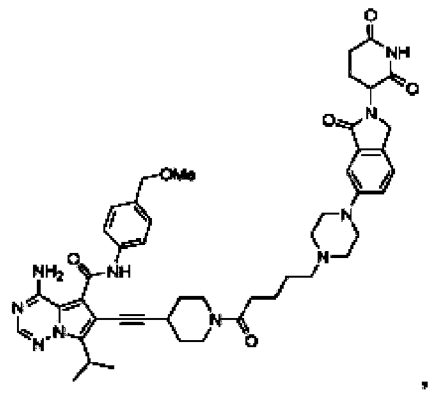
,
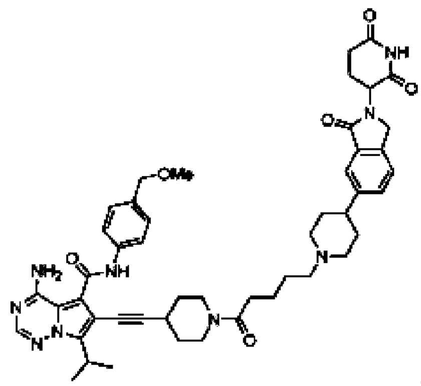
,
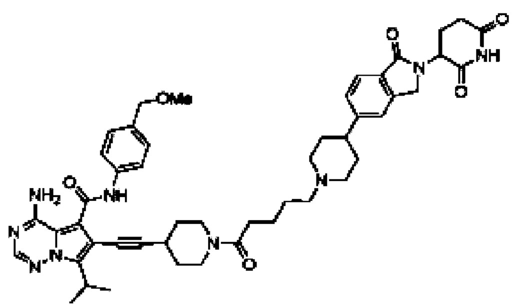
.

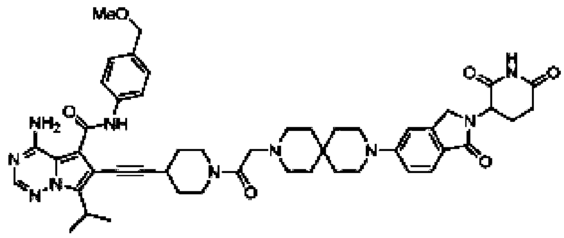

,

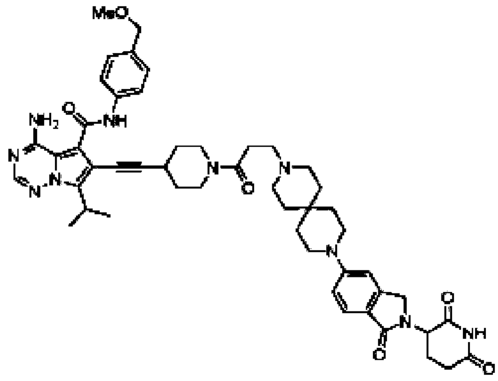

.

**10.** Pharmazeutische Zusammensetzung, die eine Verbindung nach einem der Ansprüche 1 bis 9 oder ein pharmazeutisch verträgliches Salz davon und einen pharmazeutisch verträglichen Hilfsstoff umfasst.

**11.** Verbindung nach einem der Ansprüche 1 bis 9 oder pharmazeutisch verträgliches Salz davon oder pharmazeutische Zusammensetzung nach Anspruch 10 zur Verwendung bei der Behandlung von RET-vermittelten Erkrankungen.

**12.** Verbindung oder pharmazeutische Zusammensetzung zur Verwendung nach Anspruch 11, wobei es sich bei der RET-vermittelten Erkrankung um Krebs handelt, und optional:

(i) wobei es sich bei dem Krebs um nicht-kleinzelligen Lungenkrebs handelt, und/oder
(ii) wobei der Krebs in das Gehirn metastasiert hat, und/oder
(iii) wobei es sich bei dem Krebs um einen rezidivierenden oder refraktären Krebs handelt.

**13.** Verbindung oder pharmazeutische Zusammensetzung zur Verwendung nach Anspruch 11 oder 12, wobei die RET-vermittelte Erkrankung durch ein mutiertes RET vermittelt wird.

## Revendications

**1.** Composé de Formule I :

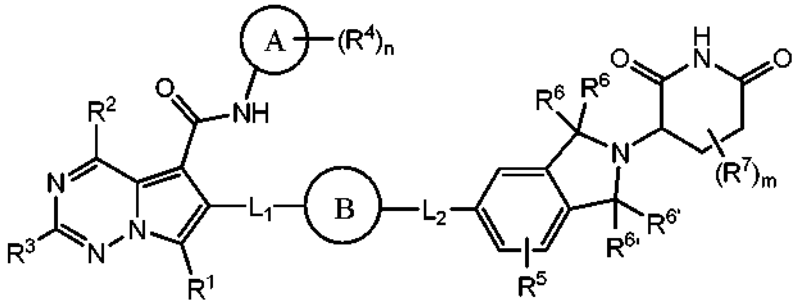

(I),

ou sel pharmaceutiquement acceptable de celui-ci,
dans lequel

$R^1$ est un alkyle en $C_1$-$C_6$, un cycloalkyle en $C_3$-$C_8$, un hétérocycloalkyle, un aryle ou un hétéroaryle, dans lequel l'alkyle, le cycloalkyle, l'hétérocycloalkyle, l'aryle ou l'hétéroaryle est facultativement substitué par un ou plusieurs $R^8$ ;
A est un aryle ou un hétéroaryle, dans lequel l'aryle ou l'hétéroaryle est facultativement substitué par un ou plusieurs $R^9$ ;
B est un hétérocycloalkyle ou un cycloalkyle en $C_3$-$C_8$ ;
$L_1$ est un alkyle en $C_1$-$C_6$, un alcényle en $C_2$-$C_6$ ou un alcynyle en $C_2$-$C_6$ ;
$L_2$ est -C(O)-$(CH_2)_p$-(hétérocycloalkyle à 4 à 12 chaînons)- ; -C(O)-$(OCH_2CH_2)_p$-(hétérocycloalkyle à 4 à 12 chaînons)-; -C(O)NH-$(CH_2)_p$-(hétérocycloalkyle à 4 à 12 chaînons)-; -C(O)NH-$(CH_2CH_2O)_p$-(hétérocycloalkyle à 4 à 12 chaînons)-; -C(O)-$(CH_2CH_2O)_p$-(hétérocycloalkyle à 4 à 12 chaînons)- ; -$(OCH_2CH_2)_p$-(hétérocycloalkyle à 4 à 12 chaînons)-; -C(O)-$(CH_2)_p$-(hétérocycloalkyle à 4 à 12 chaînons)-C(O)-; ou -$(CH_2)_p$-(hétérocycloalkyle à 4 à 12 chaînons)-C(O)- ;
$R^2$ est $NR^{10}R^{11}$ ;
$R^3$ est H, un halogène, $NH_2$ ou un alkyle $C_1$-$C_4$ ;
chaque $R^4$ est indépendamment un alkyle en $C_1$-$C_6$, un alcényle en $C_2$-$C_6$ ou un alcynyle en $C_2$-$C_6$, dans lequel l'alkyle, l'alcényle ou l'alcynyle est facultativement substitué par un ou plusieurs alcoxy en $C_1$-$C_6$, thioalkyle en $C_1$-$C_6$, $NH_2$, -NH(alkyle en $C_1$-$C_6$) ou -N(alkyle en $C_1$-$C_6$)(alkyle en $C_1$-$C_6$) ; ou
deux $R^4$, sur des carbones adjacents pris ensemble, peuvent se combiner pour former un cycloalkyle en $C_3$-$C_8$, un hétérocycloalkyle, un aryle ou un hétéroaryle ;
$R^5$ est H, OH, CN, $NO_2$, ou un alkyle en $C_1$-$C_4$;
chaque $R^6$ ou $R^6$ est H ; ou
deux $R^6$ peuvent se combiner pour former un groupe oxo ; ou
deux $R^6$ peuvent se combiner pour former un groupe oxo ;
$R^7$, $R^8$ et $R^9$ sont chacun indépendamment H, un halogène, CN, $NO_2$, OH, $NH_2$, un alkyle en $C_1$-$C_6$, un alcényle en $C_2$-$C_6$, un alcynyle en $C_2$-$C_6$, un cycloalkyle en $C_3$-$C_8$, un hétérocycloalkyle, un aryle ou un hétéroaryle ; ou
deux $R^7$, sur des carbones adjacents pris ensemble, peuvent se combiner pour former un cycloalkyle en $C_3$-$C_8$, un hétérocycloalkyle, un aryle ou un hétéroaryle ;
$R^{10}$ et $R^{11}$ sont indépendamment H ou un alkyle en $C_1$-$C_4$;
m est un entier compris entre 0 et 2 ;
n est un entier compris entre 0 et 4 ; et
p est un entier compris entre 1 et 8.

2. Composé selon la revendication 1, ou sel pharmaceutiquement acceptable de celui-ci, dans lequel le composé présente la Formule I(a) :

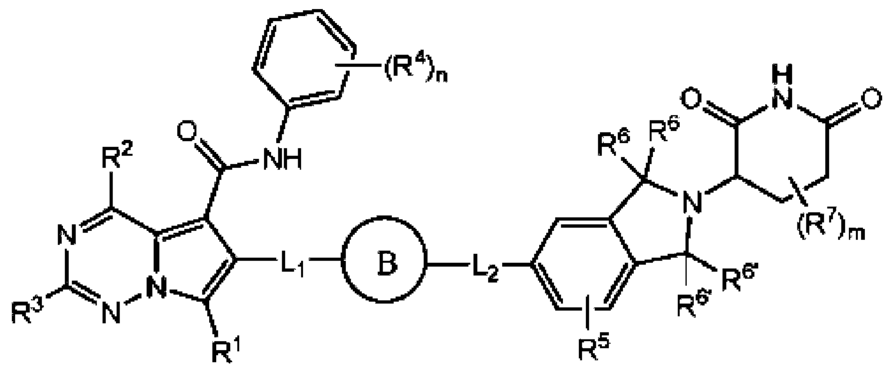

I(a);

ou

dans lequel le composé présente la Formule I(b) :

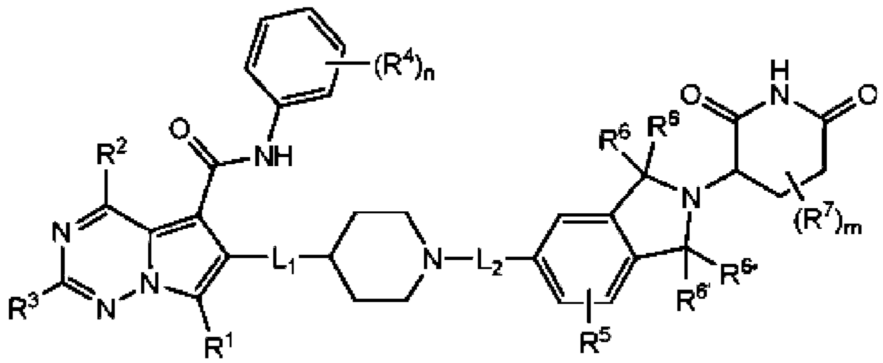

I(b);

ou

dans lequel le composé présente la Formule I(c) :

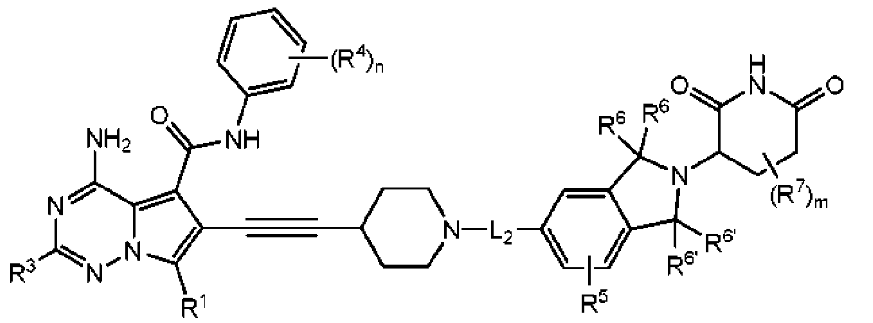

I(c);

ou

dans lequel le composé présente la Formule I(d) :

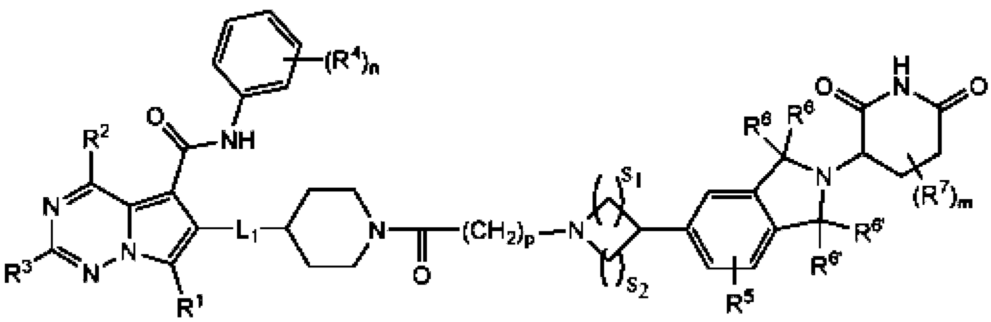

I(d),

dans lequel $s_1$ et $s_2$ sont indépendamment des entiers compris entre 1 et 2 ; ou

dans lequel le composé présente la Formule I(e) :

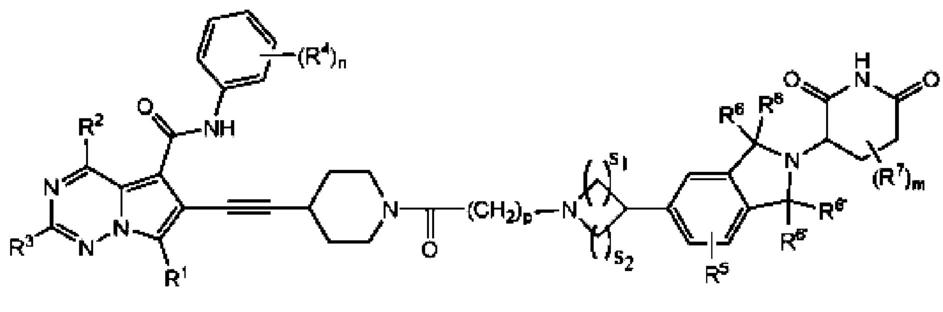

I(e),

dans lequel $s_1$ et $s_2$ sont indépendamment des entiers compris entre 1 et 2 ; ou

dans lequel le composé présente la Formule I(f) :

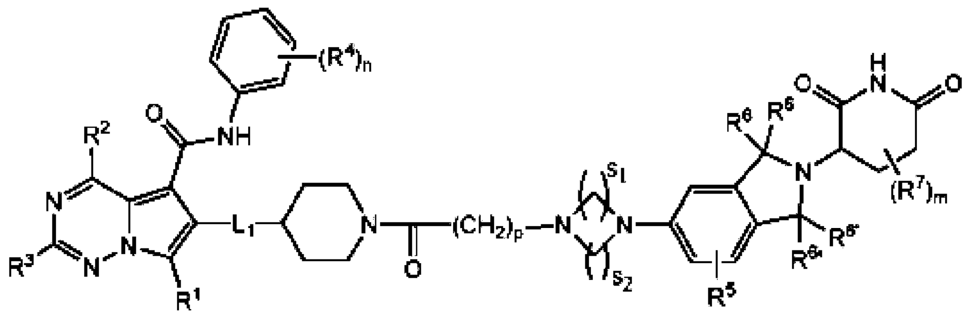

I(f),

dans lequel $s_1$ et $s_2$ sont indépendamment des entiers compris entre 1 et 2 ; ou dans lequel le composé présente la Formule I(g) :

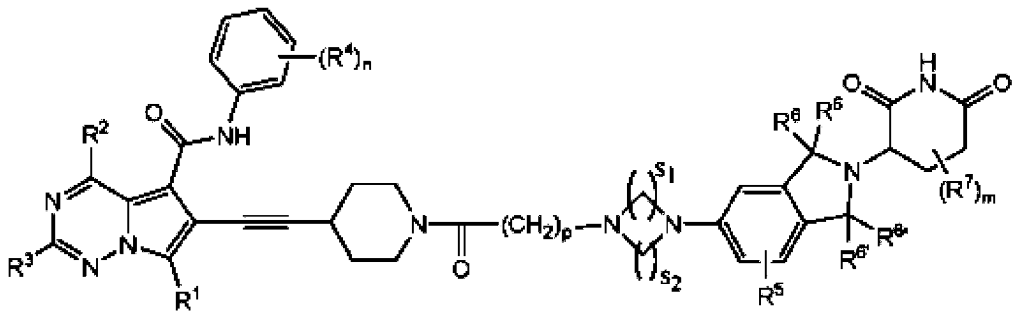

I(g),

dans lequel $s_1$ et $s_2$ sont indépendamment des entiers compris entre 1 et 2 ; ou dans lequel le composé présente la Formule I(h) :

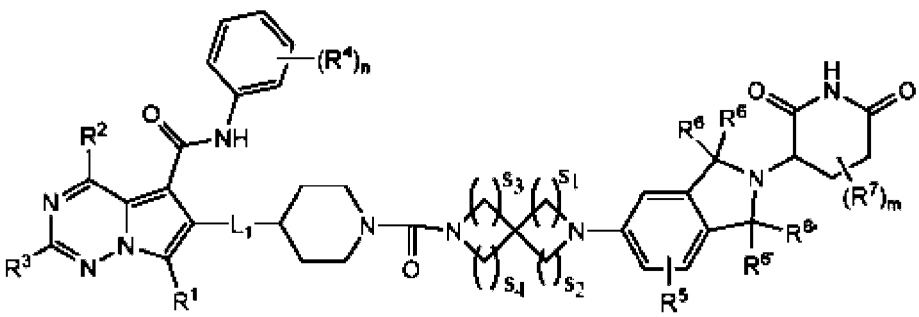

I(h),

dans lequel $s_1$, $s_2$, $s_3$ et $s_4$ sont indépendamment des entiers compris entre 1 et 2.

**3.** Composé selon la revendication 1 ou 2, ou sel pharmaceutiquement acceptable de celui-ci, dans lequel $R^1$ est un alkyle en $C_1$-$C_6$.

**4.** Composé selon l'une quelconque des revendications 1-3, ou sel pharmaceutiquement acceptable de celui-ci, dans lequel $R^1$ est un isopropyle.

**5.** Composé selon l'une quelconque des revendications 1-4, ou sel pharmaceutiquement acceptable de celui-ci, dans lequel $R^3$ est H.

**6.** Composé selon l'une quelconque des revendications 1-5, ou sel pharmaceutiquement acceptable de celui-ci, dans lequel $R^4$ est un alkyle en $C_1$-$C_6$ substitué par un ou plusieurs alcoxy en $C_1$-$C_6$.

7. Composé selon l'une quelconque des revendications 1-6, ou sel pharmaceutiquement acceptable de celui-ci, dans lequel $R^4$ est un méthoxyméthyle.

8. Composé selon l'une quelconque des revendications 1-7, ou sel pharmaceutiquement acceptable de celui-ci, dans lequel $R^6$ est H.

9. Composé selon l'une quelconque des revendications 1-8, ou sel pharmaceutiquement acceptable de celui-ci, dans lequel le composé présente la structure :

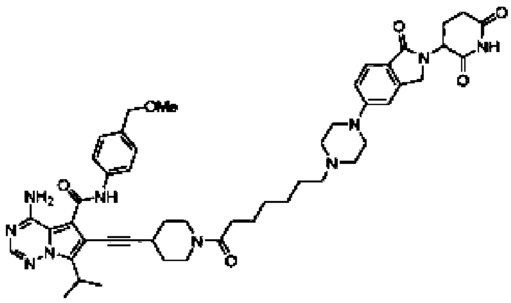
,

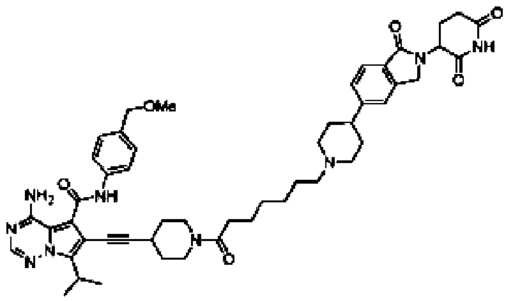
,
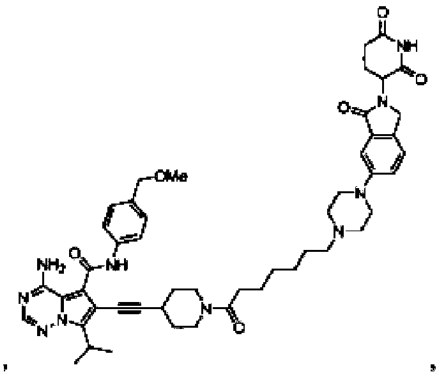
,

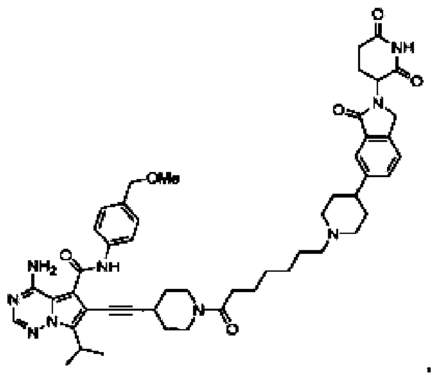
,
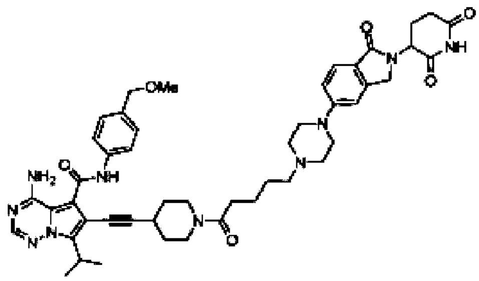
,

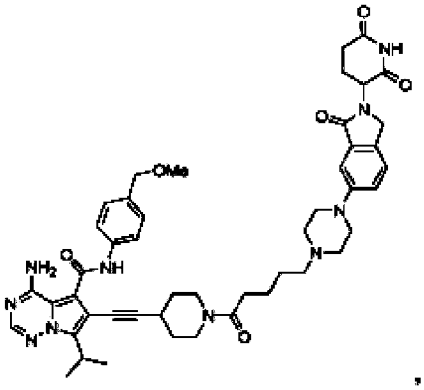,
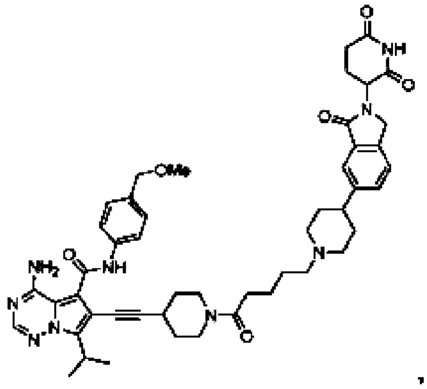,

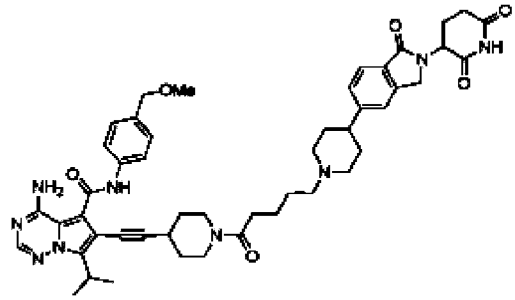.

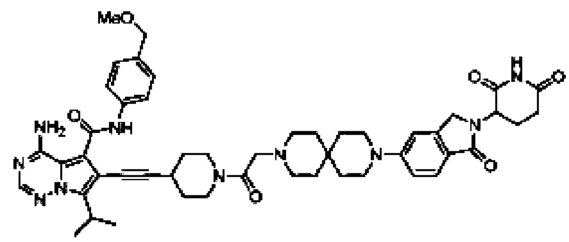,

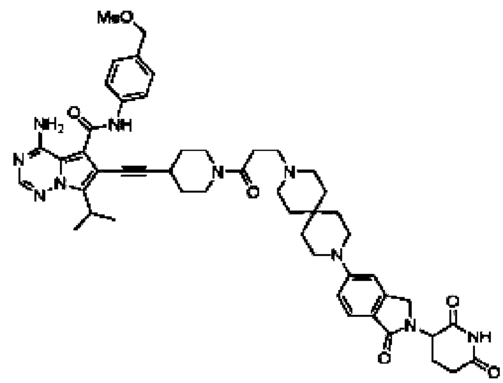.

**10.** Composition pharmaceutique comprenant un composé selon l'une quelconque des revendications 1-9 ou un sel pharmaceutiquement acceptable de celui-ci, et un excipient pharmaceutiquement acceptable.

**11.** Composé selon l'une quelconque des revendications 1-9 ou sel pharmaceutiquement acceptable de celui-ci, ou composition pharmaceutique selon la revendication 10, destiné(e) à être utilisé(e) dans le traitement d'un trouble lié au RET.

**12.** Composé ou composition pharmaceutique destiné(e) à être utilisé(e) selon la revendication 11, dans lequel/laquelle le trouble lié au RET est un cancer, et facultativement :

(i) dans lequel le cancer est un cancer du poumon non à petites cellules, et/ou
(ii) dans lequel le cancer a métastasé au cerveau, et/ou
(iii) dans lequel le cancer est un cancer récidivant ou réfractaire.

**13.** Composé ou composition pharmaceutique destiné(e) à être utilisé(e) selon la revendication 11 ou 12, dans lequel/laquelle le trouble lié au RET est induit par un RET mutant.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020063751 A1
- EP 3974422 A1
- US 2009131425 A1
- US 4522811 A
- WO 201419176 A
- WO 2013090921 A
- WO 2014203129 A
- WO 2014203132 A
- US 20130178445 A
- US 9078871 B
- US 8853423 B
- US 8703810 B
- US 20150005286 A
- WO 2014205136 A
- WO 2014205138 A
- US 4418068 A
- US 5478847 A
- US 5393763 A
- US 5457117 A
- WO 2011156518 A
- US 8455534 B
- US 8299112 B
- US 20160175289 A
- US 20150258080 A
- WO 2014191726 A
- WO 2012084711 A
- WO 2002013802 A
- WO 2002004418 A
- WO 2002003992 A
- WO 2002003991 A
- WO 2002003990 A
- WO 2002003989 A
- WO 2002003988 A
- WO 2002003986 A
- WO 2002003977 A
- WO 2002003976 A
- WO 2002003975 A
- WO 2006078834 A
- US 6821989 B
- US 20020128276 A
- US 6777424 B
- US 20020016340 A
- US 6326392 B
- US 6756401 B
- US 20020013327 A
- US 6512002 B
- US 6632834 B
- US 20010056099 A
- US 6583170 B
- US 6479535 B
- WO 1999024027 A
- US 6005102 A
- EP 0802184 A
- US 5998402 A
- US 5780497 A
- US 5880137 A
- WO 2012048058 A
- WO 2007087684 A
- US 20110117073 A


### Non-patent literature cited in the description


- **LU, G. et al.** The myeloma drug lenalidomide promotes the cereblon-dependent destruction of Ikaros proteins. *Science*, 2014, vol. 343, 305-309
- **KRÖNKE, J. et al.** Lenalidomide causes selective degradation of IKZF1 and IKZF3 in multiple myeloma cells. *Science*, 2014, vol. 343, 301-305
- **LIU XUAN et al.** RET kinase alterations in targeted cancer therapy. *Cancer Drug Resist*, 2020
- **MULLIGAN LM.** RET revisited: expanding the oncogenic portfolio. *Nat Rev Cancer.*, 2014, vol. 14 (3), 173-186
- **SOLOMON et al.** RET Solvent Front Mutations Mediated Acquired Resistance to Selective RET Inhibition in RET-driven malignancies. *J Thoracic Oncolog.*, 2020
- **SONG M.** Progress in Discovery of KIF5B-RET Kinase Inhibitors for the Treatment of Non-Small-Cell Lung Cancer. *J Med Chem.*, 2015, vol. 58 (9), 3672-3681
- **WATSON AJ. et al.** Identification of selective inhibitors of RET and comparison with current clinical candidates through development and validation of a robust screening cascade. *F1000Research*, 2016, vol. 5, 1005

- **SMITH, M. B.** ; **MARCH, J.** March's Advanced Organic Chemistry: Reactions, Mechanisms, and Structure. John Wiley & Sons, 2001
- **GREENE, T. W.** ; **WUTS, P. G. M.** Protective Groups in Organic Synthesis. John Wiley & Sons, 1999
- **R. LAROCK**. Comprehensive Organic Transformations. VCH Publishers, 1989
- **L. FIESER** ; **M. FIESER**. Fieser and Fieser's Reagents for Organic Synthesis. John Wiley and Sons, 1994
- Encyclopedia of Reagents for Organic Synthesis. John Wiley and Sons, 1995
- **AUSUBEL et al.** Current Protocols in Molecular Biology. John Wiley and Sons, Inc., 2005
- **SAMBROOK et al.** Molecular Cloning, A Laboratory Manual. Cold Spring Harbor Press, 2000
- **COLIGAN et al.** Current Protocols in Immunology. John Wiley & Sons
- **ENNA et al.** Current Protocols in Pharmacology. John Wiley & Sons
- The Pharmacological Basis of Therapeutics. **FINGL et al.** Remington's Pharmaceutical Sciences. Mack Publishing Co., 1975
- Remington: the Science and Practice of Pharmacy. Mack Publishing Co, 1995
- **CAHN et al.** *Angew. Chem. Inter. Edit.*, 1966, vol. 5, 385
- **CAHN et al.** *Angew. Chem.*, 1966, vol. 78, 413
- **CAHN** ; **INGOLD**. *J. Chem Soc.*, 1951, 612
- **CAHN et al.** *Experientia*, 1956, vol. 12, 81
- **CAHN**. *J. Chem. Educ.*, 1964, vol. 41, 116
- **J. MARCH**. Advanced Organic Chemistry. John Wiley and Sons, 2001
- Methods in Enzymology. Academic Press, 1985, vol. 42, 309-396
- Design of Pro-drugs. Elsevier, 1985
- Design and Application of Pro-drugs. **H. BUNDGAARD**. A Textbook of Drug Design and Development. 1991, 113-191
- **H. BUNDGAARD**. *Advanced Drug Delivery Reviews*, 1992, vol. 8, 1-38
- **H. BUNDGAARD et al.** *Journal of Pharmaceutical Sciences*, 1988, vol. 77, 285
- **N KAKEYA et al.** *Chem. Pharm Bull.*, 1984, vol. 32, 692
- **T. HIGUCHI** ; **V. STELLA**. Pro-Drugs as Novel Delivery Systems. *A.C.S. Symposium Series*, vol. 14
- Bioreversible Carriers in Drug Design. Pergamon Press, 1987
- **AKINLEYE**. *Journal of Hematology & Oncology*, 2013, vol. 6, 59
- **SINGH et al.** Discovery and Development of Spleen Tyrosine Kinase (SYK) Inhibitors. *J. Med. Chem.*, 2012, vol. 55, 3614-3643
- **AGNEW**. *Chem. Inti. Ed Engl.*, 1994, vol. 33, 183-186
- **SALTZ et al.** *Proc. Am. Soc. Clin. Oncol.*, 1999, vol. 18, 233a
- **DOUILLARD et al.** *Lancet*, 2000, vol. 355 (9209), 1041-1047
- Remington's Pharmaceutical Sciences. Mack Publishing Company, 1990
- *Pharmaceutical Research*, 1986, vol. 3 (6), 318